# EUROPEAN PATENT SPECIFICATION

(11) **EP 3 980 764 B1**
(45) Date of publication and mention of the grant of the patent: **18.11.2026**
(21) Application number: 20819321.9
(22) Date of filing: 05.06.2020
(51) Int. Cl.: G01N 23/223, G01N 33/68, G16H 50/20, G01N 33/84, H01J 49/10, G01N 1/04

(54) **SYSTEMS AND METHODS FOR DIAGNOSTICS FOR BIOLOGICAL DISORDERS ASSOCIATED WITH PERIODIC VARIATIONS IN METAL METABOLISM**
SYSTEME UND VERFAHREN ZUR DIAGNOSE VON BIOLOGISCHEN ERKRANKUNGEN IM ZUSAMMENHANG MIT PERIODISCHEN VARIATIONEN DES METALLMETABOLISMUS
SYSTÈMES ET MÉTHODES DE DIAGNOSTIC DE TROUBLES BIOLOGIQUES ASSOCIÉS À DES VARIATIONS PÉRIODIQUES DU MÉTABOLISME DES MÉTAUX

(30) Priority: 06.06.2019 US 201962858260 P
(43) Date of publication of application: 13.04.2022
(73) Proprietor: Icahn School of Medicine at Mount Sinai, New York, NY 10029 (US)
(72) Inventor: ARORA, Manish, New York, NY 10029 (US); CURTIN, Paul, New York, NY 10029 (US); AUSTIN, Christine, New York, NY 10029 (US)
(74) Representative: Grund, Martin
(86) International application number: PCT/US2020/036368
(87) International publication number: WO 2020/247781

(56) References cited:
- US-A1- 2007 176 088
- US-A1- 2007 258 561
- US-A1- 2016 055 420
- US-B2- 7 653 496
- GAUDIUSO ROSALBA ET AL: "Laser-induced breakdown spectroscopy for human and animal health: A review", SPECTROCHIMICA ACTA. PART B: ATOMIC SPECTROSCOPY, vol. 152, 24 November 2018 (2018-11-24), pages 123 - 148, XP085615940, ISSN: 0584-8547, DOI: 10.1016/J.SAB.2018.11.006
- POZEBON D ET AL: "Elemental hair analysis: A review of procedures and applications", ANALYTICA CHIMICA ACTA, vol. 992, 13 September 2017 (2017-09-13), pages 1 - 23, XP085235098, ISSN: 0003-2670, DOI: 10.1016/J.ACA.2017.09.017
- LIMBECK ANDREAS ET AL: "Recent advances in quantitative LA-ICP-MS analysis: challenges and solutions in the life sciences and environmental chemistry", vol. 407, no. 22, 14 July 2015 (2015-07-14), Berlin/Heidelberg, pages 6593 - 6617, XP093048528, ISSN: 1618-2642, Retrieved from the Internet <URL:http://link.springer.com/content/pdf/10.1007/s00216-015-8858-0.pdf> DOI: 10.1007/s00216-015-8858-0
- MORISHITA HIROFUMI ET AL: "Tooth-Matrix Biomarkers to Reconstruct Critical Periods of Brain Plasticity", TRENDS IN NEUROSCIENCES, vol. 40, no. 1, 27 December 2016 (2016-12-27), pages 1 - 3, XP029873898, ISSN: 0166-2236, DOI: 10.1016/J.TINS.2016.11.003
- SAGHAZADEH AMENE ET AL: "Status of essential elements in autism spectrum disorder: systematic review and meta-analysis", REVIEWS IN THE NEUROSCIENCES, 29 June 2017 (2017-06-29), XP093049156, Retrieved from the Internet <URL:https://www.degruyter.com/document/doi/10.1515/revneuro-2017-0015/html> [retrieved on 20230524]
- MARYAM M ABDULLAH ET AL: "Heavy Metal in Children's Tooth Enamel: Related to Autism and Disruptive Behaviors?", JOURNAL OF AUTISM AND DEVELOPMENTAL DISORDERS, KLUWER ACADEMIC PUBLISHERS-PLENUM PUBLISHERS, NE, vol. 42, no. 6, 7 July 2011 (2011-07-07), pages 929 - 936, XP035061727, ISSN: 1573-3432, DOI: 10.1007/S10803-011-1318-6
- J. SABINE BECKER ET AL: "Bioimaging of metals by laser ablation inductively coupled plasma mass spectrometry (LA-ICP-MS)", MASS SPECTROMETRY REVIEWS, 25 June 2009 (2009-06-25), pages 156 - 175, XP055186658, ISSN: 0277-7037, DOI: 10.1002/mas.20239
- POZEBON ET AL: "Monitoring of platinum in a single hair by laser ablation inductively coupled plasma mass spectrometry (LA-ICP-MS) after cisplatin treatment for cancer", INTERNATIONAL JOURNAL OF MASS SPECTROMETRY, ELSEVIER SCIENCE PUBLISHERS , AMSTERDAM, NL, vol. 272, no. 1, 12 January 2008 (2008-01-12), pages 57 - 62, XP022520200, ISSN: 1387-3806, DOI: 10.1016/J.IJMS.2008.01.001
- GAFTER-GVILI ANAT ET AL: "Posttransplantation Anemia in Kidney Transplant Recipients", vol. 142, no. 1, 10 April 2019 (2019-04-10), CH, pages 37 - 43, XP093049154, ISSN: 0001-5792, Retrieved from the Internet <URL:https://www.karger.com/Article/Pdf/496140> DOI: 10.1159/000496140
- GÎLCA-BLANARIU GEORGIANA-EMMANUELA ET AL: "New Insights into the Role of Trace Elements in IBD", vol. 2018, 6 September 2018 (2018-09-06), pages 1 - 9, XP093049153, ISSN: 2314-6133, Retrieved from the Internet <URL:http://downloads.hindawi.com/journals/bmri/2018/1813047.xml> DOI: 10.1155/2018/1813047
- PAUL CURTIN: "Recurrence quantification analysis to characterize cyclical components of environmental elemental exposures during fetal and postnatal development", PLOS ONE, vol. 12, no. 11, 7 November 2017 (2017-11-07), US, pages e0187049, XP93168700, ISSN: 1932-6203, DOI: 10.1371/journal.pone.0187049
- PAUL R WEST; AMARAL DAVID; BAIS PREETI; SMITH ALAN; EGNASH LAURA; ROSS MARK; PALMER JESSICA; FONTAINE BURR; CONARD KEVIN; CORBETT : "Metabolomics as a Tool for Discovery of Biomarkers of Autism Spectrum Disorder in the Blood Plasma of Children", PLOS ONE, 7 November 2014 (2014-11-07), pages e112445, XP055321069, Retrieved from the Internet <URL:https://www.ncbi.nlm.nih.gov/pmc/articles/PMC4224480> [retrieved on 20200927]
- ELNAZ GHADIMI, EIMAR HAZEM, MARELLI BENEDETTO, NAZHAT SHOWAN N, ASGHARIAN MASOUD, VALI HOJATOLLAH, TAMIMI FALEH: "Trace elements can influence the physical properties of tooth ename", SPRINGERPLUS, vol. 2, no. 1, 27 September 2020 (2020-09-27), pages 499, XP055769160, Retrieved from the Internet <URL:https://www.ncbi.nlm.nih.gov/pmc/articles/PMC3795877> [retrieved on 20200927]

## Description

### TECHNICAL FIELD

The present disclosure generally relates to diagnostics for biological conditions associated with metal metabolism through the analysis of biological samples from subjects tested for such biological conditions.

### BACKGROUND

Metal ions have an important role in many biological processes having structural and functional significance for humans. An imbalanced gain of certain metal ions, either due to the amount of certain metals in nutrition or metabolic dysregulation of certain metals, is associated with many biological conditions. The imbalance includes either an excessive gain of certain metal ions or a lack of certain metal ions. Examples of biological conditions associated with metal metabolism include neurological conditions (e.g., autism spectrum disorder, schizophrenia, or attention-deficit/hyperactivity disorder (ADHD)), neurodegenerative conditions (e.g., amyotrophic lateral sclerosis (ALS), Alzheimer's disease, Parkinson's disease, and Huntington's disease), and some cancers (e.g., pediatric cancer).

Recent studies have indicated a connection between autism spectrum disorder and metabolic dysfunctions, in particular metal dysregulation *(see,* for example, Cheng et al. in "Metabolic Dysfunction Underlying Autism Spectrum Disorder and Potential Treatment Approaches," Front Mol Neurosci. 10, p. 34, February 2017 and Arora et al. in "Fetal and postnatal metal dysregulation in Autism," Nat. Commun. 8, p. 15493, June 2017). As another example, recent studies have indicated a connection between neuronal degenerations and biologic rhythms of metal detectable from a hair and/or a tooth of a subject *(see,* for example, Appenzeller et al. in "Stable Isotope Ratios in Hair and Teeth Reflect Biologic Rhythms," PLoS ONE 2(7): e636. https://doi.org/10.1371/journal.pone.0000636, April 2017).

Gaudiuso et al. (Spectrochimica Acta 2018, 152: 123-148) discloses laser-induced breakdown spectroscopy (LISB) of biological samples and analysis of their quantitative elemental composition. Pozebon et al. (Analytica Chimica Acta 2017, 992: 1-23) discloses the elemental analysis of hair by ICP-MS in the context of different diseases. Curtin et al. (PLOS One 2017, 12(11): e0187049) discloses LA-ICP-MS analysis of the spatial distribution of metal metabolites representing different periods of growth in tooth samples.

Given the above background, what is needed in the art are improved systems and methods for accurate diagnosis of biological conditions associated with metal metabolism. In particular, there is a need for biomarkers detectable with non-invasive methods for diagnosis of the biological conditions associated with metal metabolism.

### SUMMARY

Accordingly, there is a demand for accurate methods and systems for the diagnosis of biological conditions associated with metal metabolism, and especially for non-invasive diagnosis. The present disclosure addresses these needs, for example, by providing a biological sample biomarker for diagnosis of biological conditions associated with metal metabolism. The biological sample includes a human biological specimen that includes deposits of certain metals and is associated with growth. Such a biological sample could be a hair shaft. The non-invasive biomarker of the present disclosure can be used for the diagnosis of young children, even infants younger than one year old.

In accordance with some embodiments, the invention relates to a method for evaluating a subject for a first biological condition associated with metal metabolism includes sampling, with a beam, each respective position in a plurality of positions along a reference line on a biological sample associated with metal metabolism of the subject, wherein the biological sample associated with metal metabolism of the subject is an *ex vivo* hair shaft sample, thereby obtaining a plurality of ion samples. Each ion sample in the plurality of ion samples corresponds to a different position in the plurality of positions, and each position in the plurality of positions represents a different period of growth of the biological sample associated with metal metabolism. The method includes analyzing each ion sample in the plurality of ion samples with an inductively coupled plasma mass spectrometer, thereby obtaining a first dataset that includes a plurality of traces. Each trace in the plurality of traces is a concentration of a corresponding elemental isotope, in a plurality of elemental isotopes, over time collectively determined from the plurality of ion samples. The method includes deriving a second dataset comprising a number and duration of recurrence from the plurality of traces that includes a set of features. Each respective feature in the set of features is determined by a variation of a single isotope or a combination of isotopes in the plurality of traces. The method includes inputting the set of features into a trained classifier thereby determining by the trained classifier that the subject has the first biological condition associated with metal metabolism with an accuracy of at least about 80%, wherein the first biological condition associated with metal metabolism is autism spectrum disorder (ASD).

In some embodiments, the plurality of elemental isotopes is selected from the elemental isotopes listed in Table 1. In some embodiments, the plurality of elemental isotopes includes at least 22 elemental isotopes of the elemental isotopes listed in Table 1.

In accordance with some embodiments, each feature in the set of features is associated with a single respective trace of the plurality of traces or with two respective traces of the plurality of traces. In some embodiments, the set of features is selected from the features listed in Table 2, and, optionally, the set of features further includes one or more features listed in Table 3. In some embodiments, the set of features includes at least 23 features listed in Table 2.

In some embodiments, evaluating the subject for a first biological condition associated with metal metabolism further includes discriminating between the first biological condition associated with metal metabolism and a second biological condition associated with metal metabolism distinct from the first biological condition associated with metal metabolism. In some embodiments, the second biological condition is attention-deficit/hyperactivity disorder (ADHD).

In some embodiments, the subject is a human. In some embodiments, the subject is less than 1 year old, less than 2 years old, less than 3 years old, less than 4 years old or less than 5 years old.

In some embodiments, the method further includes, prior to sampling the hair shaft of the subject, pretreating the hair shaft with a solvent and/or irradiating the hair shaft with a low powered laser to remove any debris from the hair shaft. In some embodiments, the reference line corresponds to a longitudinal direction of the hair shaft.

In some embodiments, the method further includes pretreating the biological sample associated with metal metabolism of the subject with a solvent or a surfactant prior to the sampling. In some embodiments, the method further includes irradiating, with a laser, the biological sample associated with metal metabolism of the subject with a low powered laser to remove any debris from the biological sample associated with metal metabolism of the subject prior to the sampling.

In some embodiments, the sampling includes irradiating, with a laser, the biological sample associated with metal metabolism of the subject with the laser thereby extracting a plurality of particles from the biological sample associated with metal metabolism of the subject and ionizing the plurality of particles with an inductively coupled plasma mass spectrometer, thereby obtaining the plurality of ion samples.

In some embodiments, the plurality of positions is sequenced such that a first position in the plurality of positions along the biological sample associated with metal metabolism of the subject corresponds to a position closest to a tip of the biological sample associated with metal metabolism of the subject. In some embodiments, the plurality of positions includes at least 100, 150, 200, 250, 300, 350, 400, 450, or 500 positions.

In some embodiments, each trace in the plurality of traces includes a plurality of data points. Each data point is an instance of the respective position in the plurality of position.

In some embodiments, the deriving the second dataset includes removing from the plurality of data points such data points that do not meet a first criteria. The first criteria includes a mean absolute difference between adjacent data points in the plurality of data points being three times a standard deviation of the mean absolute difference between adjacent points.

In some embodiments, the concentration of the corresponding elemental isotope corresponds to a relative abundance of the corresponding elemental isotope to a control elemental isotope, the control elemental isotope included in the plurality of ion samples. In some embodiments, the control elemental isotope is sulfur.

In some embodiments, the set of features is selected from a mean diagonal length, a determinism, a recurrence time, an entropy, a trapping time, and a laminarity.

In some embodiments, the trained classifier computes: $p \left(subject\right) = \frac{1}{1 + {e}^{- \left(α+{β}_{1}{x}_{1}+⋯+{β}_{k}{x}_{k}\right)}}$ where *p(subject)* is the probability that the subject has the first biological condition associated with metal metabolism, e is Euler's number, α is a calculated parameter associated with the probability that the subject has the biological condition associated with metal metabolism when *β*₁*x*₁ *+*··· *+βₖxₖ* equals to zero, *x*_{*1*, ..*. , k*} corresponds to a value derived for each feature in the set of features, the set of features including features from 1 through *k,* and *β_{1,...,k}* corresponds to a weight parameter associated with each feature in the set of features including features from 1 through k.

In some embodiments, the method further includes, in accordance with determining *that p(subject)* is above a predetermined threshold, deeming the subject to have the first biological condition associated with metal metabolism.

In some embodiments, the biological condition associated with metal metabolism is related to a periodic dysregulation of metabolism of a plurality of metals, the plurality of metals corresponding to the plurality of elemental isotopes.

In accordance with some embodiments, the invention relates to a device for evaluating a subject for a biological condition associated with metal metabolism comprising one or more processors, and memory storing one or more programs for execution by the one or more processors. The one or more programs include instructions for sampling, with a beam, each respective position in a plurality of positions along a reference line on a biological sample associated with metal metabolism of the subject, wherein the biological sample associated with metal metabolism of the subject is an *ex vivo* hair shaft sample, thereby obtaining a plurality of ion samples. Each ion sample in the plurality of ion samples corresponds to a different position in the plurality of positions. Each position in the plurality of positions represents a different period of growth of the biological sample associated with metal metabolism. The one or more programs include instructions for receiving a first dataset comprising a plurality of traces of each ion sample in the plurality of ion samples obtained with a laser ablation inductively coupled plasma mass spectrometer. Each trace in the plurality of traces being a concentration of a corresponding elemental isotope, in a plurality of elemental isotopes, over time collectively determined from the plurality of ion samples. The one or more programs include instructions for deriving a second dataset comprising a number and a duration of recurrence from the plurality of traces that includes a set of features, each respective feature in the set of features being determined by a variation of a single isotope or a combination of isotopes in the plurality of traces. The one or more programs include instructions for inputting the set of features into a trained classifier thereby determining by the trained classifier that the subject has the biological condition associated with metal metabolism with an accuracy of at least about 80%, wherein the first biological condition associated with metal metabolism is autism spectrum disorder (ASD).

In accordance with some embodiments, the invention relates to a non-transitory computer readable storage medium embeds one or more computer programs for classification. The one or more computer programs include instructions which, when executed by a computer system, cause the computer system to perform a method for evaluating a subject for a biological condition associated with metal metabolism. The method includes sampling, with a beam, each respective position in a plurality of positions along a reference line on a biological sample associated with metal metabolism of the subject, wherein the biological sample associated with metal metabolism of the subject is an *ex vivo* hair shaft sample, thereby obtaining a plurality of ion samples. Each ion sample in the plurality of ion samples corresponds to a different position in the plurality of positions, and each position in the plurality of positions represents a different period of growth of the biological sample associated with metal metabolism. The method includes receiving a first dataset comprising a plurality of traces of each ion sample in the plurality of ion samples obtained with a laser ablation inductively coupled plasma mass spectrometer. Each trace in the plurality of traces is a concentration of a corresponding elemental isotope, in a plurality of elemental isotopes, over time collectively determined from the plurality of ion samples. The method includes deriving a second dataset comprising a number and a duration of recurrence from the plurality of traces that includes a set of features. Each respective feature in the set of features is determined by a variation of a single isotope or a combination of isotopes in the plurality of traces. The method includes inputting the set of features into a trained classifier thereby determining by the trained classifier that the subject has the first biological condition associated with metal metabolism with an accuracy of at least about 80%, wherein the first biological condition associated with metal metabolism is autism spectrum disorder (ASD).

As disclosed herein, any embodiment disclosed herein when applicable can be applied to any aspect.

Additional aspects and advantages of the present disclosure will become readily apparent to those skilled in this art from the following detailed description, wherein only illustrative embodiments of the present disclosure are shown and described. As will be realized, the present disclosure is capable of other and different embodiments, and its several details are capable of modifications in various obvious respects, all without departing from the disclosure. Accordingly, the drawings and description are to be regarded as illustrative in nature, and not as restrictive.

### BRIEF DESCRIPTION OF THE DRAWINGS

Figure 1A illustrates a block diagram of an example computing device, in accordance with some embodiments of the present disclosure.
Figure 2A provides a flow chart of a method for evaluating a subject for a biological condition, in accordance with some embodiments of the present disclosure.
Figure 2B provides exemplary illustrations of a hair, a tooth, and a nail sample of a subject, in accordance with some embodiments of the present disclosure.
Figure 2C provides an exemplary schematic illustration of laser sampling a hair shaft of a subject, in accordance with some embodiments of the present disclosure.
Figure 2D provides an exemplary illustration of a trace describing a concentration of an elemental isotope over time, in accordance with some embodiments of the present disclosure.
Figure 2E provides exemplary illustrations of features corresponding to a variation of a single isotope derived from a trace, in accordance with some embodiments of the present disclosure.
Figure 2F provides an illustration of experimental data for discriminating between an autism spectrum disorder and other neurodevelopmental disorders, in accordance with some embodiments of the present disclosure. In Figure 2F, autism spectrum disorder (labeled ASD) cases are contrasted with attention-deficit/hyperactivity disorder (labeled ADHD) cases, subjects diagnosed with comorbid ASD and ADHD diagnoses (labeled CM), and neurotypical subjects (labeled NT) who have received no neurodevelopmental disorder diagnosis.
Figures 3A-3E collectively provide a flow chart of processes and features for evaluating a subject for a biological condition, in which optional blocks are indicated with dashed boxes, in accordance with some embodiments of the present disclosure.
Figure 4 provides a flow chart of processes and features for training a classifier to evaluate a subject for a biological condition, in which optional blocks are indicated with dashed boxes, in accordance with some embodiments of the present disclosure.
Figures 5A, 5B, 5C, and 5D illustrate experimental Receiver Operating Characteristic (ROC) curves for evaluating autism spectrum disorder, in accordance with some embodiments.
Figure 6 illustrates an ROC curve for evaluating accuracy of the disclosed method for evaluating amyotrophic lateral sclerosis, in accordance with some embodiments.
Figure 7 illustrates an ROC curve for evaluating accuracy of the disclosed method for evaluating schizophrenia, in accordance with some embodiments.
Figure 8 illustrates an ROC curve for evaluating accuracy of the disclosed method for evaluating irritable bowel disorder, in accordance with some embodiments.
Figure 9 illustrates an ROC curve for evaluating accuracy of the disclosed method for evaluating kidney transplant rejection, in accordance with some embodiments.
Figure 10 illustrates an ROC curve for evaluating accuracy of the disclosed method for evaluating pediatric cancer, in accordance with some embodiments.

Like reference numerals refer to corresponding parts throughout the several views of the drawings. The drawings are not drawn to scale.

### DETAILED DESCRIPTION

The invention is defined by the appended claims.

The present disclosure provides systems and methods for evaluating a subject for a biological condition associated with metal metabolism from a biological sample associated with metal metabolism of the subject. In particular, the disclosed methods provide for a biological sample biomarker for that can be obtained from a subject non-invasively. The method can be applied to evaluate subjects of any age, and is especially useful in diagnosis of small children, even infants under 1 year of age, to enable early treatment and intervention.

### Definitions.

The terminology used in the present disclosure is for the purpose of describing particular embodiments only and is not intended to be limiting of the invention. As used in the description of the invention and the appended claims, the singular forms "a", "an" and "the" are intended to include the plural forms as well, unless the context clearly indicates otherwise. It will also be understood that the term "and/or" as used herein refers to and encompasses any and all possible combinations of one or more of the associated listed items. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

As used herein, the term "if" may be construed to mean "when" or "upon" or "in response to determining" or "in response to detecting," depending on the context. Similarly, the phrase "if it is determined" or "if [a stated condition or event] is detected" may be construed to mean "upon determining" or "in response to determining" or "upon detecting [the stated condition or event]" or "in response to detecting [the stated condition or event]," depending on the context.

As used herein, a biological condition associated with metal metabolism (also called a metal metabolism disorder) herein refers to a biological condition that is related to, or caused by, a periodic dysregulation of metabolism of certain metals. The periodic dysregulation may be manifested as periodic decrease in an uptake (e.g., deficiency) of one or more metals, as periodic increase in the uptake of one or more metals, or as a combination of periodic decrease and periodic increase in the uptake of the one or more metals. Non-limiting examples of biological conditions associated with metal metabolism include autism spectrum disorder (ADS), attention-deficit/hyperactivity disorder (ADHD), amyotrophic lateral sclerosis (ALS), schizophrenia, kidney transplant rejection, some types of cancer, Alzheimer's disease, Parkinson's disease, Huntington's disease, metabolic disorders (obesity and irritable bowel disease (IBD)), and/or any conditions or disorders associated with metal metabolism.

As used herein, a biological sample associated with metal metabolism refers herein to a human biological specimen that includes deposits of certain metals and is associated with growth (e.g., hair, nails, and teeth). The biological samples associated with metal metabolism of the present disclosure have a requirement of expressing growth along a reference line such that abundance of the deposits of certain metals are detectable with respect to time. These biological samples associated with metal metabolism thereby facilitate detection of periodic variations in abundance of the certain metals. In some embodiments, the biological sample associated with metal metabolism includes a hair shaft where a reference line corresponds to a line along the longitudinal direction of the hair shaft. In some embodiments, the biological sample associated with metal metabolism includes a tooth where a reference line corresponds to a neonatal line of the tooth on an enamel surface of the tooth. In some embodiments, the biological sample associated with metal metabolism includes a nail where a reference line corresponds to a line in direction of growth of the nail. For example, the reference line extends from the nail root toward the tip of the nail.

As used herein, the term "trained classifier" refers to a model *(e.g.,* a machine learning algorithm, such as logistic regression, neural network, regression, support vector machine, clustering algorithm, decision tree *etc.)* with specific parameters (weights) and thresholds, ready to be applied to previously unseen samples.

As used herein, the term "untrained classifier or partially trained classifier" refers to a model *(e.g.,* a machine learning algorithm, such as logistic regression, neural network, regression, support vector machine, clustering algorithm, decision tree *etc*.) with at least some unfixed parameters (weights) and thresholds, ready to be trained on a training set in order to optimize and fix the parameters and thresholds.

It will also be understood that, although the terms first, second, *etc.* may be used herein to describe various elements, these elements should not be limited by these terms. These terms are only used to distinguish one element from another. For example, a first subject could be termed a second subject, and, similarly, a second subject could be termed a first subject, without departing from the scope of the present disclosure. The first subject and the second subject are both subjects, but they are not the same subject. Furthermore, the terms "subject," "user," and "patient" are used interchangeably herein.

As used herein, the term "subject" refers to a human (e.g., a male human, female human, fetus, pregnant female, child, or the like). In some embodiments, a subject is a male or female of any stage *(e.g.,* a man, a women or a child).

As used herein, the term "autism spectrum disorder" refers to a range of neurodevelopmental conditions associated with impairments in social interactions, developmental language and communication skills and repetitive behaviors. For example, standardized criteria for diagnosis of autism spectrum disorder by Centers of Disease Control and Prevention (CDC) includes 1) persistent deficits in social communication and social interaction and 2) restricted, repetitive patterns of behavior, interests, or activities. Autism spectrum disorder includes, for example, autistic disorder (a.k.a. "classic autism"), Asperger's Syndrome, and Pervasive Developmental Disorder (a.k.a. "atypical" autism).

As used herein, the term "recurrence quantification analysis" ("RQA") refers to a non-linear data analysis that quantifies a number and duration of recurrences in dynamical systems. RQA is used for characterizing a dynamic system's behavior in a phase space.

As used herein, the term "recurrence plot" refers to a graphical visualization of time-dependent periodical structures in an experimental data.

As used herein, the term "trace" refers to a time-dependent abundance (or concentration) of an elemental isotope. The trace includes a plurality of data points, where each data point is associated with a temporal measure and an abundance measure.

As used herein, the term "feature," refers to a dynamical periodical feature extracted from a time-dependent abundance trace of an elemental isotope, or a combination of two or more time-dependent abundance traces of elemental isotopes, e.g., by using RQA.

As used herein, the term "mean diagonal length" ("MDL") refers to a critical measure derived from RQA, reflecting a straightforward measurement of an average length of diagonal lines present in a two-dimensional recurrence plot. This measure can be taken as an absolute indicator of the duration of periodic components in a given signal.

As used herein, the term "determinism," which is related to the mean diagonal length, refers to a relative ratio of periodic components to non-periodic components in a recurrence analysis. The determinism indicates an overall periodic content of a given signal.

As used herein, the term "recurrence time" ("RT2") refers to a mean time interval between diagonal elements, i.e. the interval between periodicities.

As used herein, the term "entropy" refers to a variability in the distribution of mean diagonal lengths, with low entropy signals exhibiting little complexity in a distribution of periodic components, and high entropy signals exhibiting diversity in short- and long-duration periodicities.

As used herein, the term "trapping time" ("TT") refers to a mean length of laminar (vertical or horizontal) structures in a two-dimensional recurrence plot, which indicate stable states, analogous to how mean diagonal length captures the duration of periodic processes.

As used herein, the term "laminarity" refers to an overall measure of signal stability. Laminarity quantifies a ratio of recurrence points belonging to laminar structures against the total frequency of recurrence points.

The terminology used herein is for the purpose of describing particular cases only and is not intended to be limiting. As used herein, the singular forms "a," "an" and "the" are intended to include the plural forms as well, unless the context clearly indicates otherwise. Furthermore, to the extent that the terms "including," "includes," "having," "has," "with," or variants thereof are used in either the detailed description and/or the claims, such terms are intended to be inclusive in a manner similar to the term "comprising."

Several aspects are described below with reference to example applications for illustration. It should be understood that numerous specific details, relationships, and methods are set forth to provide a full understanding of the features described herein. One having ordinary skill in the relevant art, however, will readily recognize that the features described herein can be practiced without one or more of the specific details or with other methods. The features described herein are not limited by the illustrated ordering of acts or events, as some acts can occur in different orders and/or concurrently with other acts or events. Furthermore, not all illustrated acts or events are required to implement a methodology in accordance with the features described herein.

Reference will now be made in detail to embodiments, examples of which are illustrated in the accompanying drawings. In the following detailed description, numerous specific details are set forth in order to provide a thorough understanding of the present disclosure. However, it will be apparent to one of ordinary skill in the art that the present disclosure may be practiced without these specific details. In other instances, well-known methods, procedures, components, circuits, and networks have not been described in detail so as not to unnecessarily obscure aspects of the embodiments.

### Example System Embodiments.

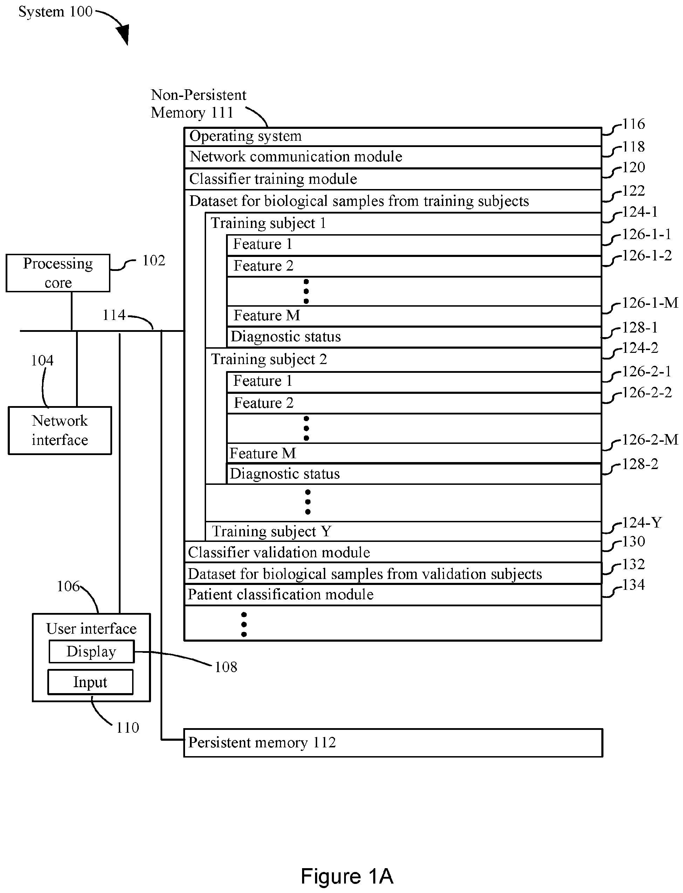

Now that an overview of some aspects of the present disclosure has been provided, details of an exemplary system are now described in conjunction with Figure 1 Figure 1A illustrates a block diagram of an example computing device 100, in accordance with some embodiments of the present disclosure. The device 100 in some implementations includes one or more processing units CPU(s) 102 (also referred to as processors), one or more network interfaces 104, a user interface 106, a non-persistent memory 111, a persistent memory 112, and one or more communication buses 114 for interconnecting these components. The one or more communication buses 114 optionally include circuitry (sometimes called a chipset) that interconnects and controls communications between system components. The non-persistent memory 111 typically includes high-speed random access memory, such as DRAM, SRAM, DDR RAM, ROM, EEPROM, flash memory, whereas the persistent memory 112 typically includes CD-ROM, digital versatile disks (DVD) or other optical storage, magnetic cassettes, magnetic tape, magnetic disk storage or other magnetic storage devices, magnetic disk storage devices, optical disk storage devices, flash memory devices, or other non-volatile solid state storage devices. The persistent memory 112 optionally includes one or more storage devices remotely located from the CPU(s) 102. The persistent memory 112, and the non-volatile memory device(s) within the non-persistent memory 112, comprise non-transitory computer readable storage medium. In some implementations, the non-persistent memory 111 or alternatively the non-transitory computer readable storage medium stores the following programs, modules and data structures, or a subset thereof, sometimes in conjunction with the persistent memory 112:
- an optional operating system 116, which includes procedures for handling various basic system services and for performing hardware dependent tasks;
- an optional network communication module (or instructions) 118 for connecting the system 100 with other devices and/or a communication network 104;
- an optional classifier training module 120 for training classifiers for evaluating a subject for a biological condition associated with metal metabolism;
- an optional data store for datasets for biological samples from training subjects 122 including feature data for one or more training subjects 124, where the feature data includes a parameter associated with each of features 126, and diagnostic status 128 *(e.g.,* an indication that a respective training subject has been diagnosed with a biological condition associated with metal metabolism or has not been diagnosed with a biological condition associated with metal metabolism);
- an optional classifier validation module 130 for validating classifiers that distinguish the a biological condition associated with metal metabolism;
- an optional data store for datasets for biological samples from validation subjects 132; and
- an optional patient classification module 134 for classifying a subject as having a biological condition associated with metal metabolism, e.g., as trained using classifier training module 120.

In various implementations, one or more of the above identified elements are stored in one or more of the previously mentioned memory devices, and correspond to a set of instructions for performing a function described above. The above identified modules, data, or programs (e.g., sets of instructions) need not be implemented as separate software programs, procedures, datasets, or modules, and thus various subsets of these modules and data may be combined or otherwise re-arranged in various implementations. In some implementations, the non-persistent memory 111 optionally stores a subset of the modules and data structures identified above. Furthermore, in some embodiments, the memory stores additional modules and data structures not described above. In some embodiments, one or more of the above identified elements is stored in a computer system, other than that of visualization system 100, that is addressable by visualization system 100 so that visualization system 100 may retrieve all or a portion of such data when needed.

In some embodiments, the system 100 is connected to, or includes, one or more analytical devices for performing chemical analyzes. For example, the optional network communication module (or instructions) 118 is configured to connect the system 100 with the one or more analytical devices, *e.g.,* via the communication network 104. In some embodiments, the one or more analytical devices include a laser ablation-inductively coupled-plasma mass spectrometer (LA-ICP-MS).

Although Figure 1 depicts a "system 100," the figure is intended more as functional description of the various features which may be present in computer systems than as a structural schematic of the implementations described herein. In practice, and as recognized by those of ordinary skill in the art, items shown separately could be combined and some items could be separated. Moreover, although Figure 1 depicts certain data and modules in non-persistent memory 111, some or all of these data and modules may be in persistent memory 112.

### Classification Methods.

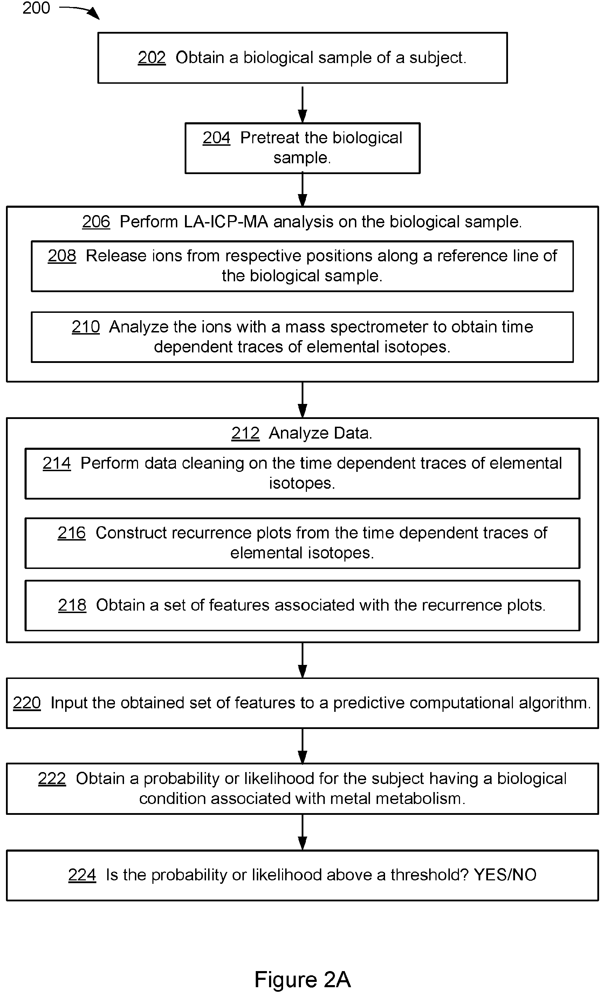

While a system in accordance with the present disclosure has been disclosed with reference to Figure 1, detailed processes and features of a method 200 for evaluating a subject for a biological condition associated with metal metabolism from a biological sample in accordance with the present disclosure is provided in conjunction with Figures 2A-2F.

As defined above, a biological sample associated with metal metabolism (also called here "a biological sample") includes a human biological specimen that with deposits of certain metals and is associated with growth (e.g., hair, nails, and teeth). The biological samples associated with metal metabolism of the present disclosure have a requirement of expressing growth along a reference line such that abundance of the deposits of certain metals are detectable with respect to time. According to the invention, the biological sample associated with metal metabolism includes a hair shaft where a reference line corresponds to a line along the longitudinal direction of the hair shaft.

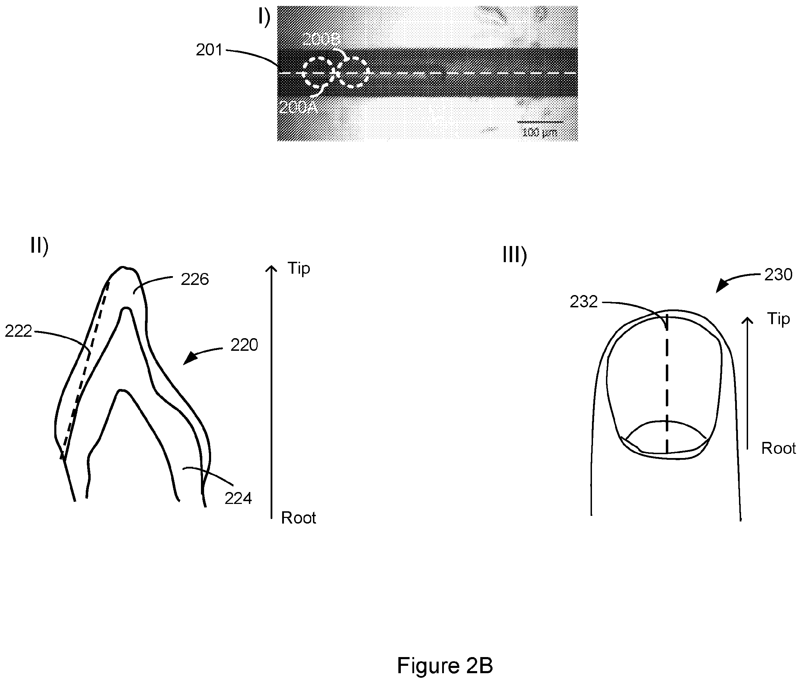

In some embodiments, the method 200 includes obtaining (202) a biological sample *(e.g.,* a strand of hair including a hair shaft). The subject is a human. In some embodiments, the subject is a child aged equal to or below 5 years (e.g., the child is aged equal to or below 5 years, 4 years, 3 years, 2 years, 1 year, 9 months, 6 months, 3 months, or 1 month). In some embodiments, the subject is an adult. Figure 2B section I provides an exemplary image of a hair sample of a subject including a hair shaft, in accordance with some embodiments of the present disclosure. The hair sample may be simply cut from the subject (e.g., with help of scissors). The method of obtaining the hair sample is therefore non-invasive. The obtained hair sample has a minimum length of 1 cm *(e.g.,* the hair sample is 1 cm, 2 cm, 3 cm, 4 cm, or 5 cm long). The hair sample may include any portion of a hair *(e.g.,* a tip or a portion between the tip and a follicle). In particular, there is no special requirement for the hair sample to include the hair follicle. Figure 2B section II provides an exemplary image of a tooth sample of a subject, in accordance with some embodiments of the present disclosure that are not part of the invention. Figure 2B section III provides an exemplary image of a nail sample of a subject, in accordance with some embodiments of the present disclosure that are not part of the invention. In instances of a tooth or a hair, obtaining a biological sample refers to positioning the subject such that the tooth or the nail could be sampled.

In some embodiments, the obtained biological sample is pretreated (204) by washing the biological sample with one or more solvents and/or surfactants and drying. In an instance that the biological sample is a hair, the hair sample is washed in TRITON X-100^{®} and ultrapure metal free water *(e.g.,* MILLI-Q^{®} water) and dried overnight in an oven *(e.g.,* at 60 degrees Celsius). The pretreatment further includes preparing the hair shaft for a measurement by placing the hair shaft on a glass slide *(e.g.,* a microscopic glass slide) with an adhesive film *(e.g., a* double sided tape). The hair shaft is positioned such that the hair shaft is substantially straight. The glass slide with the hair shaft is then placed into a laser ablation-inductively coupled-plasma mass spectrometer (LA-ICP-MS) for performing analysis (206). In an instance that the biological sample is a tooth or a nail, a surface of the biological sample is cleaned (e.g., by surfactant, water, or one or more solvents). The subject is positioned in vicinity of a LA-ICP-MS for performing the analysis.

In some embodiments, the LA-ICP-MS analyses includes pre-ablating the biological sample to remove surface debris and/or impurities from the biological sample. The pre-ablation is performed using such a low laser energy that it only releases particles on the surface of the biological sample but does not release particles from below the surface of the biological sample. For example, the pre-ablation is performed using a laser wavelength of 193 nm and laser energy below 0.4 J/cm² (*e.g.,* the laser energy is 0.4 J/cm², 0.3 J/cm², 0.2 J/cm² or 0.1 J/cm²). In some embodiments, the laser energy ranges from 0.2 J/cm² to 0.4 J/cm².

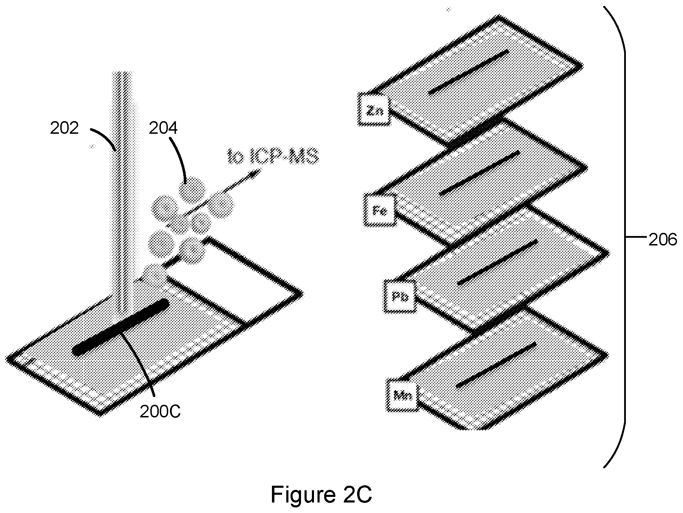

After pre-ablation, method 200 includes sampling the biological sample with a laser to obtain ion samples (208) from respective positions along a reference line of the biological sample. As explained above, in an instance of a hair shaft the reference line corresponds to a line along the longitudinal direction of the hair shaft. For example, Figure 2B section A illustrates a hair shaft with reference line 201 along the longitudinal direction of the hair shaft. In an instance of a tooth (not part of the invention), the reference line corresponds to a neonatal line of the tooth on an enamel surface of the tooth. For example, Figure 2B section II illustrates tooth 220 including portions of enamel 226 and primary dentine 224. Reference line 222 corresponds to a neonatal line of tooth 220. A neonatal line herein refers to a particular band of incremental growth lines on an enamel portion of a tooth. In an instance of a nail (not part of the invention), the reference line corresponds to a line in direction of growth of the nail. For example, Figure 2B section II illustrates nail 230 with reference line 232 extending from the nail root toward the tip of the nail. The sampling includes irradiating the biological sample with a laser beam (e.g., laser ablating the hair shaft) and ionizing the plurality of particles with an inductively coupled plasma mass spectrometer. For example, areas 200A and 200B in Figure 2B section I correspond to exemplary positions along the hair shaft that are irradiated with a laser during the laser ablation. The mass spectrometer analyzes (210) the obtained ion samples from each respective position. Figure 2C provides an exemplary schematic illustration of laser sampling a hair shaft of a subject, in accordance with some embodiments of the present disclosure. Laser 202 in Figure 2C irradiates an area 200C on the hair shaft thereby releasing particles 204. The particles 204 are ionized by an inductively-coupled-plasma (ICP), and further analyzed by a mass spectrometer (MS).

In some embodiments, the laser irradiation is performed using a laser having wavelength 193 nm and laser energy ranging from 0.6 to 1.5 J/cm² *(e.g.,* the laser energy is 0.6 J/cm², 0.7 J/cm², 0.8 J/cm², 0.9 J/cm², 1.0 J/cm², 1.1 J/cm², 1.2 J/cm², 1.3 J/cm², 1.4 J/cm², or 1.5 J/cm²). In some embodiments, the laser energy ranges from 0.9 to 1.3 J/cm². In some embodiments, the laser has a beam diameter ranging from 25 micrometers to 35 micrometers *(e.g.,* 25, 27.5, 30, 32.5, or 35 micrometers). In some embodiments, the laser has a beam diameter of 30 micrometers. In an instance of sampling a hair shaft, the laser beam size, wavelength and/or laser energy are adjusted such that the laser sampling ablates most of the hair shaft without releasing any particles from the adhesive film and/or the glass slide holding the hair shaft.

The laser irradiation is repeated, and elemental isotope data is collected, sequentially at a plurality of positions along the biological sample (e.g., the areas 200A and 200B of the hair shaft in Figure 2B section I). In some embodiments, the plurality of positions along the reference line of the biological sample includes at least 100 positions (e.g., 100, 150, 200, 250, 300, 350, 400, 450, or 500 positions). In some embodiments, the respective positions (e.g., areas 200A and 200B in Figure 2B section I) are adjacent to each other. By this method, each area corresponding to a distinct position on the biological sample (e.g., areas 200A and 200B) is thereby associated with an abundance of elemental isotopes (e.g., metal isotopes Zn, Fe, Pb, and Mn shown in Figure 2C). In some embodiments, the respective positions are separated by a predefined distance. In some embodiments, the sampling is performed along the reference line of the biological sample starting from a respective position nearest to the tip of the hair *(e.g.,* at a position that corresponds to the youngest age of the subject). In general, the sampling can be performed starting from a respective position nearest to the tip or the root, as long as the direction of the sampling is known and an appropriate trained classifier is used for the analyses.

The laser sampling thereby produces sets of data points. Each set of data points corresponds to an abundance (e.g., a concentration) of a respective elemental isotope measured at a plurality of positions along the biological sample. Each position on the reference line of the biological sample corresponds to a specific time of growth of the biological sample. In some embodiments, in an instance of the hair shaft, each position corresponds to approximately 130 min period of hair growth (e.g., the period of hair growth calculated using a 30 micrometer laser beam size and an average rate of hair growth 1 cm per month). By correlating the plurality of positions along the reference line of the biological sample to corresponding time periods of the growth, a first dataset including a plurality of traces is obtained. Each trace includes a time-dependent abundance of a respective elemental isotope measured from the biological sample.

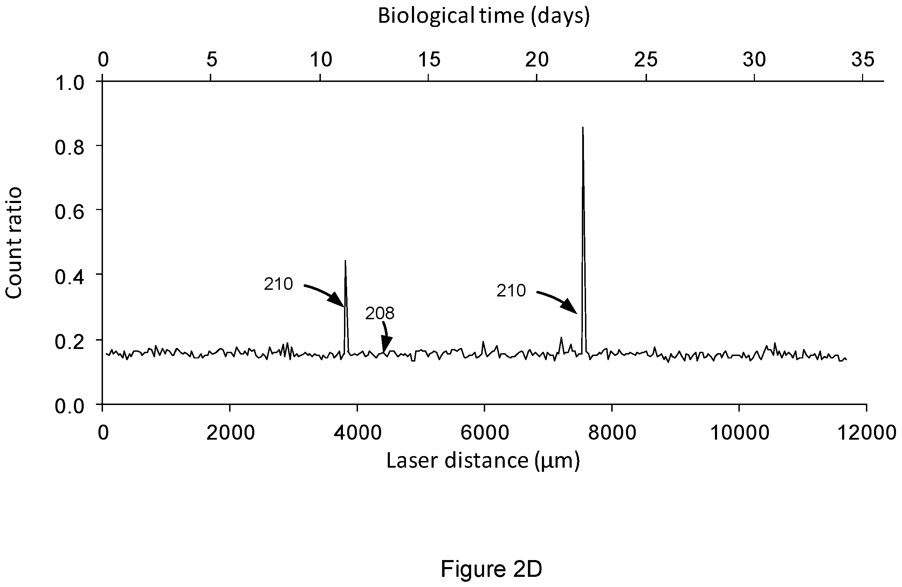

Figure 2D provides an exemplary illustration of a trace 208, in accordance with some embodiments of the present disclosure. Each data point in Figure 2D corresponds to an abundance (*i*.*e*., count ratio on the y-axis) of a particular elemental isotope measured at a plurality of positions along a biological sample *(i.e.,* laser distance on the bottom x-axis). The distance moved by the laser along the biological sample corresponds to an estimated growth of the biological sample *(i.e.,* biological time), as is illustrated on the top x-axis. For example, Figure 2D illustrates the abundance of a particular elemental isotope measured for a hair along a 1.2 cm (12 000 micrometers) distance. Such distance corresponds to a biological time of approximately 35 days. The biological time is estimated by using an average rate of hair growth *(e.g.,* 1 cm per month).

In some embodiments, the plurality of elemental isotopes is selected from the elemental isotopes listed in Table 1. In some embodiments, the plurality of elemental isotopes includes at least 50%, 60%, 70%, 80% or 90% of the isotopes included in Table 1.

**Table 1. List of Elemental Isotopes**

| **Elemental Isotope** | **Element Name** |
|---|---|
| Li-7 (Li) | lithium |
| Mg-24 (Mg) | magnesium |
| Mg-25 (Mg25) | magnesium |
| Al-27 (Al) | aluminum |
| P-31 (P) | phosphorus |
| S-34 (S) | sulfur |
| Ca-44 (Ca) | calcium |
| Ca-43 (Ca43) | calcium |
| Cr-52 (Cr) | chromium |
| Mn-55 (Mn) | manganese |
| Fe-56 (Fe) | iron |
| Co-59 (Co) | cobalt |
| Ni-60 (Ni) | nickel |
| Cu-63 (Cu) | copper |
| Zn-66 (Zn) | zinc |
| As-75 (As) | arsenic |
| Sr-88 (Sr) | strontium |
| Cd-111 (Cd) | cadmium |
| Sn-118 (Sn) | tin |
| I-127 (I) | iodine |
| Ba-138 (Ba) | barium |
| Hg-201 (Hg) | mercury |
| Pb-208 (Pb) | lead |
| Bi-209 (Bi) | bismuth |
| Mo-95(Mo) | molybdenum |

In some embodiments, the method 200 includes analyzing (212) the first dataset including the obtained plurality of traces where each trace corresponds to a time-dependent abundance (*e.g*., a time-dependent concentration) of a respective elemental isotope. In some embodiments, the analyzing the data includes performing customized operations to clean the data (214). In some embodiments, cleaning the data includes smoothening the data over a time span, and/or removing data points that are higher or lower than a predetermined threshold. In some embodiments, the data analyzing includes removing, from the traces, data points that have a mean absolute difference between adjacent data points that is three times a standard deviation of the mean absolute difference between adjacent points. Figure 2D illustrates an operation to remove data points that are higher than a predetermined threshold. Peaks 210 correspond to data points that have a mean absolute difference between adjacent data points that is more than three times the standard deviation of the mean absolute difference between adjacent points. The peaks 210 are therefore removed from the trace 208.

In some embodiments, the analyzing the data set further includes normalizing each trace against an internal standard. In some embodiments, in an instance where the sample is a hair shaft, the internal standard is sulfur which is the most abundant of the elemental isotopes in hair and therefore can be used as a measure of hair density and/or hardness. However, in practice, any element detected in the samples that is evenly incorporated during the development/growth of a biological sample that does not fluctuate with environmental exposures (*e.g.,* diet) can serve as an internal standard including any of the elements disclosed in the table of the present disclosure. For example, in the case where the sample is a tooth (nor part of the invention), Bismuth-209 can be used an in internal standard.

The method 200 includes performing recurrence quantification analysis (RQA) to analyze the first data set which includes time-dependent traces of elemental isotopes to obtain a set of features that describe dynamical periodical characteristics of the traces. RQA measures variability in the time-dependent traces of elemental isotopes. RQA involves the estimation of features that describe periodic properties in a given waveform, which include the determinism, mean diagonal length, and entropy. Methods and features of RQA are described, for example, by Webber et al. in "Simpler Methods Do It Better: Success of Recurrence Quantification Analysis as a General Purpose Data Analysis Tool," Physics Letters A 373, 3753-3756 (2009) and by Marwan et al. in "Recurrence Plots for the Analysis of Complex Systems," Physics Reports 438, 237-239 (2007). In some embodiments, the time-dependent traces of elemental isotopes are analyzed by using other analytical methods known in the art, such as Fourier Transformations, Wavelet Analysis, and Cosinor analysis. Such method can be applied to derive similar metrics, including spectral analysis of frequency components and their associated power. These metrics and associated derivative measures may be used in place of the features derived from RQA to analyze the time-dependent traces of elemental isotopes obtained from biological samples for purposes of predictive classification.

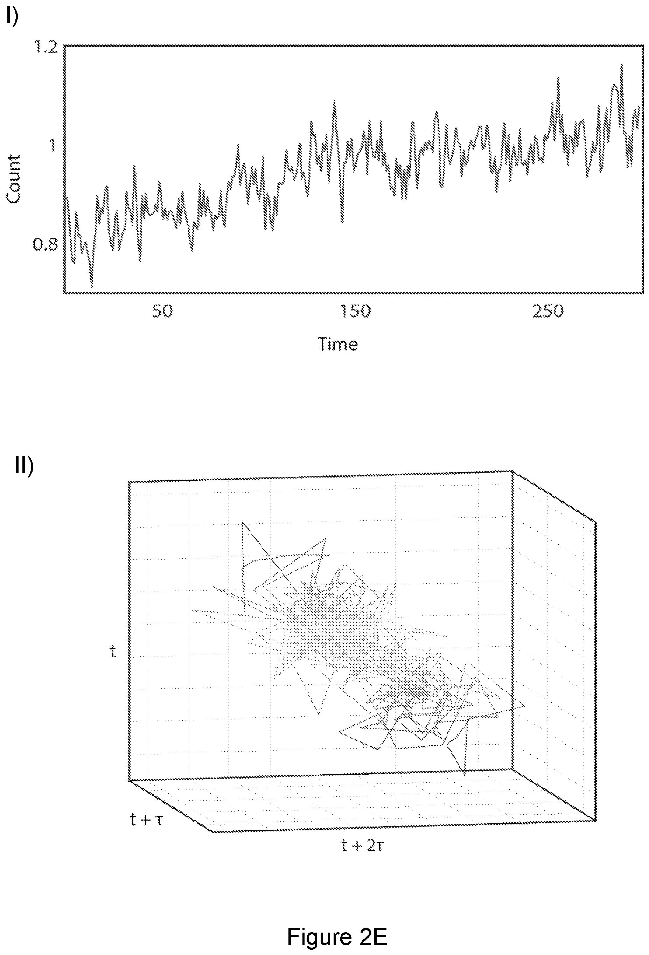

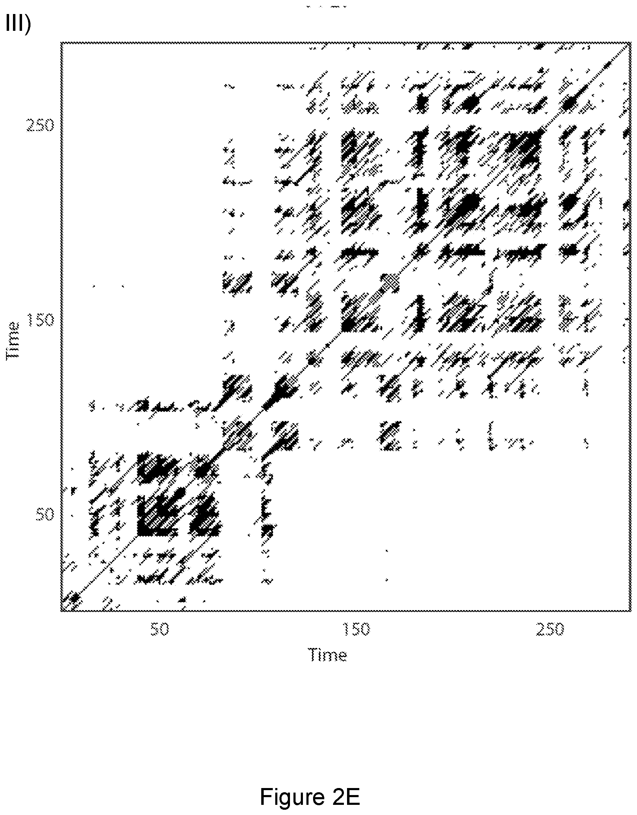

The RQA includes construction of recurrence plots (216) that visualize and analyze dynamical temporal structures in respective obtained traces. Figure 2E provides exemplary illustrations of a variation of an abundance of a single isotope derived from a respective trace, in accordance with some embodiments of the present disclosure. Section I of Figure 2E illustrates a trace corresponding to a time-dependent abundance (or concentration) of copper (Cu) as measured from the hair shaft of the subject. The y-axis illustrates measured abundance of copper, and the x-axis illustrates sequential measurements along a hair shaft, which reflect longitudinal increments of time. Section II of Figure 2E is a phase portrait derived from the trace of Section I. From the one dimensional trace measured from the hair shaft, additional dimensions are computationally derived to embed the trace in a higher dimensional space referred to as a phase portrait, where t refers to the values of the original trace, and dimensions (t+τ) and (t+2τ) are derived from lagging the original time series by interval τ. Subsequent analyses are then undertaken on the embedded phase portrait to construct recurrence plots and recurrence quantification analysis. Section III illustrates a recurrence quantification plot of the copper isotope derived from the phase portrait illustrated in Section II. The RQA method examines the interval of delay between states in a given system, with a black point reflecting the temporal interval when a system revisits the same state. Periodic processes, where a system successively reiterates a given pattern of states, will manifest in a recurrence plot as diagonal black lines, whereas periods of stability will manifest as square structures, spurious repetitions as black dots, and, unique events as white space.

In some embodiment, the recurrence plots are constructed for traces of a single elemental isotope or a combination of two elemental isotopes (e.g., for elemental isotopes selected from Table 1.) For example, Figure 2E illustrates a recurrence plot of copper isotope. Alternatively, a recurrence plot is constructed to visualize an interactive periodic pattern of two elemental isotopes. In some embodiments, the recurrence plots are constructed for a combination of three or more elemental isotopes.

The method 200 further includes analyzing the recurrence plots to obtain (218) a set of features associated with the recurrence plots. The features, which interchangeably can be termed "rhythmicity features," or "dynamic features," provide a quantitative measure describing the periodicity present in the plurality of traces. The features are selected from a mean diagonal length (MDL), determinism (or predictability), recurrence time (RT), entropy, trapping time (TT), and laminarity. Definitions of each of these feature types are provided above in the Definitions section.

In some embodiments, the set of features, where each feature is associated with a respective elemental isotope or a combination of elemental isotopes *(e.g.,* a combination of two elemental isotopes, or a combination of more than two element isotopes), is selected from the features listed in Table 2.

In some embodiments, the set of features includes all the features listed in Table 2.

In some embodiments, the set of features includes at least 50 %, 60 %, 70 %, 80 % or 90 % of the features listed in Table 2. In some embodiments, the features drawn from Table 2 in this manner, are considered to be the "core" features for evaluating a subject for a first biological condition (e.g., autism spectrum disorder, *etc.),* in accordance with the present disclosure. In some embodiments, the set of features further includes one or more features listed in Table 3 (in addition to the core features).

**Table 2. List of features associated with their respective elemental isotopes or respective combination of two elemental isotopes.**

| **Feature** | **Elemental Isotope name** |
|---|---|
| Determinism (Determinism_Cd) | Cd |
| Determinism (Determinism_Cr) | Cr |
| Determinism (Determinism_ZnHg) | ZnHg |
| Determinism (Determinism_Cu) | Cu |
| Determinism (Determinism_ZnMn) | ZnMn |
| Determinism (Determinism_Sr) | Sr |
| Entropy (Entropy_As) | As |
| Determinism (Determinism_Mg) | Mg |
| Entropy (Entropy_Li) | Li |
| Determinism (Determinism_ZnCu) | ZnCu |
| Entropy (Entropy_ZnCu) | ZnCu |
| Mean Diagonal length (MDL_ZnCu) | ZnCu |
| Determinism (Determinism_Ca) | Ca |
| Determinism (Determinism_Mn) | Mn |
| Determinism (Determinism_Ni) | Ni |
| Determinism (Determinism_ZnMg) | ZnMg |
| Determinism (Determinism_ZnCr) | ZnCr |
| Determinism (Determinism _Pb) | Pb |
| Determinism (Determinism_ZnNi) | ZnNi |
| Determinism (Determinism_ZnSn) | ZnSn |
| Determinism (Determinism_Li) | Li |
| Determinism (Determinism_Hg) | Hg |
| Determinism (Determinism_Fe) | Fe |
| Determinism (Determinism_As) | As |
| Determinism (Determinism_ZnI) | ZnI |

**Table 3. List of additional features associated with their respective elemental isotopes or respective combination of two elemental isotopes.**

| **Feature** | **Elemental Isotope name** |
|---|---|
| Entropy (Entropy_Ni) | Ni |
| Determinism (Determinism_Bi) | Bi |
| Laminarity (Laminarity_Li) | Li |
| Determinism (Determinism_ZnSr) | ZnSr |
| MDL (MDL_As) | As |
| Determinism (Determinism_ZnAs) | ZnAs |
| Determinism (Determinism_ZnCd) | ZnCd |
| Determinism (Determinism_ZnS) | ZnS |
| Determinism (Determinism_ZnCa) | ZnCa |
| Determinism (Determinism_ZnPb) | ZnPb |
| Determinism (Determinism_ZnFe) | ZnFe |
| Determinism (Determinism_S) | S |
| Entropy (Entropy_Cu) | Cu |
| Entropy (Entropy_Sr) | Sr |
| Entropy (Entropy_Pb) | Pb |
| Entropy (Entropy_Ca) | Ca |
| MDL (MDL_Ni) | Ni |
| MDL (MDL_Li) | Li |
| Entropy (Entropy_P) | P |
| Laminarity (Laminarity_As) | As |
| Entropy (Entropy_Cr) | Cr |
| Laminarity (Laminarity_Mn) | Mn |
| Laminarity (Laminarity_Cd) | Cd |
| Entropy (Entropy_Co) | Co |
| Laminarity ^{.}(Laminarity_Mg) | Mg |
| Entropy (Entropy_Cd) | Cd |
| Entropy (Entropy_Mg) | Mg |
| TT (TT_Pb) | Pb |
| Entropy (Entropy_Sn) | Sn |
| Entropy (Entropy_ZnCd) | ZnCd |
| TT (TT_P) | P |
| Laminarity (Laminarity_Cu) | Cu |
| TT (TT_Zn) | Zn |
| Laminarity (Laminarity_Sn) | Sn |
| MDL (MDL_P) | P |
| MDL (MDL_ZnCd) | ZnCd |
| Laminarity (Laminarity_Fe) | Fe |
| Laminarity (Laminarity_Co) | Co |
| MDL (MDL_Pb) | Pb |
| TT (TT_As) | As |
| MDL (MDL_Sr) | Sr |
| MDL (MDL_Cd) | Cd |
| MDL (MDL_Ca) | Ca |
| Determinism (Determinism_ZnLi) | ZnLi |
| MDL (MDL_Cu) | Cu |
| Laminarity (Laminarity_Pb) | Pb |
| Laminarity (Laminarity_Bi) | Bi |
| Entropy (Entropy_Mn) | Mn |
| MDL (MDL_Cr) | Cr |
| MDL (MDL_Mg) | Mg |
| TT (TT_Mn) | Mn |
| TT (TT_S) | S |
| MDL (MDL_Sn) | Sn |
| Determinism (Determinism _ZnAl) | ZnAl |
| TT (TT_Mg) | Mg |
| MDL (MDL_ZnAs) | ZnAs |
| RT2 (RT2_Mn) | Mn |
| TT (TT_Li) | Li |
| TT (TT_Sr) | Sr |
| Entropy (Entropy_ZnMn) | ZnMn |
| MDL (MDL_Co) | Co |
| Determinism (Determinism_Co) | Co |
| TT (TT_Ca) | Ca |
| TT (TT_Cd) | Cd |
| RT2 (RT2_Ni) | Ni |
| TT (TT_Fe) | Fe |
| RT (RT_Fe) | Fe |
| MDL (MDL_ZnBi) | ZnBi |
| RT2 (RT2_ZnAl) | ZnAl |
| RT2 (RT2_Zn) | Zn |
| RT2 (RT2_Al) | Al |
| MDL (MDL_ZnMn) | ZnMn |
| Laminarity (Laminarity_Zn) | Zn |
| TT (TT_Cu) | Cu |
| MDL (MDL_ZnBa) | ZnBa |
| RT2 (RT2_P) | P |
| RT2 (RT2_ZnFe) | ZnFe |
| MDL (MDL_Mn) | Mn |
| RT2 (RT2_Cr) | Cr |
| Entropy (Entropy_ZnBa) | ZnBa |
| RT2 (RT2_Cd) | Cd |
| RT2 (RT2_ZnS) | ZnS |
| RT2 (RT2_S) | S |
| RT2 (RT2_Pb) | Pb |
| RT2 (RT2_ZnMn) | ZnMn |
| MDL (MDL_ZnLi) | ZnLi |
| RT2 (RT2_ZnAs) | ZnAs |
| Entropy (Entropy_ZnAs) | ZnAs |
| RT2 (RT2_Sr) | Sr |
| RT2 (RT2_ZnSr) | ZnSr |
| MDL (MDL_Zn) | Zn |
| Laminarity (Laminarity_Ca) | Ca |
| RT2 (RT2_ZnCd) | ZnCd |
| RT2 (RT2_ZnLi) | ZnLi |
| RT2 (RT2_ZnSn) | ZnSn |
| MDL (MDL_ZnMg) | ZnMg |
| RT2 (RT2_Sn) | Sn |
| RT2 (RT2_ZnMg) | ZnMg |
| Entropy (Entropy_ZnBi) | ZnBi |
| RT2 (RT2_ZnNi) | ZnNi |
| MDL (MDL_ZnNi) | ZnNi |
| RT2 (RT2_ZnBi) | ZnBi |
| RT2 (RT2_Mg) | Mg |
| RT2 (RT2_Ba) | Ba |
| RT2 (RT2_ZnCu) | ZnCu |
| RT2 (RT2_ZnBa) | ZnBa |
| RT2 (RT2_ZnP) | ZnP |
| RT2 (RT2_Co) | Co |
| RT2 (RT2_ZnHg) | ZnHg |
| RT2 (RT2_Cu) | Cu |
| RT2 (RT2_ZnCo) | ZnCo |
| Laminarity (Laminarity_Sr) | Sr |
| RT2 (RT2_Ca) | Ca |
| RT2 (RT2_ZnCa) | ZnCa |
| MDL (MDL_ZnCa) | ZnCa |
| RT2 (RT2_ZnPb) | ZnPb |
| Entropy (Entropy_Zn) | Zn |
| RT2 (RT2_Bi) | Bi |
| MDL (MDL_I) | I |
| Entropy (Entropy_I) | I |
| Laminarity (Laminarity_Ni) | Ni |
| MDL (MDL_ZnSr) | ZnSr |
| MDL (MDL_ZnP) | ZnP |
| RT2 (RT2_ZnCr) | ZnCr |
| RT2 (RT2_ZnI) | ZnI |
| MDL (MDL_ZnFe) | ZnFe |
| RT2 (RT2_As) | As |
| Entropy (Entropy_ZnMg) | ZnMg |
| MDL (MDL_ZnSn) | ZnSn |
| TT (TT_Al) | Al |
| MDL (MDL_ZnHg) | ZnHg |
| Entropy (Entropy_ZnSn) | ZnSn |
| MDL (MDL_ZnCr) | ZnCr |
| MDL (MDL_Ba) | Ba |
| TT (TT_Bi) | Bi |
| RT2 (RT2_Hg) | Hg |
| Entropy (Entropy_ZnP) | ZnP |
| MDL (MDL_ZnPb) | ZnPb |
| TT (TT_Sn) | Sn |
| RT2 (RT2_I) | I |
| TT (TT_Ba) | Ba |
| TT (TT_I) | I |
| TT (TT_Ni) | Ni |
| MDL (MDL_ZnAl) | ZnAl |
| MDL (MDL-Bi) | Bi |
| RT2 (RT2_Li) | Li |
| Entropy (Entropy_ZnCo) | ZnCo |
| Entropy (Entropy_ZnL1) | ZnLi |
| Entropy (Entropy_ZnNi) | ZnNi |
| Entropy (Entropy_ZnCa) | ZnCa |
| MDL (MDL_Fe) | Fe |
| MDL (MDL_S) | S |
| MDL (MDL_ZnCo) | ZnCo |
| Entropy (Entropy_ZnHg) | ZnHg |
| TT (TT_Co) | Co |
| MDL (MDL_ZnI) | ZnI |
| Entropy (Entropy_ZnPb) | ZnPb |
| MDL (MDL_Al) | Al |
| Entropy (Entropy_ZnCr) | ZnCr |
| Entropy (Entropy_Ba) | Ba |
| Entropy (Entropy_ZnFe) | ZnFe |
| MDL (MDL_ZnS) | ZnS |
| MDL (MDL_Hg) | Hg |
| Entropy (Entropy_ZnSr) | ZnSr |
| Entropy (Entropy_S) | S |
| TT (TT_Hg) | Hg |
| Laminarity (Laminarity_Al) | Al |
| Entropy (Entropy_ZnAl) | ZnAl |
| Entropy (Entropy_Bi) | Bi |
| Determinism (Determinism_ZnP) | ZnP |
| Entropy (Entropy_Fe) | Fe |
| Determinism (Determinism ZnBi) | ZnBi |
| Entropy (Entropy_ZnI) | ZnI |
| Laminarity (Laminarity_Ba) | Ba |
| Determinism (Determinism_I) | I |
| TT (TT_Cr) | Cr |
| Determinism (Determinism_Ba) | Ba |
| Laminarity (Laminarity_I) | I |
| Determinism (Determinism_Sn) | Sn |
| Determinism (Determinism_ZnBa) | ZnBa |
| Entropy (Entropy_Al) | Al |
| Determinism (Determinism_ZnCo) | ZnCo |
| Entropy (Entropy_Hg) | Hg |
| Laminarity (Laminarity_Cr) | Cr |
| Laminarity (Laminarity_P) | P |
| Laminarity (Laminarity_S) | S |
| Determinism (Determinism_Zn) | Zn |
| Entropy (Entropy_ZnS) | ZnS |
| Determinism (Determinism_Al) | Al |
| Determinism (Determinism_P) | P |
| Laminarity (Laminarity_Hg) | Hg |

In some embodiments, the set of features, where each feature is associated with a respective elemental isotope or a combination of elemental isotopes *(e.g.,* a combination of two elemental isotopes, or a combination of more than two element isotopes), is selected from the features listed in Table 3. In some embodiments, the set of features includes all the features listed in Table 3. In some embodiments, the set of features includes at least 50%, 60%, 70%, 80% or 90% of the features listed in Table 3.

In some embodiments, the set of features, where each feature is associated with a respective elemental isotope or a combination of elemental isotopes *(e.g.,* a combination of two elemental isotopes, or a combination of more than two element isotopes), is selected from the features listed in Tables 2 and 3. In some embodiments, the set of features includes all the features listed in Tables 2 and 3. In some embodiments, the set of features includes at least 50%, 60%, 70%, 80% or 90% of the features listed in Tables 2 and 3.

In some embodiments, the set of features, where each feature is associated with a respective elemental isotope or a combination of elemental isotopes *(e.g.,* a combination of two elemental isotopes, or a combination of more than two element isotopes), is selected from the features listed in Table 4. In some embodiments, the set of features includes all the features listed in Table 4. In some embodiments, the set of features includes at least 50%, 60%, 70%, 80% or 90% of the features listed in Table 4.

In some embodiments, the set of features, where each feature is associated with a respective elemental isotope or a combination of elemental isotopes *(e.g.,* a combination of two elemental isotopes, or a combination of more than two element isotopes), is selected from the features listed in Table 5. In some embodiments, the set of features includes all the features listed in Table 5. In some embodiments, the set of features includes at least 50%, 60%, 70%, 80% or 90% of the features listed in Table 5.

In some embodiments, the set of features, where each feature is associated with a respective elemental isotope or a combination of elemental isotopes *(e.g.,* a combination of two elemental isotopes, or a combination of more than two element isotopes), is selected from the features listed in Table 6. In some embodiments, the set of features includes all the features listed in Table 6. In some embodiments, the set of features includes at least 50%, 60%, 70%, 80% or 90% of the features listed in Table 6.

In some embodiments, the set of features, where each feature is associated with a respective elemental isotope or a combination of elemental isotopes *(e.g.,* a combination of two elemental isotopes, or a combination of more than two element isotopes), is selected from the features listed in Table 7. In some embodiments, the set of features includes all the features listed in Table 7. In some embodiments, the set of features includes at least 50%, 60%, 70%, 80% or 90% of the features listed in Table 7.

In some embodiments, the set of features, where each feature is associated with a respective elemental isotope or a combination of elemental isotopes *(e.g.,* a combination of two elemental isotopes, or a combination of more than two element isotopes), is selected from the features listed in Table 8. In some embodiments, the set of features includes all the features listed in Table 8. In some embodiments, the set of features includes at least 50%, 60%, 70%, 80% or 90% of the features listed in Table 8.

In some embodiments, the set of features, where each feature is associated with a respective elemental isotope or a combination of elemental isotopes *(e.g.,* a combination of two elemental isotopes, or a combination of more than two element isotopes), is selected from the features listed in Table 9. In some embodiments, the set of features includes all the features listed in Table 9. In some embodiments, the set of features includes at least 50%, 60%, 70%, 80% or 90% of the features listed in Table 9.

In some embodiments, the set of features, where each feature is associated with a respective elemental isotope or a combination of elemental isotopes *(e.g.,* a combination of two elemental isotopes, or a combination of more than two element isotopes), is selected from the features listed in Table 10. In some embodiments, the set of features includes all the features listed in Table 10. In some embodiments, the set of features includes at least 50%, 60%, 70%, 80% or 90% of the features listed in Table 10.

In some embodiments, the set of features, where each feature is associated with a respective elemental isotope or a combination of elemental isotopes *(e.g.,* a combination of two elemental isotopes, or a combination of more than two element isotopes), is selected from the features listed in any combination of Tables 2, 3, 4, 5, 6, 7, 8, 9 and 10. In some embodiments, the set of features includes all the features listed in Tables 2, 3, 4, 5, 6, 7, 8, 9 and 10. In some embodiments, the set of features includes at least 5%, 10%, 15%, 20% or 25% of the features listed in Tables 2, 3, 4, 5, 6, 7, 8, 9 and 10.

Method 200 further includes inputting the obtained set of features (220) to a trained classifier. In some embodiments, the trained classifier includes a predictive computational algorithm to obtain a probability (222) for the subject having a biological condition associated with metal metabolism. In some embodiments, the predictive computational algorithm computes Equation 1: $p \left(subject\right) = \frac{1}{1 + {e}^{- \left(α+{β}_{1}{x}_{1}+⋯+{β}_{k}{x}_{k}\right)}}$ (1) where,
*p(subject)* is the probability that the subject has the biological condition associated with metal metabolism,
*e* is Euler's number,
α is a calculated parameter associated with a probability that the subject has the biological condition associated with metal metabolism when *β*₁*x*₁ *+*··· *+βₖxₖ* equals to zero,
*β_{1, ... ,k}* corresponds to a weight parameter associated with each feature in the set of features including features from 1 through *k, k,* and
*x*_{*1*, ..*. , k*} corresponds to a value derived for each feature in the set of features, the set of features including features from 1 through k.

The features from 1 through k are selected from the features listed in Table 2, and optionally, additionally, from Table 3. The weight parameters *β_{1, ... ,k}* are defined based on classifier training. The probability *p(subject)* is provided as a number ranging from 0 to 1, where 1 corresponds to a 100 % probability that the subject has a biological condition associated with metal metabolism.

In some embodiments, the method 200 also includes applying a predetermined threshold (224) to the obtained probability *p(subject).* If the obtained probability *p(subject)* is above the predetermined threshold, the subject is evaluated as having a biological condition associated with metal metabolism. If the obtained probability is below the predetermined threshold, the subject is evaluated as not having a biological condition associated with metal metabolism. In some embodiments, the predetermined threshold is between 0.3-0.6 *(e.g.,* the predetermined threshold is 0.3, 0.35, 0.4, 0.45, 0.5, 0.55, or 0.6). In some embodiments, the predetermined threshold is 0.45. In some embodiments, the obtained probability is expressed in terms of associated odds *(e.g.,* odds ratio (OR), which may be derived from a probability such that *OR=p*/*(1-p)).* For example, the evaluation includes evaluating odds that the subject has the biological condition associated with metal metabolism.

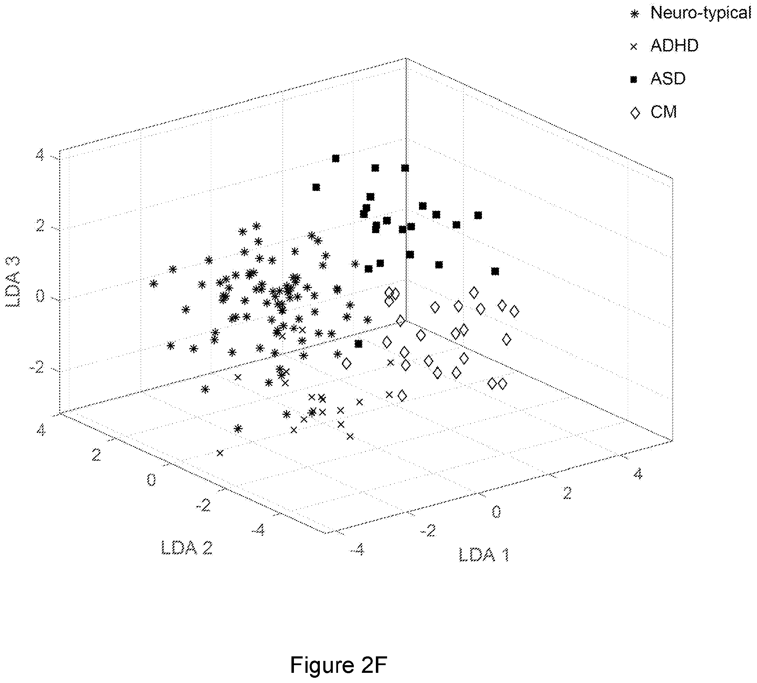

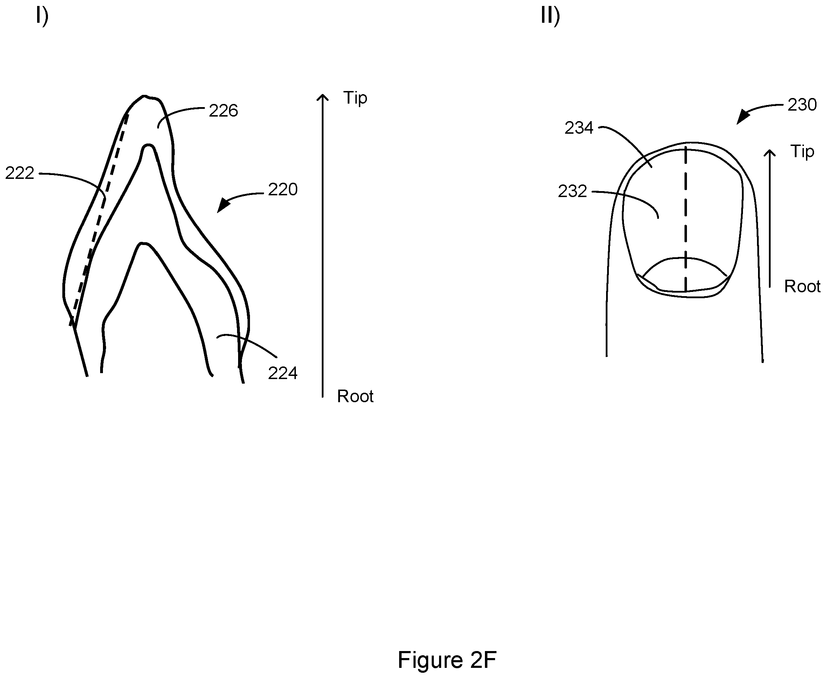

In some embodiments, the method 200 further includes discriminating a first biological condition associated with metal metabolism from an alternative condition, e.g., a second, biological condition associated with metal metabolism. In some embodiments, the alternative condition is associated with no known condition *(e.g.,* a neurotypical condition (NT)). In some embodiments, the alternative condition is associated with an attention-deficit/hyperactivity disorder (ADHD). In some embodiments, the alternative condition is any other neurodevelopmental condition, or a comorbid diagnosis for two neurodevelopmental conditions. Figure 2F provides an illustration of experimental data describing discriminating between an autism spectrum disorder (ASD) and other neurodevelopmental disorders, in accordance with some embodiments of the present disclosure. It is noted that, based on the experimental data shown in Figure 2F, the method 200 of the present disclosure is capable of discriminating between autism spectrum disorder and ADHD. As shown, the present disclosure is also capable of distinguishing autism spectrum disorder from co-morbid (CM) cases diagnosed for both autism spectrum disorder and ADHD.

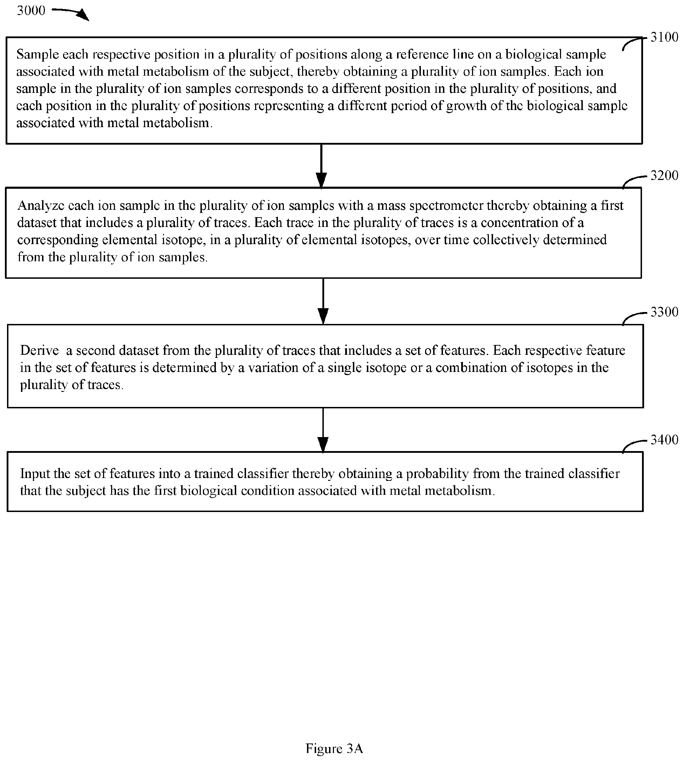

Now that the details of processes and features of the method 200 for evaluating a subject for a biological condition associated with metal metabolism from a biological sample has been disclosed with reference to Figure 2, Figures 3A-3E collectively provide a flow chart of fundamental processes and features of a method 3000 for evaluating a subject for a biological sample associated with metal metabolism, in which optional blocks are indicated with dashed boxes, in accordance with some embodiments of the present disclosure. In some embodiments, the method 3000 corresponds to the method 200.

*Block 3100 of* *Figure 3A**.* The method 3000 includes sampling, *e.g.,* with a laser *(e.g.,* with a LA-ICP-MS), each respective position in a plurality of positions along a reference line on a biological sample associated with metal metabolism of the subject, thereby obtaining a plurality of ion samples (e.g., the areas 200A and 200B of a hair shaft in Figure 2B section I). Each ion sample in the plurality of ion samples corresponds to a different position in the plurality of positions, and each position in the plurality of positions representing a different period of growth of the biological sample associated with metal metabolism.

*Block 3200 of* *Figure 3A**.* The method 3000 includes analyzing each ion sample in the plurality of ion samples with a mass spectrometer, thereby obtaining a first dataset. The first dataset includes a plurality of traces (e.g., the trace 208 in Figure 2D). Each trace in the plurality of traces is a concentration of a corresponding elemental isotope, in a plurality of elemental isotopes, over time collectively determined from the plurality of ion samples.

*Block 3300 of* *Figure 3A**.* The method 3000 includes deriving a second dataset from the plurality of traces that includes a set of features (e.g., a set of features selected from features listed in Table 2). Each respective feature in the set of features is determined by a variation of a single isotope or a combination of isotopes in the plurality of traces. For example, Section III of Figure 2E illustrates a recurrence plot of copper isotope derived from the trace of Section II of Figure 2E. The variation of the copper isotope abundance is observed as diagonal patterns in the recurrence plot.

*Block 3400 of* *Figure 3A**.* In some embodiments, the method 3000 also includes inputting the set of features into a trained classifier thereby obtaining a probability from the trained classifier that the subject has the first biological condition associated with metal metabolism. In some embodiments, is the trained classifier is a neural network algorithm, a support vector machine algorithm, a decision tree algorithm, an unsupervised clustering model algorithm, a supervised clustering model algorithm, or a regression model.

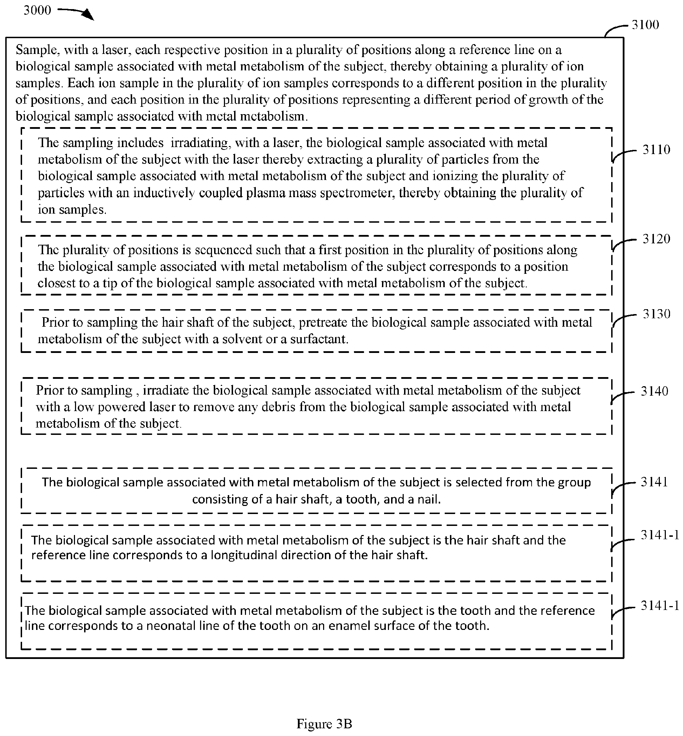

*Block 3110 of* *Figure 3B**.* In some embodiments, the sampling the hair shaft includes irradiating, with a laser, the biological sample associated with metal metabolism of the subject with the laser thereby extracting a plurality of particles from the biological sample associated with metal metabolism of the subject and ionizing the plurality of particles with an inductively coupled plasma mass spectrometer, thereby obtaining the plurality of ion samples (e.g., Figure 2C).

*Block 3120 of* *Figure 3B**.* In some embodiments, the plurality of positions *(e.g.,* the areas 200A and 200B of a hair shaft in Figure 2B section I) along the hair shaft is sequenced a first position in the plurality of positions along the biological sample associated with metal metabolism of the subject corresponds to a position closest to a tip of the biological sample associated with metal metabolism of the subject.

*Block 3130 of* *Figure 3B**.* The method 3000 also includes, prior to sampling the hair shaft of the subject, the biological sample associated with metal metabolism of the subject with a solvent or a surfactant. For example, the hair shaft is washed with TRITON X-100^{®} and ultrapure metal free water *(e.g.,* MILLI-Q^{®} water) and dried overnight in an oven *(e.g.,* at 60 degrees Celsius).

*Block 3140 of* *Figure 3B**.* The method 3000 also includes, prior to sampling the hair shaft of the subject, irradiating the biological sample associated with metal metabolism of the subject with a low powered laser to remove any debris from the biological sample associated with metal metabolism of the subject (e.g., pre-ablating a hair shaft, a tooth, or a nail). For example, the pre-ablation is performed using a laser wavelength of 193 nm and laser energy below 0.4 J/cm² (*e.g.,* the laser energy is 0.4 J/cm², 0.3 J/cm², 0.2 J/cm² or 0.1 J/cm²). In some embodiments, the laser energy ranges from 0.2 J/cm² to 0.4 J/cm².

*Block 3141 of* *Figure 3B**.* The biological sample associated with metal metabolism of the subject is selected from the group consisting of a hair shaft, a tooth (not part of the invention), and a nail (not part of the invention), e.g., a hair shaft, a tooth, and a nail illustrated in sections I, II, and III of Figure 2B, respectively.

*Block 3141-1 of* *Figure 3B**.* The biological sample associated with metal metabolism of the subject is the hair shaft and the reference line corresponds to a longitudinal direction of the hair shaft (e.g., reference line 201 in Figure 2B section I).

*Block 3141-1 of* *Figure 3B* *(not part of the invention).* The biological sample associated with metal metabolism of the subject is the tooth and the reference line corresponds to a neonatal line of the tooth on an enamel surface of the tooth (e.g., reference line 222 along a neonatal line of tooth 220 in Figure 2B section II). In some embodiments, the biological sample associated with metal metabolism of the subject is the nail and the reference line corresponds to a line extending from a root of the nail to the tip of the nail (e.g., reference line 232 of nail 230 in Figure 2B section III).

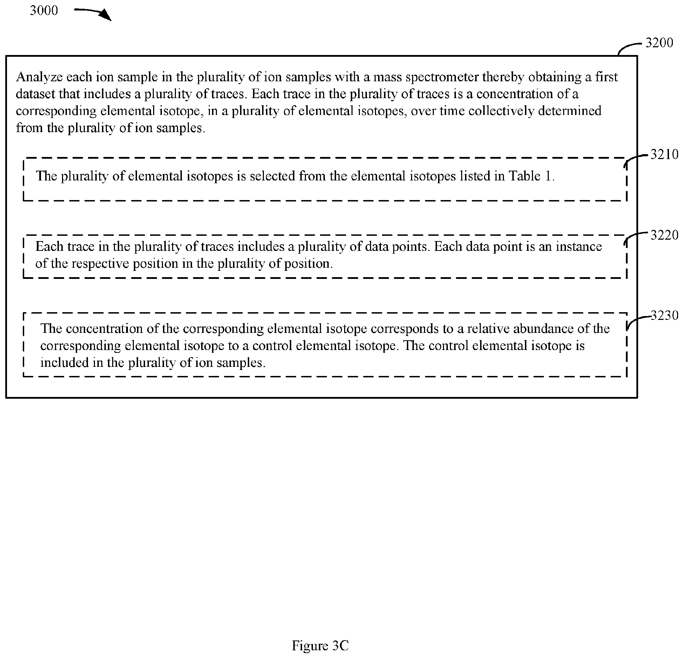

*Block 3210 of* *Figure 3C**.* The plurality of elemental isotopes is selected from the elemental isotopes listed in Table 1. In some embodiments, the plurality of elemental isotopes includes at least 50 %, 60 %, 70 %, 80 % or 90 % of the isotopes included in Table 1.}

*Block 3220 of* *Figure 3C**.* Each trace in the plurality of traces includes a plurality of data points. Each data point is an instance of the respective position in the plurality of position. In some embodiments, each trace includes at least 100 positions (e.g., 100, 150, 200, 250, 300, 350, 400, 450, or 500 positions). In some embodiments, each data point corresponds to approximately 130 min period of hair growth (e.g., the period of hair growth being calculated using a 30 micrometer laser beam size and an average rate of hair growth 1 cm per month.

*Block 3230 of* *Figure 3C**.* The concentration of the corresponding elemental isotope corresponds to a relative abundance of the corresponding elemental isotope to a control elemental isotope. The control elemental isotope is included in the plurality of ion samples. In some embodiments, the control elemental isotope is sulfur.

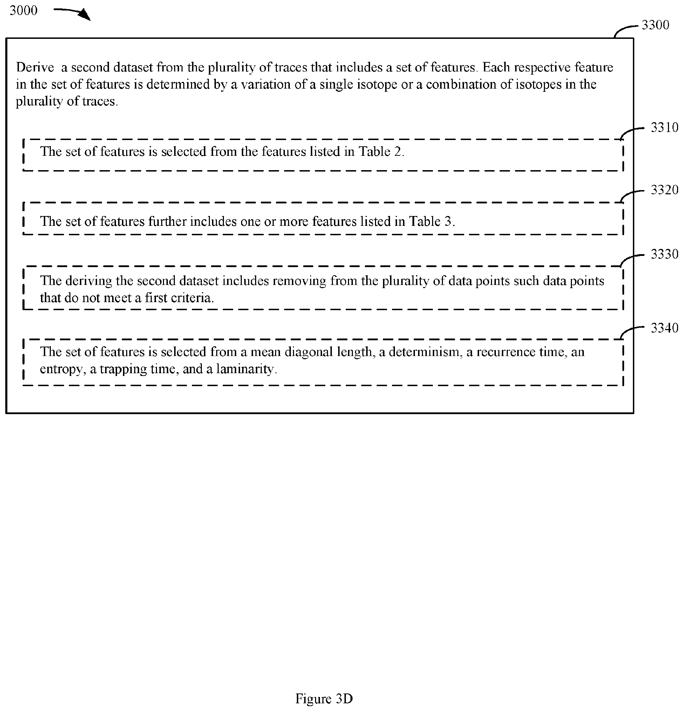

*Block 3310 of* *Figure 3D**.* The set of features is selected from the features listed in Table 2. In some embodiments, the set of features includes the features listed in Table 2. In some embodiments, the set of features includes at least 50 %, 60 %, 70 %, 80 % or 90 % of the features listed in Table 2. Each feature in the set of features is associated with a single respective trace of the plurality of traces or with two respective traces of the plurality of traces.

*Block 3320 of* *Figure 3D**.* The set of features further includes, in addition to the features selected from the features listed in Table 2, one or more features listed in Table 3.

*Block 3330 of* *Figure 3D**.* The deriving of the second dataset includes removing from the plurality of data points such data points that do not meet a first criteria. In some embodiments, the first criteria includes a mean absolute difference between adjacent data points in the plurality of data points being three times a standard deviation of the mean absolute difference between adjacent points *(e.g.,* the peaks 210 are removed from the trace 208 in Figure 2D).

*Block 3340 of* *Figure 3D**.* The set of features is selected from a mean diagonal length, a determinism, a recurrence time, an entropy, a trapping time, and a laminarity.

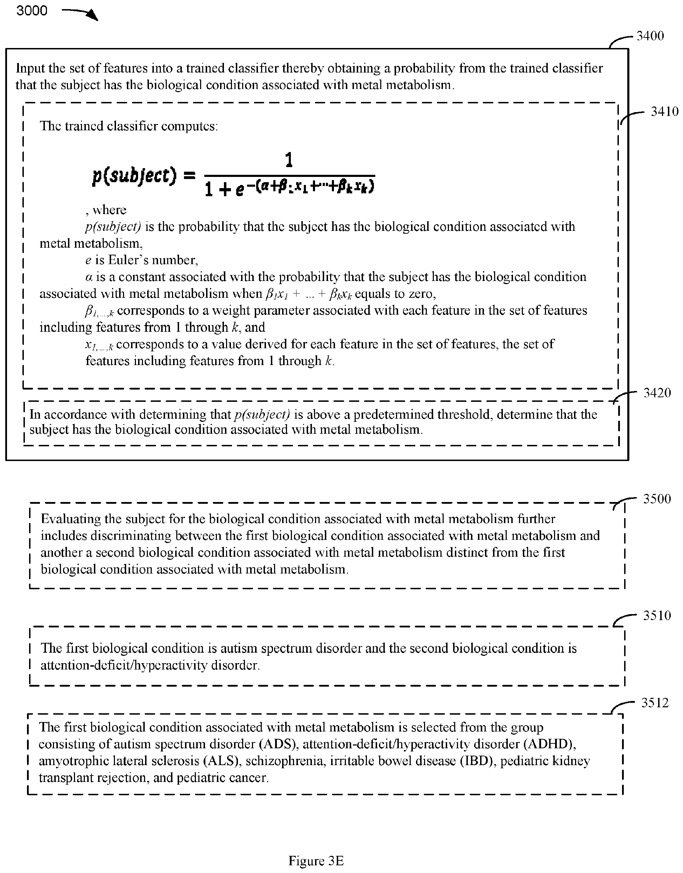

*Block 3410 of* *Figure 3E**.* In some embodiments, the trained classifier computes: $p \left(subject\right) = \frac{1}{1 + {e}^{- \left(α+{β}_{1}{x}_{1}+⋯+{β}_{k}{x}_{k}\right)}}$ (1) where, *p(subject)* is the probability that the subject has the biological condition associated with metal metabolism, e is Euler's number, *α* is a calculated parameter associated with a probability that the subject has the biological condition associated with metal metabolism when *β*₁*x*₁ *+*··· +*βₖxₖ* equals to zero, *β_{1, ... ,k}* corresponds to a weight parameter associated with each feature in the set of features including features from 1 through *k,* and *x*_{*1*, ..*. , k*} value derived for each feature in the set of features, the set of features including features 1 through k.

*Block 3420 of* *Figure 3E**.* In accordance with determining that *p(subject)* is above a predetermined threshold, determine that the subject has the biological condition associated with metal metabolism.

*Block 3500 of* *Figure 3E**.* In some embodiments, evaluating the subject for the biological condition associated with metal metabolism further includes discriminating between the first biological condition associated with metal metabolism and a second biological condition associated with metal metabolism distinct from the first biological condition associated with metal metabolism.

*Block 3510 of* *Figure 3E**.* In some embodiments, the first biological condition is autism spectrum disorder and the second biological condition is attention-deficit/hyperactivity disorder.

*Block 3510 of* *Figure 3E**.* According to the invention, the first biological condition associated with metal metabolism is autism spectrum disorder (ADS). In embodiments that are not part of the invention, the first biological condition may be attention-deficit/hyperactivity disorder (ADHD), amyotrophic lateral sclerosis (ALS), schizophrenia, irritable bowel disease (IBD), pediatric kidney transplant rejection, or pediatric cancer.

In some embodiments, the method 3000 described with respect to Figures 3A-3E is performed by a device executing one or more programs (e.g., one or more programs stored in the Non-Persistent Memory 111 or in the Persistent Memory 112 in Figure 1) including instructions to perform the method 3000. In some embodiments, the method 3000 is performed by a system comprising at least one processor *(e.g.,* the processing core 102) and memory *(e.g.,* one or more programs stored in the Non-Persistent Memory 111 or in the Persistent Memory 112) comprising instructions to perform the method 3000.

### Classifier training (not part of the invention).

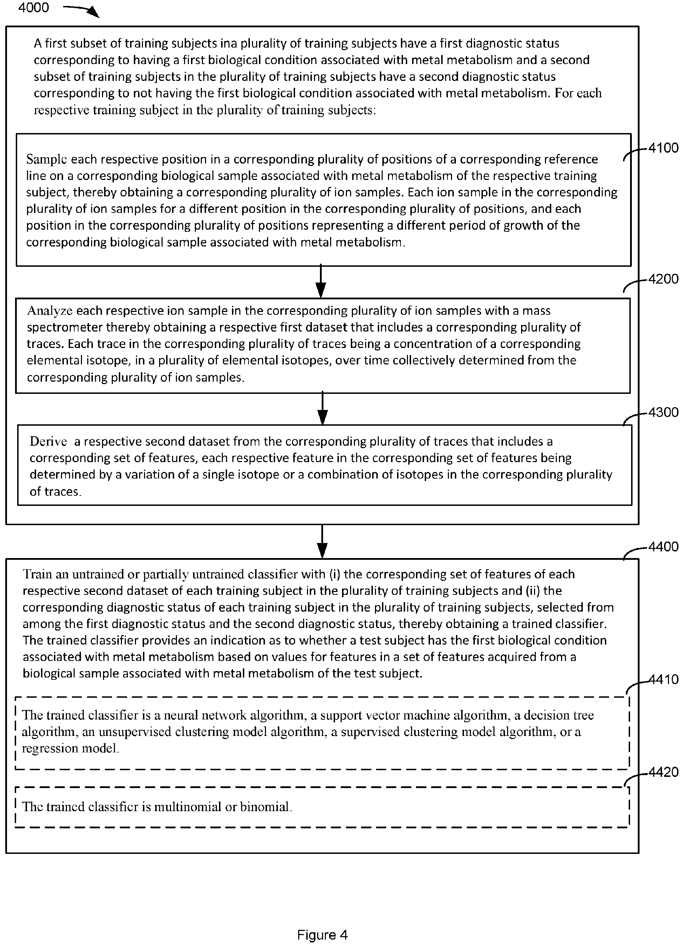

Now that the methods and features of the method 3000 have been disclosed with reference to Figures 3A-3E, Figure 4 provides a flow chart of processes and features of a method 4000 for training a classifier for evaluating a subject for a biological condition associated with metal metabolism, in which optional blocks are indicated with dashed boxes, in accordance with some embodiments of the present disclosure. The method of training a classifier includes collecting biological sample associated with metal metabolism of a respective training subject from a plurality of training subjects and training the classifier using the collected biological samples. The training subjects are humans. Each training has a diagnostic status indicating that they have either been diagnosed with the biological condition associated with metal metabolism, or have not been diagnosed with the biological condition associated with metal metabolism. In some embodiments, the training subjects are children aged equal to, or below, 5 years (e.g., equal to or below 5 years, 4 years, 3 years, 2 years, 1 year, 9 months, 6 months, 3 months or 1 month). Steps of the method 4000 described below with respect to Blocks 4100-4300 are performed for each training subject in a plurality of training subjects.

*Block 4100 of* *Figure 4**.* The method 4000 includes sampling, with a laser, each respective position in a corresponding plurality of positions of a corresponding reference line on a corresponding biological sample associated with metal metabolism of the respective training subject, thereby obtaining a corresponding plurality of ion samples. Each ion sample in the corresponding plurality of ion samples for a different position in the corresponding plurality of positions, and each position in the corresponding plurality of positions representing a different period of growth of the corresponding biological sample associated with metal metabolism.

*Block 4200 of* *Figure 4**.* The method 4000 includes each respective ion sample in the corresponding plurality of ion samples with a mass spectrometer thereby obtaining a respective first dataset that includes a corresponding plurality of traces. Each trace in the corresponding plurality of traces being a concentration of a corresponding elemental isotope, in a plurality of elemental isotopes, over time collectively determined from the corresponding plurality of ion samples.

*Block 4300 of* *Figure 4**.* The method 4000 includes deriving a respective second dataset from the corresponding plurality of traces that includes a corresponding set of features, each respective feature in the corresponding set of features being determined by a variation of a single isotope or a combination of isotopes in the corresponding plurality of traces.

*Block 4400 of* *Figure 4**.* The method 4000 further includes training an untrained or partially untrained classifier with (i) the corresponding set of features of each respective second dataset of each training subject in the plurality of training subjects and (ii) the corresponding diagnostic status of each training subject in the plurality of training subjects, selected from among the first diagnostic status and the second diagnostic status, thereby obtaining a trained classifier. The trained classifier provides an indication as to whether a test subject has the first biological condition associated with metal metabolism based on values for features in a set of features acquired from a biological sample associated with metal metabolism of the test subject. In some embodiments, *(Block 4410)* the trained classifier is a neural network algorithm, a convolutional neural network algorithm, a support vector machine algorithm, a decision tree algorithm, an unsupervised clustering model algorithm, a supervised clustering model algorithm, or a regression model. In some embodiments, *(Block 4420)* the trained classifier is multinomial or binomial. In some embodiments, the trained classifier can be used to make a binary prediction as to whether a sample was derived from a subject with the first biological condition associated with metal metabolism or not; or, may be multinomial, distinguishing subjects with no diagnosis from those with the first biological condition associated with metal metabolism or a second biological condition associated with metal metabolism, where the second biological condition is distinct from the first biological condition.

In some embodiments, the classifier is a neural network or a convolutional neural network. *See,* Vincent et al., 2010, "Stacked denoising autoencoders: Learning useful representations in a deep network with a local denoising criterion," J Mach Learn Res 11, pp. 3371-3408; Larochelle et al., 2009, "Exploring strategies for training deep neural networks," J Mach Learn Res 10, pp. 1-40; and Hassoun, 1995, Fundamentals of Artificial Neural Networks, Massachusetts Institute of Technology.

SVMs are described in Cristianini and Shawe-Taylor, 2000, "An Introduction to Support Vector Machines," Cambridge University Press, Cambridge; Boser et al., 1992, "A training algorithm for optimal margin classifiers," in Proceedings of the 5th Annual ACM Workshop on Computational Learning Theory, ACM Press, Pittsburgh, Pa., pp. 142-152; Vapnik, 1998, Statistical Learning Theory, Wiley, New York; Mount, 2001, Bioinformatics: sequence and genome analysis, Cold Spring Harbor Laboratory Press, Cold Spring Harbor, N.Y.; Duda, Pattern Classification, Second Edition, 2001, John Wiley & Sons, Inc., pp. 259, 262-265; and Hastie, 2001, The Elements of Statistical Learning, Springer, New York; and Furey et al., 2000, Bioinformatics 16, 906-914. When used for classification, SVMs separate a given set of binary labeled data with a hyper-plane that is maximally distant from the labeled data. For cases in which no linear separation is possible, SVMs can work in combination with the technique of 'kernels', which automatically realizes a non-linear mapping to a feature space. The hyper-plane found by the SVM in feature space corresponds to a non-linear decision boundary in the input space.

Decision trees are described generally by Duda, 2001, Pattern Classification, John Wiley & Sons, Inc., New York, pp. 395-396. Tree-based methods partition the feature space into a set of rectangles, and then fit a model (like a constant) in each one. In some embodiments, the decision tree is random forest regression. One specific algorithm that can be used is a classification and regression tree (CART). Other specific decision tree algorithms include, but are not limited to, ID3, C4.5, MART, and Random Forests. CART, ID3, and C4.5 are described in Duda, 2001, Pattern Classification, John Wiley & Sons, Inc., New York. pp. 396-408 and pp. 411-412. CART, MART, and C4.5 are described in Hastie et al., 2001, The Elements of Statistical Learning, Springer-Verlag, New York, Chapter 9. Random Forests are described in Breiman, 1999, "Random Forests--Random Features," Technical Report 567, Statistics Department, U.C. Berkeley, September 1999.

Clustering (e.g., unsupervised clustering model algorithms and supervised clustering model algorithms) is described at pages 211-256 of Duda and Hart, Pattern Classification and Scene Analysis, 1973, John Wiley & Sons, Inc., New York, (hereinafter "Duda 1973"). As described in Section 6.7 of Duda 1973, the clustering problem is described as one of finding natural groupings in a dataset. To identify natural groupings, two issues are addressed. First, a way to measure similarity (or dissimilarity) between two samples is determined. This metric (similarity measure) is used to ensure that the samples in one cluster are more like one another than they are to samples in other clusters. Second, a mechanism for partitioning the data into clusters using the similarity measure is determined. Similarity measures are discussed in Section 6.7 of Duda 1973, where it is stated that one way to begin a clustering investigation is to define a distance function and to compute the matrix of distances between all pairs of samples in the training set. If distance is a good measure of similarity, then the distance between reference entities in the same cluster will be significantly less than the distance between the reference entities in different clusters. However, as stated on page 215 of Duda 1973, clustering does not require the use of a distance metric. For example, a nonmetric similarity function s(x, x') can be used to compare two vectors x and x'. Conventionally, s(x, x') is a symmetric function whose value is large when x and x' are somehow "similar." An example of a nonmetric similarity function s(x, x') is provided on page 218 of Duda 1973. Once a method for measuring "similarity" or "dissimilarity" between points in a dataset has been selected, clustering requires a criterion function that measures the clustering quality of any partition of the data. Partitions of the data set that extremize the criterion function are used to cluster the data. *See* page 217 of Duda 1973. Criterion functions are discussed in Section 6.8 of Duda 1973. More recently, Duda et al., Pattern Classification, 2nd edition, John Wiley & Sons, Inc. New York, has been published. Pages 537-563 describe clustering in detail. More information on clustering techniques can be found in Kaufman and Rousseeuw, 1990, Finding Groups in Data: An Introduction to Cluster Analysis, Wiley, New York, N.Y.; Everitt, 1993, Cluster analysis (3d ed.), Wiley, New York, N.Y.; and Backer, 1995, Computer-Assisted Reasoning in Cluster Analysis, Prentice Hall, Upper Saddle River, New Jersey. Particular exemplary clustering techniques that can be used in the present disclosure include, but are not limited to, hierarchical clustering (agglomerative clustering using nearest-neighbor algorithm, farthest-neighbor algorithm, the average linkage algorithm, the centroid algorithm, or the sum-of-squares algorithm), k-means clustering, fuzzy k-means clustering algorithm, and Jarvis-Patrick clustering. In some embodiments, the clustering comprises unsupervised clustering, where no preconceived notion of what clusters should form when the training set is clustered, are imposed.

Regression models, such as the of the multi-category logit models, are described in Agresti, An Introduction to Categorical Data Analysis, 1996, John Wiley & Sons, Inc., New York, Chapter 8. In some embodiments, the classifier makes use of a regression model disclosed in Hastie et al., 2001, The Elements of Statistical Learning, Springer-Verlag, New York.

In some embodiments, the method 4000 described with respect to Figure 4 is performed by a device executing one or more programs (e.g., one or more programs stored in the Non-Persistent Memory 111 or in the Persistent Memory 112 in Figure 1) including instructions to perform the method 4000. In some embodiments, the method 4000 is performed by a system comprising at least one processor *(e.g.,* the processing core 102) and memory *(e.g.,* one or more programs stored in the Non-Persistent Memory 111 or in the Persistent Memory 112) comprising instructions to perform the method 4000.

### Examples.

### Example 1- Evaluation of a subject for autism spectrum disorder

Two subjects (Subject 1 and Subject 2) were evaluated for autism spectrum disorder using the method 200 described with respect to Figures 2A-2F. Table 4 illustrates the results including the features from Table 2 *(e.g.,* column "Features") associated with respective parameter estimate *β* values obtained from a training set and empirical results *(e.g., x* values) for Subject 1 and Subject 2. The *β* values are obtained by estimating each feature in the training data set that describes a change in log odds of autism spectrum disorder status associated with a 1-unit change for a respective feature. The estimated parameters *β* and the x values for each respective subject are input to the algorithm computing *p(subject)* for each respective subject (see, Equation 1 above) given a calculated α parameter of 36.31. For Subject 1, the estimated parameters *β* and empirical results *x* yielded an estimated probability *p(subject₁)* of 2.28 % that Subject 1 has autism spectrum disorder. For Subject 2, the estimated parameters *β* and empirical results x yielded an estimated probability *p(subject₂)* of 96.9% that Subject 2 has autism spectrum disorder. With a predetermined threshold of 50 %, Subject 1 was therefore evaluated as not having autism spectrum disorder and Subject 2 is evaluated has having autism spectrum disorder. Furthermore, odds for Subject 1 having autism spectrum disorder equal to 0.023 and odds for Subject 2 having autism spectrum disorder equal to 31.2. Odds are calculated from probability using Equation 2. $Odds = \frac{p \left({subject}_{i}\right)}{1 - p \left({subject}_{i}\right)}$ (2)

**Table 4. Features with associated parameter estimates obtained from a training set and empirical x values for Subject 1 and Subject 2.**

| **Feature** | ***β* value obtained from a training set** | **x value for Subject 1** | **x value for Subject 2** | **Feature present in Table 2 or 3?** |
|---|---|---|---|---|
| Determinism_Cd | 0.429 | 0.903 | 0.932 | Yes |
| Determinism_Cr | -36.579 | 0.904 | 0.893 | Yes |
| Determinism_ZnHg | -68.008 | 0.863 | 0.903 | Yes |
| Determinism_Cu | -1.834 | 0.901 | 0.901 | Yes |
| Determinism_ZnMn | -2.447 | 0.909 | 0.933 | Yes |
| Determinism_Sr | -3.453 | 0.951 | 0.929 | Yes |
| Entropy_As | -12.823 | 1.972 | 1.778 | Yes |
| Determinism_Mg | -3.018 | 0.969 | 0.932 | Yes |
| Entropy_Li | -9.800 | 2.096 | 1.758 | Yes |
| Determinism_ZnCu | -7.356 | 0.911 | 0.866 | Yes |
| Entropy_ZnCu | -0.255 | 1.962 | 1.856 | Yes |
| MDL_ZnCu | -0.006 | 4.325 | 3.883 | Yes |
| Determinism_Ca | -37.419 | 0.947 | 0.909 | Yes |
| Determinism_Mn | 1.600 | 0.919 | 0.900 | Yes |
| Determinism_Ni | -5.041 | 0.893 | 0.888 | Yes |
| Determinism_ZnMg | 14.018 | 0.941 | 0.921 | Yes |
| Determinism_ZnCr | -5.769 | 0.899 | 0.930 | Yes |
| Determinism_Pb | -8.253 | 0.918 | 0.914 | Yes |
| Determinism_ZnNi | 7.175 | 0.905 | 0.935 | Yes |
| Determinism_ZnSn | 15.285 | 0.931 | 0.935 | Yes |
| Determinism_Li | 21.384 | 0.917 | 0.864 | Yes |
| Determinism_Hg | 12.154 | 0.888 | 0.883 | Yes |
| Determinism_Fe | 26.657 | 0.895 | 0.941 | Yes |
| Determinism_As | 33.235 | 0.892 | 0.873 | Yes |
| Determinism_ZnI | 55.574 | 0.861 | 0.907 | Yes |

### Example 2- Receiver Operating Characteristics (ROC) curve.

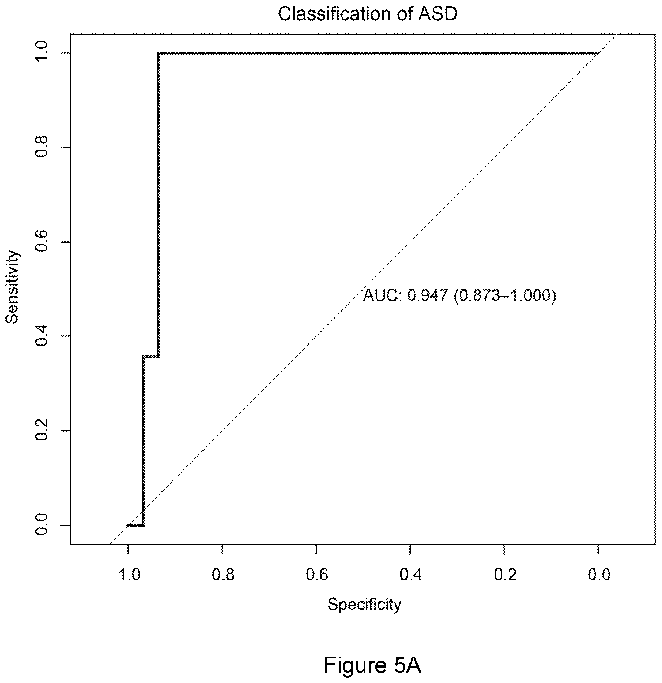

Figure 5A illustrates an experimental Receiver Operating Characteristics (ROC) curve for evaluating accuracy of the disclosed method of evaluating a subject for autism spectrum disorder, in accordance with some embodiments. In the experiment described with respect to Figure 5A, the evaluation is performed by measuring hair shaft of the subject. A ROC curve can be used for evaluating a performance of a binary classifier. A ROC curve is plotted as sensitivity (also called as a true positive rate) against specificity (also called as a true negative rate). A perfect classifier would have a 100% sensitivity and 100% specificity and an area under the curve (AUC) corresponding to 1. As shown in Figure 5A, the ROC curve derived from experimental data to evaluate the performance of the disclosed classification method has an AUC corresponding to 0.947, indicating that the disclosed method has above 90 % accuracy for evaluating that a subject has autism spectrum disorder.

### Example 3- Evaluation of a subject for autism spectrum disorder from a hair sample of one or two parents

To develop a classifier that could determine whether a subject has autism spectrum disorder or not, hair was collected from parents (biological mother and father) of twins in a study based in Sweden (Roots of Autism and ADHD Study in Sweden - RATSS; Marwan et al., 2007, "Recurrence plots for the analysis of complex systems," Phys. Rep. 438, 237-329.). The aim of the study was to predict the autism spectrum disorder (ASD) diagnosis of the children from only the parents' hair. The children have undergone clinical testing for autism. In this analysis, no data on the child is used other than the diagnosis. Three classifiers were developed: a) classifier using only mother's hair to predict child autism (n=29; 14 ASD cases, 15 controls); b) classifier using only father's hair (n=23; 9 ASD cases and 14 controls); and c) classifier using both mother's and father's hair (n=52; 23 ASD cases, 29 controls.

Table 5 illustrates the features used and their *β* values for the mother's hair cohort, father's hair cohort, and the combination of mother's and father's hair coort. The *β* values are obtained by estimating each feature in the respective cohort that describes a change in log odds of autism spectrum disorder status associated with a 1-unit change for a respective feature.

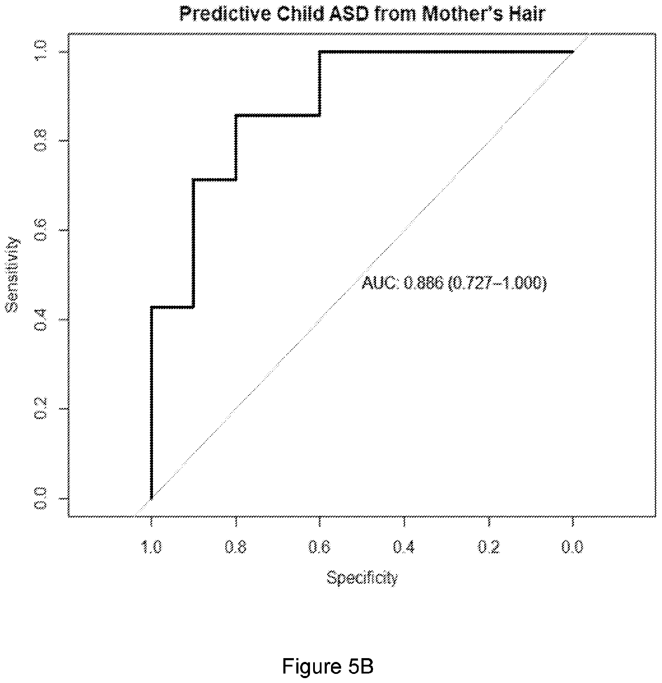

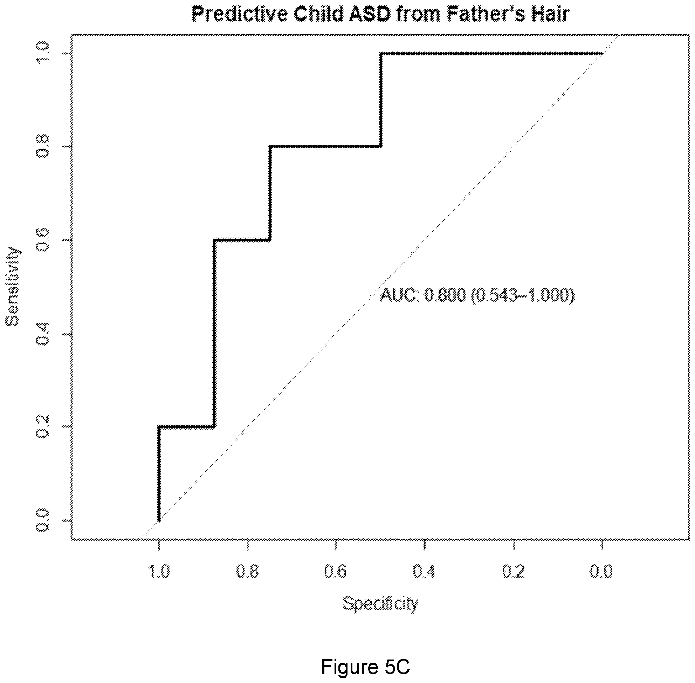

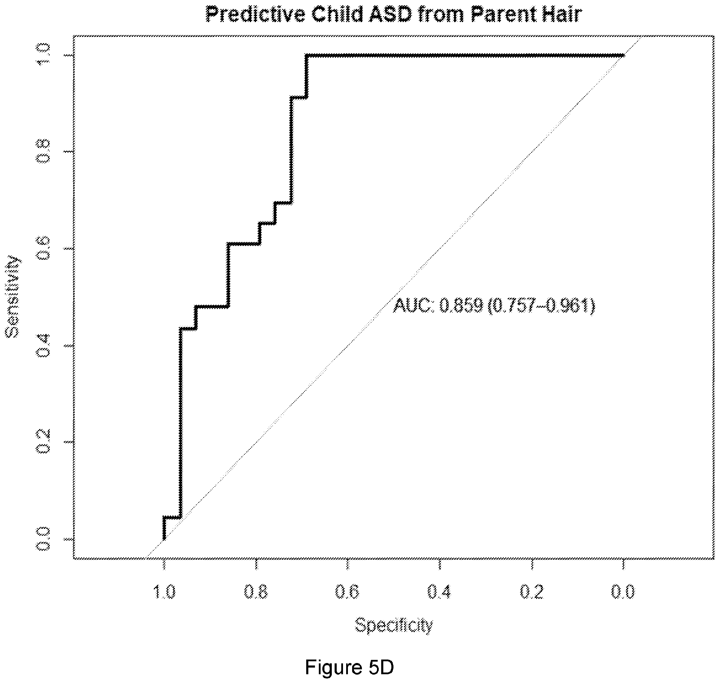

Figures 5B, 5C, and 5D respectively illustrate the experimental ROC curves for evaluating accuracy of the trained classifier for autism spectrum disorder based on mother's hair, father's hair, and the combination of the mother's and the father's hair, in accordance with some embodiments. As shown in Figure 5B, the ROC curve derived from experimental data to evaluate the performance of the disclosed classification method has an AUC corresponding to 0.886, indicating that the disclosed method has above 85% accuracy for evaluating that a subject has autism spectrum disorder based on a sample of the subject's mother's hair. As shown in Figure 5C, the ROC curve derived from experimental data to evaluate the performance of the disclosed classification method has an AUC corresponding to 0.800, indicating that the disclosed method has 80% accuracy for evaluating that a subject has autism spectrum disorder based on a sample of the subject's father's hair. As shown in Figure 5D, the ROC curve derived from experimental data to evaluate the performance of the disclosed classification method has an AUC corresponding to 0.859, indicating that the disclosed method has above 85 % accuracy for evaluating that a subject has autism spectrum disorder based on a combination of a sample of the subject's mother's hair and the subject's father's hair.

**Table 5. Features with empirical x values for a subject based on a sample taken from the subject's mother, subject's farther and a combination of the subject's mother and father.**

| **Feature** | ***β* value obtained from mother cohort** | ***β* value obtained from father cohort** | ***β* value obtained from combined cohort** | **Feature in Table 2 or 3?** |
|---|---|---|---|---|
| Determinism_Ba | 0.26995224 | -0.099215 | -0.018623 | Yes |
| Determinism_Ca | 0.30179438 | -0.582311 | 0.8019875 | Yes |
| Determinism_Cr | 1.41987747 | 1.22403 | 1.066073 | Yes |
| Determinism_Cu | -0.8056872 | 0.390644 | -0.127668 | Yes |
| Determinism_Fe | 0.61809714 | -0.337452 | 0.8514657 | Yes |
| Determinism_I | -1.6204623 | -1.000588 | -3.012296 | Yes |
| Determinism_Mg | 0.49033131 | -0.895876 | -0.032059 | Yes |
| Determinism_Mn | 2.13899536 | 0.6433259 | 2.1328616 | Yes |
| Determinism_P | 0.18257282 | 0.4688593 | -1.905231 | Yes |
| Determinism_Pb | -0.89968 | -1.816234 | 0.3425074 | Yes |
| Determinism S | 1.53986487 | -5.893958 | -0.507446 | Yes |
| Determinism_Sn | 0.22557473 | -0.961602 | 0.0582074 | Yes |
| Determinism_Sr | -0.5469032 | 1.0391182 | 0.8212948 | Yes |
| Determinism_Zn | 0.17079778 | 0.2256547 | -0.235289 | No |
| Determinism_ZnBa | 0.20471351 | 0.5033085 | 0.3960209 | Yes |
| Determinism_ZnCa | 0.21490655 | -0.038427 | 0.1804033 | Yes |
| Determinism_ZnCr | 0.57026275 | -0.047577 | -0.080889 | Yes |
| Determinism _ZnCu | 0.30597749 | 0.5666415 | 0.0273199 | Yes |
| Determinism_ZnFe | -0.1061911 | 0.3103529 | 0.2348702 | Yes |
| Determinism_ZnI | 0.06927202 | 0.3023726 | -0.772397 | Yes |
| Determinism_ZnMg | 0.31475826 | 0.3667723 | 0.3215559 | Yes |
| Determinism_ZnMn | 0.06897562 | -0.375193 | -0.566958 | Yes |
| Determinism_ZnP | -0.1060363 | 0.976308 | -0.566118 | Yes |
| Determinism_ZnPb | 0.16626519 | 1.0407674 | 0.1548639 | Yes |
| Determinism_ZnS | 0.58909549 | -0.042635 | -0.586531 | Yes |
| Determinism_ZnSn | 0.21543319 | 0.2843955 | 0.1103138 | Yes |
| Determinism_ZnSr | 0.12936851 | 0.2932143 | 0.2832403 | Yes |
| MDL_Ba | -0.0175887 | 0.0168481 | 0.0280754 | Yes |
| MDL_Ca | 0.012664 | -0.023574 | 0.0268955 | Yes |
| MDL_Cr | 0.13840947 | 0.1117677 | 0.1047073 | Yes |
| MDL_Cu | -0.0195811 | 0.016205 | 0.0122942 | Yes |
| MDL_Fe | 0.0533853 | -0.020462 | -0.00092 | Yes |
| MDL_I | -0.1399248 | -0.159781 | -0.244053 | Yes |
| MDL_Mg | 0.01089451 | 0.009199 | 0.0078554 | Yes |
| MDL_Mn | 0.10680868 | -0.000665 | 0.0578152 | Yes |
| MDL_P | 0.06724766 | -0.005505 | -0.137642 | Yes |
| MDL_Pb | -0.0472965 | 0.0006465 | 0.0219535 | Yes |
| MDL_S | 0.09097365 | -0.024026 | -0.030172 | Yes |
| MDL_Sn | 0.01869144 | 0.0166052 | 0.0320733 | Yes |
| MDL_Sr | -0.0224024 | 0.0440478 | 0.0236678 | Yes |
| MDL_Zn | -0.0122259 | -0.020605 | -0.038804 | Yes |
| MDL _ZnBa | -0.0101652 | 0.0025333 | 0.0119736 | Yes |
| MDL_ZnCa | -0.0042781 | -0.015974 | -0.011638 | Yes |
| MDL_ZnCr | 0.03220882 | -0.001205 | -0.023751 | Yes |
| MDL_ZnCu | -0.0151274 | -0.003606 | -0.019749 | Yes |
| MDL_ZnFe | -0.0109976 | -0.057743 | -0.004976 | Yes |
| MDL_ZnI | -0.0259466 | 0.0044879 | -0.060072 | Yes |
| MDL_ZnMg | -0.005591 | 0.0015727 | -0.002783 | Yes |
| MDL_ZnMn | 0.01023798 | -0.042947 | -0.040765 | Yes |
| MDL_ZnP | -0.0110143 | 0.0628644 | 0.0339964 | Yes |
| MDL_ZnPb | -0.0175028 | -0.013542 | -0.020343 | Yes |
| MDL_ZnS | 0.03383046 | -0.033119 | -0.067766 | Yes |
| MDL_ZnSn | 0.02019903 | 0.0107794 | 0.0243532 | Yes |
| MDL_ZnSr | -0.0046591 | -0.011486 | -0.005694 | Yes |
| Entropy_Ba | -0.028773 | 0.0610208 | 0.0982191 | Yes |
| Entropy_Ca | 0.05788363 | -0.093436 | 0.1229156 | Yes |
| Entropy_Cr | 0.37859451 | 0.3216797 | 0.2793699 | Yes |
| Entropy_Cu | -0.0516145 | 0.0699055 | 0.0539494 | Yes |
| Entropy_Fe | 0.14061759 | -0.045755 | 0.0528002 | Yes |
| Entropy_I | -0.3312469 | -0.416349 | -0.597241 | Yes |
| Entropy_Mg | 0.05500931 | 0.0072411 | 0.0322303 | Yes |
| Entropy_Mn | 0.27564835 | 0.0167556 | 0.2061444 | Yes |
| Entropy_P | 0.16962518 | -0.010326 | -0.350157 | Yes |
| Entropy_Pb | -0.110991 | 0.0140247 | 0.092294 | Yes |
| Entropy_S | 0.27532791 | -0.122115 | -0.100868 | Yes |
| Entropy_Sn | 0.0546037 | 0.0211283 | 0.0877334 | Yes |
| Entropy_Sr | -0.0635242 | 0.1687305 | 0.1053903 | Yes |
| Entropy_Zn | -0.0099921 | -0.052325 | -0.082714 | Yes |
| Entropy_ZnBa | -0.0034346 | 0.0311443 | 0.0666616 | Yes |
| Entropy_ZnCa | 0.0157374 | -0.009689 | 0.012736 | Yes |
| Entropy ZnCr | 0.10055577 | 0.0126058 | -0.043222 | Yes |
| Entropy_ZnCu | -0.0126217 | 0.018627 | -0.03692 | Yes |
| Entropy_ZnFe | 0.0380713 | -0.155539 | 0.0069564 | Yes |
| Entropy_ZnI | -0.0033752 | 0.0344947 | -0.103396 | Yes |
| Entropy_ZnMg | 0.01394646 | 0.0315456 | 0.0418649 | Yes |
| Entropy_ZnMn | 0.09446064 | -0.124788 | -0.019134 | Yes |
| Entropy_ZnP | -8.00E-05 | 0.1807677 | 0.0826718 | Yes |
| Entropy_ZnPb | -0.0235718 | -0.027717 | -0.045725 | Yes |
| Entropy_ZnS | 0.1063242 | -0.097799 | -0.179409 | Yes |
| Entropy_ZnSn | 0.06490719 | 0.0156185 | 0.065172 | Yes |
| Entropy_ZnSr | 0.04530203 | 0.0014764 | 0.0352422 | Yes |
| Laminarity_Ba | 0.12745187 | 0.1155964 | 0.2371277 | Yes |
| Laminarity_Ca | -0.0180744 | 0.013396 | 0.0142638 | Yes |
| Laminarity_Cr | -0.1043894 | 0.0172006 | 0.4130502 | Yes |
| Laminarity_Cu | 0.07412039 | 0.042768 | 0.2020793 | Yes |
| Laminarity_Fe | 0.70827704 | -0.638965 | -0.06603 | Yes |
| Laminarity_I | -0.1197673 | -0.013497 | -0.222448 | Yes |
| Laminarity_Mg | 0.14505143 | 0.0176582 | 0.1166678 | Yes |
| Laminarity_Mn | -0.000969 | -0.341907 | -0.112651 | Yes |
| Laminarity_P | -0.6526684 | -0.156536 | -0.613885 | Yes |
| Laminarity_Pb | -0.4757098 | 0.0270424 | -0.02499 | Yes |
| Laminarity_S | 0.74634502 | 0.0656507 | -0.174632 | Yes |
| Laminarity_Sn | -0.0258771 | -0.059034 | -0.211966 | Yes |
| Laminarity_Sr | -0.0391883 | 0.1600092 | 0.1981753 | Yes |
| Laminarity_Zn | -0.1632985 | -0.218497 | -0.342863 | Yes |
| Laminarity_ZnBa | -0.1445399 | -0.026258 | -0.013961 | No |
| Laminarity_ZnCa | -0.0237264 | -0.192702 | -0.074107 | No |
| Laminarity_ZnCr | -0.1550697 | -0.233279 | 0.1056167 | No |
| Laminarity_ZnCu | -0.0559319 | 0.0193648 | 0.0287148 | No |
| Laminarity_ZnFe | -0.1135849 | -0.465737 | -0.076332 | No |
| Laminarity_ZnI | -0.2049815 | -0.278001 | -0.426026 | No |
| Laminarity_ZnMg | 0.01570192 | -0.166143 | -0.067021 | No |
| Laminarity_ZnMn | -0.2066339 | -0.437386 | -0.449783 | No |
| Laminarity_ZnP | -0.3425974 | -0.188652 | -0.194951 | No |
| Laminarity_ZnPb | -0.2914047 | 0.057845 | -0.111126 | No |
| Laminarity_ZnS | 0.24119318 | 0.2077668 | -0.395565 | No |
| Laminarity_ZnSn | -0.1111781 | -0.097204 | -0.229503 | No |
| Laminarity_ZnSr | -0.1221951 | -0.137035 | -0.116571 | No |
| TT_Ba | 0.02051527 | 0.0235494 | 0.0713402 | Yes |
| TT_Ca | 0.00141797 | -0.019106 | 0.020209 | Yes |
| TT_Cr | -0.0517325 | -0.049081 | -0.00155 | Yes |
| TT_Cu | -0.0481607 | 0.0075708 | -1.95E-05 | Yes |
| TT_Fe | 0.19242796 | -0.110022 | -0.01397 | Yes |
| TT_I | -0.095872 | 0.0490718 | -0.149766 | Yes |
| TT_Mg | 0.02247774 | 0.0086803 | 0.0083814 | Yes |
| TT_Mn | -0.0306581 | -0.178184 | -0.193638 | Yes |
| TT_P | -0.2443903 | 0.0237726 | -0.217035 | Yes |
| TT_Pb | -0.124633 | -0.015009 | 0.0165998 | Yes |
| TT_S | 0.14002116 | 0.0316466 | -0.033064 | Yes |
| TT_Sn | 0.08078177 | 0.014262 | -8.82E-05 | Yes |
| TT_Sr | -0.0329912 | 0.0347752 | 0.0433465 | Yes |
| TT_Zn | -0.0330303 | -0.035842 | -0.100424 | Yes |
| TT_ZnBa | -0.0259845 | -0.000934 | 0.0254744 | Yes |
| TT_ZnCa | -0.008845 | -0.022942 | -0.00739 | No |
| TT_ZnCr | -0.0031782 | -0.072472 | 0.0274496 | No |
| TT_ZnCu | -0.0629332 | 0.0155481 | 0.0080091 | No |
| TT ZnFe | -0.0025839 | -0.04601 | -0.002762 | No |
| TT_ZnI | -0.0386104 | -0.029132 | -0.06475 | No |
| TT_ZnMg | -0.0045818 | -0.018092 | -0.010328 | No |
| TT_ZnMn | -0.0104633 | -0.205922 | -0.02292 | No |
| TT_ZnP | -0.1875745 | 0.0690681 | 0.0053797 | No |
| TT_ZnPb | -0.0955299 | -0.014054 | -0.009815 | No |
| TT_ZnS | 0.13780415 | 0.1133418 | -0.037847 | No |
| TT_ZnSn | 0.02869677 | 0.018639 | 0.0222917 | No |
| TT_ZnSr | -0.006393 | -0.024858 | -0.009046 | No |
| RT2_Ba | 0.01753878 | -0.005345 | 0.0096051 | Yes |
| RT2_Ca | 0.0057303 | 0.0147895 | 0.0100111 | Yes |
| RT2_Cr | -0.0109448 | 0.035535 | 0.0976647 | Yes |
| RT2_Cu | -0.0054492 | -0.019742 | -0.005034 | Yes |
| RT2_Fe | 0.01192527 | 0.0086596 | -0.005167 | No |
| RT2_I | 0.10029701 | -0.002962 | 0.0345432 | Yes |
| RT2_Mg | 0.00441249 | -0.003118 | -0.001238 | Yes |
| RT2_Mn | 0.01166653 | 0.0282244 | 0.0445817 | Yes |
| RT2_P | -0.0467173 | -0.014714 | -0.030091 | Yes |
| RT2_Pb | -0.0356385 | 0.0121896 | -0.005266 | Yes |
| RT2_S | -0.0126324 | 0.0037994 | 0.0116706 | Yes |
| RT2_Sn | 0.01129278 | -0.051285 | -0.021345 | Yes |
| RT2_Sr | 0.0080479 | 0.012611 | 0.0044624 | Yes |
| RT2_Zn | 0.00181413 | 0.0131742 | 0.0078403 | Yes |
| RT2_ZnBa | 0.01226505 | -0.007477 | -0.001362 | Yes |
| RT2_ZnCa | 0.00551711 | 0.0145315 | 0.0114526 | Yes |
| RT2_ZnCr | -0.0162167 | 0.0371156 | 0.0076685 | Yes |
| RT2_ZnCu | -0.0044728 | 0.0003192 | -6.73E-05 | Yes |
| RT2_ZnFe | 0.02795988 | 0.0166935 | 0.0145322 | Yes |
| RT2_ZnI | 0.02424935 | 0.0020863 | 0.0189538 | Yes |
| RT2_ZnMg | 0.0031477 | -5.67E-05 | -0.008444 | Yes |
| RT2_ZnMn | 0.00746097 | 0.0225867 | 0.0065851 | Yes |
| RT2_ZnP | -0.0250943 | -0.001581 | -0.024449 | Yes |
| RT2_ZnPb | -0.0041028 | 0.0130104 | -0.0014 | Yes |
| RT2_ZnS | -0.0076205 | 0.0106253 | 0.0129481 | Yes |
| RT2_ZnSn | -0.0038046 | -0.012371 | -0.017383 | Yes |
| RT2_ZnSr | 0.00105254 | 0.008575 | -0.00386 | Yes |

### Example 4- Amyotrophic lateral sclerosis (ALS)

ALS participants, meeting revised EI Escorial Word Federation of Neurology criteria (N = 36) were recruited at an ALS clinic. Clinical and family history data were obtained. Age- and sex-matched control participants were recruited at the Oral Surgery Clinic. Control subjects (N = 31) were excluded if they or a first- or second-degree family member had a neurodegenerative disease. Participants or next of kin provided informed consent.

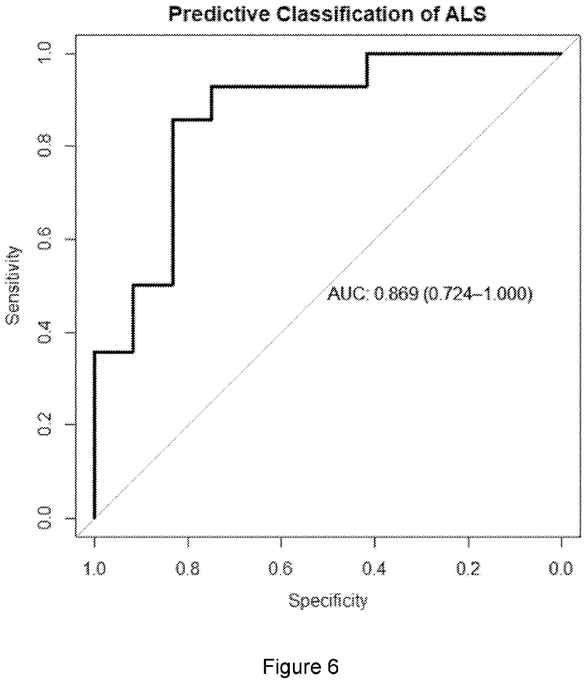

For ALS, the evaluation was performed from tooth samples. Table 6 illustrates the features used and their corresponding *β* values. The *β* values are obtained by estimating each feature in the respective cohort that describes a change in log odds of ALS status associated with a 1-unit change for a respective feature. Figure 6 illustrates the experimental ROC curve for evaluating accuracy of the disclosed method of evaluating ALS across the cohort. As shown in Figure 6, the ROC curve derived from experimental data to evaluate the performance of the disclosed classification method has an AUC corresponding to 0.869, indicating that the disclosed method has 85% accuracy across the cohort for evaluation of ALS based on tooth samples.

**Table 6. Features with empirical x values for a subject based on a tooth sample of the subject for evaluating the subject for ALS.**

| **Feature** | ***β* value** | **Feature in Table 2 or 3?** |
|---|---|---|
| (Intercept) | 83.96232 | No |
| result. Cu.MaxFreq | -1.27115 | No |
| result.Li.MaxFreq | -0.0579 | No |
| result.Mg.MaxFreq | -267.415 | No |
| result.Mn.MaxFreq | 23.69603 | No |
| result.Zn.MaxFreq | -35.6081 | No |
| Determinism_Cu | 48.30136 | Yes |
| Determinism_Li | -96.9188 | Yes |
| Determinism_Mg | 43.43997 | Yes |
| Determinism_Mn | 63.09591 | Yes |
| Determinism Zn | 123.8952 | No |
| Entropy_Cu | -68.1936 | Yes |
| Entropy_Li | 61.47467 | Yes |
| Entropy_Mg | -3.63648 | Yes |
| Entropy_Mn | -17.6021 | Yes |
| Entropy _Zn | -28.8004 | Yes |
| MDL_Cu | 8.374507 | Yes |
| MDL_Li | -11.6838 | Yes |
| MDL_Mg | 83.96232 | Yes |
| MDL_Mn | -1.27115 | Yes |
| MDL Zn | -0.0579 | Yes |

### Example 5- Schizophrenia

Participants with a DSM-IV diagnosis of schizophrenia were selected from the Genetic Risk and OUtcome of Psychosis (GROUP) study (n = 20) and unaffected siblings were used as controls (n = 7). Severity of positive symptoms, negative symptoms, and general psychopathology were assessed by the Positive and Negative Symptom Scale (PANSS). In addition, participants with a DSM-IV diagnosis of schizophrenia (n = 25) and controls (n = 24) were selected from the Avon Longitudinal Study of Parents and Children (ALSPAC), a prospective longitudinal cohort study based in the UK. Presence of DSM-IV schizophrenia in ALSAPC was determined at age 18 and 24 using a semi-structured interview based on the Schedules for Clinical Assessment in Neuropsychiatry psychosis section (SCAN version 2.0).

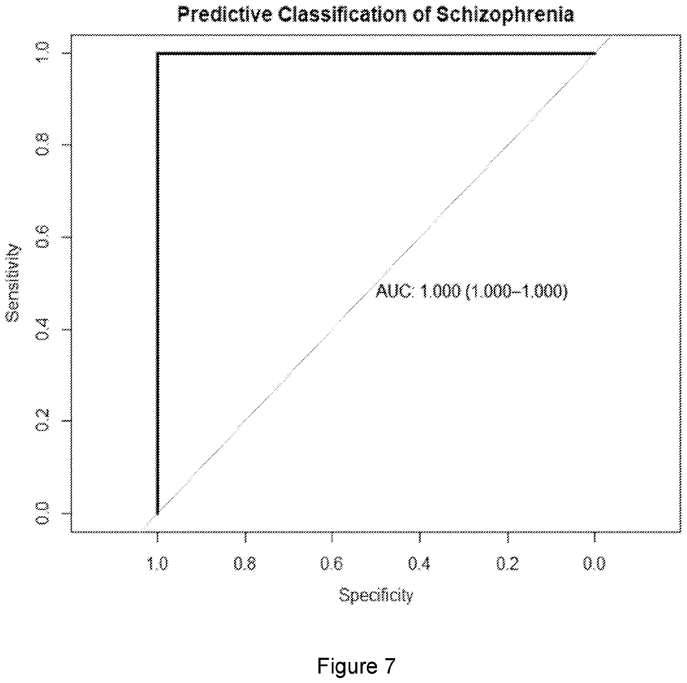

For schizophrenia, the evaluation was performed from tooth samples. Table 7 illustrates the features used and their corresponding *β* values. The *β* values are obtained by estimating each feature in the respective cohort that describes a change in log odds of schizophrenia status associated with a 1-unit change for a respective feature. Figure 7 illustrates the experimental ROC curve for evaluating schizophrenia across the cohort. As shown in Figure 7, the ROC curve has an AUC corresponding to 1.000, indicating that the disclosed method has 100% accuracy in determining schizophrenia based on tooth samples across the cohort.

**Table 7. Features with empirical x values for a subject based on a tooth sample of the subject for evaluating the subject for schizophrenia.**

| **Feature** | ***β* value** | **Feature in Table 2 or 3?** |
|---|---|---|
| Determinism_Sr _ | -0.457644394 | Yes |
| DeterminismMn | -0.442460904 | Yes |
| Determinism_Mg | -0.402732595 | **Yes** |
| Determinism Zn | -0.384259825 | No |
| Determinism_Ca | -0.382835469 | Yes |
| Determinism Li | -0.335344646 | Yes |
| Determinism_As | -0.320110102 | Yes |
| Determinism_Al | -0.318766205 | Yes |
| Determinism_Ba | -0.277937215 | Yes |
| Determinism_Cu | -0.259516956 | Yes |
| Determinism_Se | -0.245987898 | No |
| Determinism_Cr | -0.244131668 | Yes |
| Determinism_Pb | -0.236575919 | Yes |
| Determinism_Ni | -0.230505691 | Yes |
| Determinism_Sn | -0.225723456 | Yes |
| Determinism_Co | -0.198677773 | Yes |
| Entropy_Li | -0.129943086 | Yes |
| Entropy_Al | -0.120001216 | Yes |
| Entropy_Zn | -0.116505828 | Yes |
| Entropy_Mg | -0.110037638 | Yes |
| Entropy_As | -0.109300067 | Yes |
| Entropy_Ca | -0.105637312 | Yes |
| Entropy_Mn | -0.101053528 | Yes |
| MDL_Li | -0.095287157 | Yes |
| Entropy_Se | -0.090043146 | No |
| MDL_Al | -0.086620564 | Yes |
| Entropy_Pb | -0.084836256 | Yes |
| Entropy_Sr | -0.084833755 | Yes |
| Entropy_Cu | -0.084363276 | Yes |
| Entropy_Sn | -0.083399856 | Yes |
| Entropy_Cr | -0.081410354 | Yes |
| Entropy_Ni | -0.080268765 | Yes |
| Entropy_Co | -0.072165671 | Yes |
| MDL As | -0.069228208 | Yes |
| MDL_Se | -0.058812871 | No |
| MDL_Zn | -0.058073407 | Yes |
| Entropy_Ba | -0.053496347 | Yes |
| MDL_Mg | -0.050907767 | Yes |
| MDL_Ca | -0.048437027 | Yes |
| MDL_Sn | -0.045623074 | Yes |
| MDL_Pb | -0.04510374 | Yes |
| MDL_Cr | -0.041331302 | Yes |
| MDL_Cu | -0.040429247 | Yes |
| MDL_Co | -0.039999687 | Yes |
| MDL_Ni | -0.03813012 | Yes |
| MDL_Mn | -0.034183415 | Yes |
| MDL_Sr | -0.024060458 | Yes |
| MDL_Ba | -0.012524148 | Yes |
| MDL_Bi | 0.052480638 | No |
| Entropy_Bi | 0.118716291 | Yes |
| Determinism_Bi | 0.336109649 | Yes |

### Example 6- Irritable bowel disease (IBD)

Subjects were recruited from a study based in Portugal. Tooth samples were obtained from 11 patients diagnosed with IBD (Chron's Disease = 6, ulcerative colitis/indeterminate colitis = 5) and 16 unaffected controls. All participants were born and grew up in the same Portuguese Province. Each subject was evaluated for IDB using a similar method as described above with respect to Examples 2 and 3. For IDB the evaluation was performed from a tooth sample. Table 8 illustrates the features used and their corresponding *β* values. The *β* values are obtained by estimating each feature in the respective cohort that describes a change in log odds of IBD status associated with a 1-unit change for a respective feature.

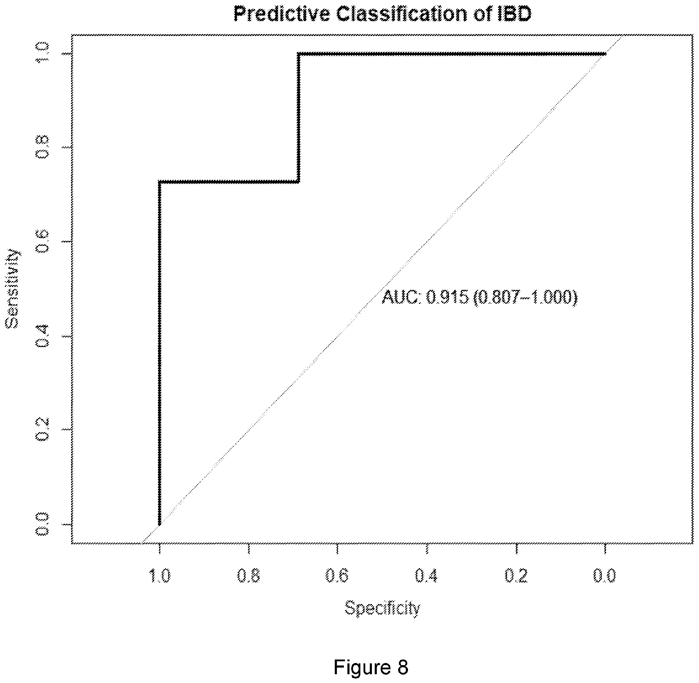

Figure 8 illustrates experimental ROC curves for evaluating accuracy of the disclosed method of evaluating a subject for schizophrenia. As shown in Figure 8, the ROC curve derived from experimental data to evaluate the performance of the disclosed classification method has an AUC corresponding to 0.915, indicating that the disclosed method has above 90 % accuracy for IBD determination based on tooth samples.

**Table 8. Features with empirical x values for IBD.**

| **Feature** | ***β* value** | **Feature in Table 2 or 3?** |
|---|---|---|
| Determinism_Pb | 0.837721661 | Yes |
| Determinism_CoCd | 0.608033699 | No |
| Determinism_NiCd | 0.583651036 | No |
| Determinism_Mn | 0.580065903 | Yes |
| Determinism_ZnSn | 0.579284697 | Yes |
| Determinism_AlPb | 0.577467897 | No |
| Determinism_Bi | 0.537065232 | Yes |
| Determinism_SrPb | 0.493960945 | No |
| Determinism_CoCu | 0.463814561 | No |
| Determinism_CoAs | 0.456605412 | No |
| Determinism_SrSn | 0.447913275 | No |
| Determinism_NiMo | 0.446153665 | No |
| Determinism_NiSn | 0.442477131 | No |
| Determinism_CoSn | 0.440085861 | No |
| Determinism_MnCu | 0.420156241 | No |
| Determinism_AlAs | 0.394030337 | No |
| Determinism_AlCa43 | 0.393499257 | No |
| Determinism_AlMn | 0.388228381 | No |
| Determinism_CoNi | 0.369794172 | No |
| Determinism_Ca43Cu | 0.367746635 | No |
| Determinism_AlCu | 0.367706871 | No |
| Determinism_NiPb | 0.366479003 | No |
| Determinism_CuPb | 0.365132868 | No |
| Determinism_CoZn | 0.358897573 | No |
| Determinism_SnBa | 0.348806193 | No |
| Determinism_NiBi | 0.347509524 | No |
| Determinism_Ca43Ba | 0.338250186 | No |
| Determinism_CoSr | 0.335913385 | No |
| Determinism_CoPb | 0.330166861 | No |
| Determinism_AlSr _ | 0.327066202 | No |
| Determinism_CrCu | 0.323166738 | No |
| Determinism_NiAs | 0.319802598 | No |
| Determinism_MnAs | 0.303183281 | No |
| Determinism_AlBa | 0.283176281 | No |
| Determinism_MnSn | 0.277834073 | No |
| Determinism_Cu | 0.272437834 | No |
| Determinism_NiZn | 0.271167365 | No |
| Determinism_AlSn | 0.266885502 | No |
| Determinism_CdSn | 0.264283744 | No |
| Determinism_SrMo | 0.258823829 | No |
| Determinism_SnPb | 0.258076389 | No |
| Determinism_Co | 0.251749701 | Yes |
| Determinism_AlZn | 0.251072583 | No |
| Determinism_Ni | 0.243471714 | Yes |
| Determinism_MnNi | 0.236046191 | No |
| Determinism_CuBa | 0.225800693 | No |
| Determinism _Ca43Pb | 0.221077979 | No |
| Determinism_AsSr | 0.21986349 | No |
| Determinism_AlNi | 0.208256778 | No |
| Determinism_Mg25Pb | 0.204110935 | No |
| Determinism_CoBi | 0.192229974 | No |
| Determinism_AlBi | 0.19174288 | No |
| Determinism_NiCu | 0.189023023 | No |
| Determinism_Al | 0.188772179 | Yes |
| Determinism_MnBi | 0.181404932 | No |
| Determinism_SrBi | 0.179589562 | No |
| Determinism_CoMo | 0.178920834 | No |
| Determinism_CuSr | 0.173849996 | No |
| Determinism_AlCr | 0.169620126 | No |
| Determinism_Sn | 0.157158501 | Yes |
| Entropy_MnCu | 0.150045454 | No |
| Determinism_CoBa | 0.14806543 | No |
| Determinism_SnBi | 0.146080673 | No |
| Determinism_MnCo | 0.13689247 | No |
| Determinism_AlMo | 0.135095212 | No |
| Entropy_SrBi | 0.133631105 | No |
| Determinism_MnBa | 0.131543875 | No |
| Determinism_BaPb | 0.129043442 | No |
| Determinism_NiSr | 0.118585723 | No |
| Entropy_NiMo | 0.117468751 | No |
| Determinism_SrBa | 0.110628434 | No |
| Determinism_CdBa | 0.105904929 | No |
| Entropy_Pb | 0.096701332 | Yes |
| Entropy_LiNi | 0.096600763 | No |
| Entropy_AlNi | 0.096261656 | No |
| Entropy_LiCr | 0.095557786 | No |
| Determinism_AlCd | 0.093269413 | No |
| Entropy_NiCd | 0.090526996 | No |
| Entropy_Mn | 0.088216945 | Yes |
| Entropy_AsSr | 0.086331274 | No |
| Determinism_Mg25Cu | 0.085901097 | No |
| Entropy_LiCa43 | 0.085357299 | No |
| Entropy_MnNi | 0.085231831 | No |
| Entropy_MoCd | 0.084513633 | No |
| Entropy_AlCr | 0.083519607 | No |
| Entropy_AlSn | 0.081448169 | No |
| Entropy_LiAl | 0.078847087 | No |
| Entropy_Sn | 0.07782499 | Yes |
| Determinism_Mg25Ni | 0.077455521 | No |
| Determinism_MoSn | 0.074550109 | No |
| Entropy_NiAs | 0.073763013 | No |
| Determinism_AsBa | 0.073113685 | No |
| Determinism_Mg25Mo | 0.072837473 | No |
| Determinism_Mg25Ca43 | 0.066896429 | No |
| Entropy_CuSr | 0.066616184 | No |
| Entropy_MnBi | 0.065186033 | No |
| Entropy_SrSn | 0.064276558 | No |
| Determinism_ZnBi | 0.063181991 | No |
| Entropy_LiZn | 0.06274205 | No |
| Entropy_NiSn | 0.062065578 | No |
| Entropy_AlCd | 0.061932776 | No |
| Entropy_MnSn | 0.061917389 | No |
| Entropy_AlAs | 0.060589179 | No |
| Entropy_LiAs | 0.059907306 | No |
| Entropy_AlCu | 0.059570784 | No |
| MDL_NiPb | 0.059356348 | No |
| Determinism_CuSn | 0.057883324 | No |
| Entropy_CoAs | 0.056975722 | No |
| Entropy_CoCd | 0.056619699 | No |
| Entropy_AlBi | 0.056592751 | No |
| Entropy_LiSn | 0.055407714 | No |
| MDL_NiCu | 0.05462465 | No |
| Determinism_Sr | 0.052769123 | Yes |
| MDL_SrBi | 0.052757632 | No |
| Entropy_MnCd | 0.051491996 | No |
| MDL_AlCu | 0.051084234 | No |
| Entropy_MnZn | 0.05095569 | No |
| MDL_NiCd | 0.050821168 | No |
| MDL NiAs | 0.049999502 | No |
| MDL_NiMo | 0.049942464 | No |
| MDL _LiPb | 0.049236366 | No |
| Entropy_BaBi | 0.04888417 | No |
| Entropy_AlSr | 0.048461583 | No |
| Entropy_NiZn | 0.04818889 | No |
| MDL_MnNi | 0.045506307 | No |
| Entropy_ZnBa | 0.045366615 | Yes |
| MDL_CoAs | 0.045133938 | No |
| MDL_AlNi | 0.044994651 | No |
| Entropy_Cu | 0.044750116 | Yes |
| MDL_AlPb | 0.044651067 | No |
| MDL_NiSn | 0.04447437 | No |
| Determinism_CuZn | 0.04346297 | No |
| MDL_MnBi | 0.04293887 | No |
| Entropy_SrCd | 0.041911866 | No |
| Entropy_CoNi | 0.040717095 | No |
| Entropy_SnBa | 0.040439261 | No |
| MDL_LiCu | 0.040400882 | No |
| MDL_CoNi | 0.039722706 | No |
| MDL_CrCu | 0.039141039 | No |
| MDL_Sn | 0.039030533 | Yes |
| Determinism_LiCu | 0.03819333 | No |
| Entropy_MnAs | 0.037845825 | No |
| Entropy_LiMo | 0.037836232 | No |
| Entropy_Mg25Cu | 0.037830094 | No |
| MDL_CoSn | 0.037703149 | No |
| MDL_AlCr | 0.036913043 | No |
| MDL_ZnPb | 0.036670246 | Yes |
| Entropy_LiCu | 0.035253147 | No |
| MDL_MoCd | 0.034863673 | No |
| MDL_AlSn | 0.033729785 | No |
| MDL_AlCd | 0.033444913 | No |
| Entropy_Mg25Ca43 | 0.033104137 | No |
| MDL_MnCd | 0.032959444 | No |
| Entropy_LiBi | 0.032933544 | No |
| MDL_BaBi | 0.032773839 | No |
| Determinism_CrBa | 0.03267654 | No |
| Entropy_CuBa | 0.032551901 | No |
| Entropy_LiPb | 0.03241727 | No |
| Entropy_AlPb | 0.031942708 | No |
| Determinism_MnMo | 0.03151546 | No |
| MDL_AsSr | 0.030340482 | No |
| MDL_Pb | 0.030277868 | Yes |
| MDL_CoCd | 0.030102262 | No |
| Entropy_AsPb | 0.029893974 | No |
| Entropy_SrMo | 0.028854133 | No |
| MDL_MnAs | 0.028558981 | No |
| MDL_Bi | 0.028474966 | No |
| MDL_AlBi | 0.028265509 | No |
| Entropy_CrSr | 0.028254263 | No |
| MDL_Mn | 0.02754259 | Yes |
| Entropy_LiMg25 | 0.02719571 | No |
| MDL_CuSn | 0.026802102 | No |
| Entropy_Al | 0.026660621 | Yes |
| MDL_SrSn | 0.026417738 | No |
| Determinism_BaBi | 0.026118071 | No |
| MDL_CuMo | 0.025833509 | No |
| MDL_Ca43Cu | 0.025021625 | No |
| Entropy_CrBa | 0.024590968 | No |
| Entropy_CuSn | 0.023936374 | No |
| Entropy_SnBi | 0.023796602 | No |
| Entropy_NiBi | 0.023666703 | No |
| Entropy_BaPb | 0.02316239 | No |
| MDL_SnBi _ | 0.022967977 | No |
| MDL_MnZn | 0.022794853 | No |
| Entropy_AsBa | 0.022724576 | No |
| Entropy_AlBa | 0.022251972 | No |
| MDL_MnSn | 0.022230802 | No |
| MDL_ZnAs | 0.022102064 | Yes |
| Entropy_ZnAs | 0.021389434 | Yes |
| MDL_BaPb | 0.021250278 | No |
| MDL_NiZn | 0.020720792 | No |
| Entropy_Ba | 0.020553585 | Yes |
| Entropy_Co | 0.019950137 | Yes |
| Entropy_NiPb | 0.019835982 | No |
| MDL_AlAs | 0.019594507 | No |
| MDL_CuSr | 0.019428846 | No |
| Determinism_CdPb | 0.019354351 | No |
| MDL_Cr | 0.019090509 | Yes |
| Entropy_NiCu | 0.018579507 | No |
| MDL_MnPb | 0.018264661 | No |
| Entropy_Cr | 0.017852524 | Yes |
| MDL_AsPb | 0.017053517 | No |
| MDL_Al | 0.016864812 | Yes |
| Entropy_ZnPb | 0.016837243 | Yes |
| MDL_NiBi | 0.015778181 | No |
| MDL_AlBa | 0.015732134 | No |
| Determinism_Ba | 0.015387888 | Yes |
| Entropy_CoSn | 0.015061341 | No |
| MDL_CuBi | 0.014143895 | No |
| Entropy_AlZn | 0.014101886 | No |
| Entropy_CoMo | 0.014040446 | No |
| Entropy_CuMo | 0.013876978 | No |
| MDL_CrBa | 0.013871868 | No |
| MDL_Ba | 0.013657256 | Yes |
| MDL_MnCu | 0.013536051 | No |
| Entropy_Bi | 0.013378595 | Yes |
| MDL_CrNi | 0.013115327 | No |
| MDL_CuBa | 0.012143264 | No |
| MDL_SrCd | 0.011897196 | No |
| Entropy_Ca43Sr | 0.011668152 | No |
| Entropy_ZnSn | 0.010322362 | Yes |
| Entropy_Mg25Cr | 0.010258145 | No |
| Entropy_CuBi | 0.009857028 | No |
| MDL_SnBa | 0.009355043 | No |
| MDL_CoPb | 0.009250621 | No |
| MDL_ZnSn | 0.009214463 | Yes |
| MDL_CdPb | 0.008436296 | No |
| MDL_ZnBa | 0.008373169 | Yes |
| MDL_CrSn | 0.00831045 | No |
| MDL_CoMo | 0.008195326 | No |
| MDL_CrPb | 0.008157123 | No |
| MDL_Mg25Ca43 | 0.00808495 | No |
| MDL_CoZn | 0.008064695 | No |
| Entropy_MnBa | 0.00798082 | No |
| Entropy_NiSr | 0.007615698 | No |
| MDL_Mg25Cu | 0.007513622 | No |
| MDL_CrZn | 0.007458255 | No |
| MDL_Cu | 0.007404707 | Yes |
| MDL_AlSr | 0.006611509 | No |
| Entropy_Ni | 0.006471257 | No |
| MDL_AsBa | 0.006254217 | No |
| MDL_MnBa | 0.00598819 | No |
| Entropy_Ca43Cu | 0.005658041 | No |
| MDL_Mg25Pb | 0.00559757 | No |
| Determinism_LiNi | 0.004788003 | No |
| MDL_AlZn | 0.004786941 | No |
| MDL_CuZn | 0.004075936 | No |
| MDL_Mg25Sn | 0.003699523 | No |
| MDL_MnMo | 0.003341513 | No |
| Entropy_CoPb | 0.002872541 | No |
| MDL_SrPb | 0.0026864 | No |
| MDL_LiCa43 | 0.002653581 | No |
| MDL_SnPb | 0.002566204 | No |
| MDL_CrSr | 0.002467427 | No |
| MDL_CoSr | 0.002339729 | No |
| MDL_Sr | 0.002243815 | Yes |
| Entropy_Mg25Pb | 0.002122864 | No |
| MDL_LiNi | 0.001696513 | No |
| Entropy_CrCu | 0.001582979 | No |
| MDL_SrBa | 0.001162027 | No |
| Entropy_SnPb | 0.000916207 | No |
| Determinism_Mg25 | 0.000803475 | No |
| MDL**_**Co | 0.00025716 | Yes |
| Entropy_⁻AlCa43 | -1.38E-05 | No |
| MDL_NiBa | -0.000244236 | No |
| MDL_Ca43Sn | -0.001253881 | No |
| MDL_SrMo | -0.001497868 | No |
| Entropy_Mg25Sn | -0.001842896 | No |
| MDL_CrAs | -0.002029876 | No |
| MDL_Ni | -0.002069796 | Yes |
| Entropy_MnPb | -0.00291857 | No |
| MDL_AsCd | -0.002927082 | No |
| MDL_LiBi | -0.003023387 | No |
| MDL_CuAs | -0.003515146 | No |
| Entropy_LiBa | -0.003785078 | No |
| MDL_AlCa43 | -0.003900959 | No |
| Entropy_CrZn | -0.005150422 | No |
| Determinism_Ca43 | -0.005493317 | No |
| Entropy_Ca43 | -0.005523451 | No |
| MDLA_lMn | -0.005635517 | No |
| MDL_Zn | -0.006177405 | Yes |
| Entropy_CoSr | -0.006810491 | No |
| Entropy_AsCd | -0.007060322 | No |
| Determinism_CrSr | -0.007322712 | No |
| Determinism_SrCd | -0.007335886 | No |
| MDL_Mg25Ba | -0.007450877 | No |
| Entropy_Sr | -0.007681616 | Yes |
| MDL_AlMo | -0.007777482 | No |
| MDL_MoSn | -0.007813266 | No |
| MDL_MnSr | -0.0078902 | No |
| MDL_CoBa | -0.007987333 | No |
| MDL_Mg25Cr | -0.008021723 | No |
| MDL_CoBi | -0.008026512 | No |
| MDL_LiBa | -0.008125012 | No |
| MDL_Ca43Pb | -0.00876952 | No |
| MDL_CuCd | -0.009012709 | No |
| MDL_CrBi | -0.009417947 | No |
| MDL**_**Ca43Sr | -0.009530661 | No |
| MDL_LiSr | -0.00958387 | No |
| MDL_CoCu | -0.010617794 | No |
| MDL_LiZn | -0.010869207 | No |
| Determinism_Mg25Cd | -0.011141724 | No |
| MDL_NiSr | -0.01145434 | No |
| MDL_Ca43 | -0.011517201 | No |
| Entropy_LiMn | -0.01154657 | No |
| MDL_Mg25 | -0.011778792 | No |
| MDL_LiMn | -0.012781052 | No |
| Entropy_CrPb | -0.012946148 | No |
| MDL_Mg25Zn | -0.013235864 | No |
| Determinism_AlCo | -0.013262931 | No |
| Entropy_Ca43Sn | -0.013327267 | No |
| Entropy_AlMo | -0.013771315 | No |
| Entropy_MnMo | -0.014437184 | No |
| MDL_LiMg25 | -0.01450352 | No |
| MDL_MoBi | -0.014867918 | No |
| Entropy_SrPb | -0.015029508 | No |
| MDL_CrMo | -0.015069864 | No |
| MDL_CrMn | -0.015081756 | No |
| MDL_CuPb | -0.015290387 | No |
| MDL_CrCd | -0.01558969 | No |
| MDL_Mo | -0.016203811 | No |
| MDL**_**Ca43As | -0.016903291 | No |
| MDL_LiCr | -0.016947259 | No |
| MDL_PbBi | -0.017265814 | No |
| Entropy_AlMn | -0.017868533 | No |
| Entropy_SrBa | -0.017897945 | No |
| Entropy_LiCd | -0.018744594 | No |
| MDL_Ca43Cr | -0.019023403 | No |
| MDL_AlCo | -0.019624384 | No |
| Entropy_Zn | -0.019976226 | Yes |
| MDL_Mg25Sr | -0.02039631 | No |
| Entropy_CrAs | -0.020718782 | No |
| MDL_ZnBi | -0.020858078 | Yes |
| MDL _Ca43Ba | -0.021093231 | No |
| MDL_LiAl | -0.021601851 | No |
| MDL_LiAs | -0.021825883 | No |
| MDL_Mg25Ni | -0.021988545 | No |
| Entropy_CoBi | -0.022110929 | No |
| MDL_Mg25Al | -0.023379235 | No |
| Entropy_LiSr | -0.023993698 | No |
| Entropy_CrNi | -0.024059903 | No |
| MDL_Ca43Bi | -0.025026622 | No |
| MDL_AsBi | -0.025351338 | No |
| Determinism_CrMn | -0.025693548 | No |
| Entropy_NiBa | -0.025877792 | No |
| MDL_Mg25As | -0.027214955 | No |
| Entropy_CrSn | -0.027976947 | No |
| Determinism_LiAs | -0.028028835 | No |
| Entropy_MoSn | -0.028055204 | No |
| MDL_LiCo | -0.028277584 | No |
| MDL_LiSn | -0.029087385 | No |
| MDL_Mg25Bi | -0.029221437 | No |
| Determinism_ZnPb | -0.029334441 | Yes |
| MDL_Li | -0.030323494 | No |
| Determinism_MoCd | -0.03175983 | No |
| Entropy_Ca43Cr | -0.032616587 | No |
| Entropy_Mg25 | -0.032844403 | No |
| MDL_CrCo | -0.033010033 | No |
| Determinism_Mg25Mn | -0.033143092 | No |
| Entropy_CuZn | -0.033520671 | No |
| Entropy_Mg25Al | -0.034991785 | No |
| MDL_Ca43Mn | -0.035057 | No |
| MDL_Ca43Zn | -0.035089518 | No |
| Determinism_Mg25As | -0.035099283 | No |
| Entropy_Mg25Ba | -0.03522107 | No |
| MDL_LiCd | -0.035288476 | No |
| Entropy_Ca43As | -0.03555932 | No |
| MDL_Mg25Mn | -0.036626043 | No |
| Entropy_MnSr | -0.036794479 | No |
| MDL_MoPb | -0.036998779 | No |
| Determinism_Mg25Ba | -0.037297617 | No |
| MDL_Mg25Cd | -0.037841873 | No |
| Entropy_CoZn | -0.038888092 | No |
| MDL_Ca43Cd | -0.0398772 | No |
| MDL_ZnSr | -0.039929996 | Yes |
| Entropy_CrBi | -0.040063787 | No |
| MDL_MoBa | -0.040294352 | No |
| Entropy_PbBi | -0.040644702 | No |
| Entropy_Mg25Zn | -0.040674759 | No |
| Entropy_CoBa | -0.041682071 | No |
| MDL_AsSn | -0.042482389 | No |
| Determinism_AsCd | -0.042590229 | No |
| MDL**_**As | -0.042816404 | Yes |
| MDL_LiMo | -0.042942789 | No |
| Entropy_Mo | -0.04356696 | No |
| MDL_Ca43Ni | -0.044126031 | No |
| Entropy_LiCo | -0.044596271 | No |
| Entropy_Ca43Ba | -0.045718289 | No |
| MDL_Mg25Mo | -0.046804847 | No |
| Entropy_Mg25As | -0.046981031 | No |
| MDL_Mg25Co | -0.04714293 | No |
| Entropy_CrMn | -0.047468897 | No |
| Entropy_AsBi | -0.049035215 | No |
| Entropy_ZnBi | -0.049648752 | Yes |
| Entropy_Mg25Ni | -0.049731513 | No |
| Entropy_CrMo | -0.049796621 | No |
| Determinism_Mg25Sn | -0.049818644 | No |
| Determinism_CrAs | -0.051814773 | No |
| MDL_Ca43Mo | -0.051841513 | No |
| Entropy_CuCd | -0.051964319 | No |
| Entropy_MoBi | -0.05278141 | No |
| MDL_MnCo | -0.053184893 | No |
| Entropy_Mg25Sr | -0.053816077 | No |
| MDL_ZnMo | -0.054941163 | No |
| Determinism_CrSn | -0.056475746 | No |
| MDL_ZnCd | -0.056742477 | Yes |
| Entropy_CrCd | -0.057661784 | No |
| Entropy_Ca43Bi | -0.057689103 | No |
| Entropy_Mg25Bi | -0.058660945 | No |
| MDL_CdBa | -0.060345967 | No |
| Determinis_AsPb | -0.062519923 | No |
| Entropy_CuPb | -0.062639141 | No |
| Entropy_CoCu | -0.063222545 | No |
| MDL_Ca43Co | -0.063449824 | No |
| MDL_AsMo | -0.063500069 | No |
| Determinism_CuAs | -0.06375552 | No |
| Entropy_CrCo | -0.066089162 | No |
| Determinism_NiBa | -0.066485204 | No |
| Determinism_MnZn | -0.066897861 | No |
| Determinism_Mg25Cr | -0.067032328 | No |
| Determinism_LiPb | -0.068610383 | No |
| MDL_Cd | -0.070338308 | Yes |
| Entropy_Ca43Cd | -0.070640761 | No |
| Determinism_CrZn | -0.070982816 | No |
| MDL_CdBi | -0.071508472 | No |
| Entropy_CuAs | -0.071694453 | No |
| Entropy_As | -0.073888148 | No |
| Determinism_MnPb | -0.075416383 | No |
| Determinism_AsSn | -0.076106607 | No |
| Entropy_AsSn | -0.077219424 | No |
| Entropy_Li | -0.079541735 | Yes |
| Entropy_Mg25Cd | -0.081355567 | No |
| MDL_CdSn | -0.085397153 | No |
| Determinism_CrCd | -0.086603414 | No |
| Entropy_Ca43Mn | -0.087840267 | No |
| Entropy_MoPb | -0.088690854 | No |
| Determinism_CrNi | -0.08955169 | No |
| Entropy_Ca43Pb | -0.091069981 | No |
| Entropy_ZnMo | -0.092435832 | No |
| Entropy_Mg25Co | -0.094448688 | No |
| Entropy_ZnSr | -0.095115262 | Yes |
| Determinism_Ca43Mn | -0.095454483 | No |
| Entropy_MoBa | -0.096175158 | No |
| Entropy_AlCo | -0.09643681 | No |
| Determinism_AsBi | -0.098945245 | No |
| Determinism_CuMo | -0.100397016 | No |
| Determinism_CrPb | -0.100511882 | No |
| Determinism_CuBi | -0.104531163 | No |
| Determinism_ZnAs | -0.106361378 | Yes |
| Entropy_Ca43Zn | -0.110030137 | No |
| Entropy_MnCo | -0.112253585 | No |
| Determinism_Ca43Bi | -0.116845644 | No |
| Entropy_Mg25Mo | -0.119482329 | No |
| Entropy_Mg25Mn | -0.120712767 | No |
| Entropy_AsMo | -0.125823549 | No |
| Entropy_Ca43Mo | -0.126525635 | No |
| Determinism_Ca43Sr | -0.129139365 | No |
| Determinism_Mg25Al | -0.129310933 | No |
| Determinism_Mg25Co | -0.132447668 | No |
| Entropy_Ca43Ni | -0.135349623 | No |
| Entropy_ZnCd | -0.140786669 | Yes |
| Determinism_MoBi | -0.142377275 | No |
| Entropy_Ca43Co | -0.142669153 | No |
| Determinism_Ca43As | -0.143199433 | No |
| Determinism_Mg25Zn | -0.156576846 | No |
| Determinism_Cr | -0.158077761 | Yes |
| Entropy_CdBa | -0.163994048 | No |
| Determinism_Ca43Sn | -0.164777802 | No |
| Determinism_ZnSr | -0.175344304 | Yes |
| Determinism_CrBi | -0.177702068 | No |
| Entropy_CdPb | -0.177794228 | No |
| Determinism_MoPb | -0.183135158 | No |
| Entropy_CdSn | -0.185633704 | No |
| Entropy_Cd | -0.187420522 | Yes |
| Determinism_ZnBa | -0.191891102 | Yes |
| Determinism_ZnCd | -0.195471091 | Yes |
| Entropy_CdBi | -0.208651743 | No |
| Determinism_MnCd | -0.20928994 | No |
| Determinism_Mg25Sr | -0.212734283 | No |
| Determinism_Ca43Zn | -0.219269022 | No |
| Determinism_LiZn | -0.228405173 | No |
| Determinism_AsMo | -0.229631149 | No |
| Determinism_Ca43Ni | -0.240518024 | No |
| Determinism_As | -0.253072839 | Yes |
| Determinism_Ca43Mo | -0.265534336 | No |
| Determinism_ZnMo | -0.272024886 | No |
| Determinism_MoBa | -0.274507417 | No |
| Determinism_PbBi | -0.274576625 | No |
| Determinism_Ca43Cd | -0.277001258 | No |
| Determinism_Mg25Bi | -0.280768606 | No |
| Determinism_Zn | -0.285103674 | No |
| Determinism_LiCa43 | -0.30617652 | No |
| Determinism_CrMo | -0.308477976 | No |
| Determinism_Ca43Co | -0.30955258 | No |
| Determinism_LiSn | -0.329271649 | No |
| Determinism_MnSr | -0.337554128 | No |
| Determinism_CuCd | -0.34833967 | No |
| Determinism_CrCo | -0.348342147 | No |
| Determinism_LiCd | -0.381890549 | No |
| Determinism_Ca43Cr | -0.437649851 | No |
| Determinism_LiMo | -0.466404292 | No |
| Determinism_LiSr | -0.486704206 | No |
| (Intercept) | -0.535887209 | No |
| Determinism_LiMn | -0.564746942 | No |
| Determinism_LiAl | -0.583409959 | No |
| Determinism_LiMg25 | -0.610883387 | No |
| Determinism_LiBa | -0.620245549 | No |
| Determinism_LiCr | -0.640710424 | No |
| Determinism_Li | -0.669879537 | Yes |
| Determinism_LiCo | -0.689653181 | No |
| Determinism_CdBi | -0.690031813 | No |
| Determinism_Mo | -0.697738514 | No |
| Determinism_LiBi | -0.823539112 | No |
| Determinism_Cd | -1.116088768 | Yes |

### Example 7- kidney transplant rejection prediction

Hair samples were collected from kidney transplant recipients at the time of biopsy-proven acute rejection (n = 6) and age- and sex-matched control kidney transplant recipients with no acute rejection at surveillance biopsy at the same time after transplant (n = 5). All participants were recruited from the Mount Sinai Hospital. Table 9 illustrates the features used and their corresponding *β* values. The *β* values are obtained by estimating each respective feature in the respective cohort that describes a change in log odds of kidney transplate status associated with a 1-unit change for the respective feature.

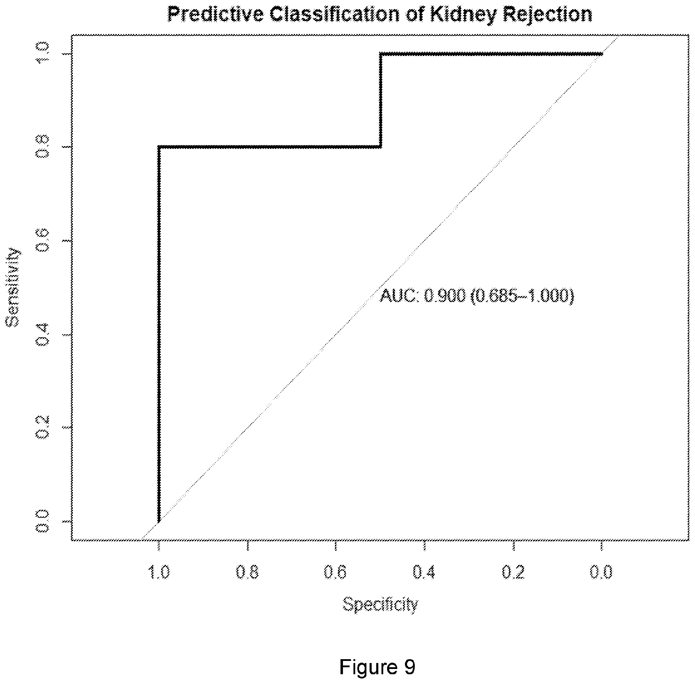

Figure 9 illustrates the ROC curve for evaluating accuracy of the disclosed method of evaluating subjects for kidney transplant rejection. As shown in Figure 9, the ROC curve derived from experimental data to evaluate the performance of the disclosed classification method has an AUC corresponding to 0.900, indicating that the disclosed method has 90% accuracy for evaluating kidney transplant rejection based on a hair sample.

**Table 9. Features with empirical x values for kidney transplant rejection prediction.**

| **Feature** | ***β* value** | **Feature in Table 2 or 3?** |
|---|---|---|
| Determinism_Bi | 2.937600551 | Yes |
| Determinism_Al | 2.276918409 | Yes |
| Determinism_P | 1.593375022 | Yes |
| Determinism_I | 1.576526144 | Yes |
| Determinism_Hg | 1.352609521 | Yes |
| Determinism_Mn | 1.324694388 | Yes |
| Determinism_S | 1.251337611 | Yes |
| Entropy_Bi | 1.057509572 | Yes |
| Determinism_Sn | 0.910193817 | Yes |
| Determinism_Ba | 0.882379546 | Yes |
| Determinism_As | 0.612241143 | Yes |
| Determinism_Pb | 0.410747 | Yes |
| Determinism_Sr | 0.378952574 | Yes |
| MDL_Bi | 0.335770724 | No |
| Entropy_Hg | 0.259591313 | Yes |
| Entropy_Mn | 0.220488734 | Yes |
| Entropy_Zn | 0.170166112 | Yes |
| Entropy_P | 0.169228029 | Yes |
| Entropy_S | 0.165291088 | Yes |
| Entropy_Sn | 0.140767004 | Yes |
| Entropy_I | 0.131182754 | Yes |
| Entropy_Ba | 0.123782562 | Yes |
| Entropy_Ni | 0.119190841 | Yes |
| Entropy_Pb | 0.095014851 | Yes |
| Entropy_Cu | 0.093029191 | Yes |
| MDL_Hg | 0.090579868 | Yes |
| MDL_Mn | 0.070363291 | Yes |
| MDL Zn | 0.069119728 | Yes |
| MDL_Ni | 0.065047882 | Yes |
| Entropy_Mg | 0.060600108 | Yes |
| MDL_S | 0.055566047 | Yes |
| MDL_P | 0.054680842 | Yes |
| MDL_Sn | 0.037235088 | Yes |
| MDL_I | 0.037166283 | Yes |
| MDLPb | 0.027730044 | Yes |
| MDL_Cu | 0.019758971 | Yes |
| MDL_Ba | 0.017861445 | Yes |
| MDL_Mg | 0.013919756 | Yes |
| Entropy_Sr | 0.012056904 | Yes |
| MDL_Sr | 0.011186722 | Yes |
| Determinism_Mg | -0.00081932 | Yes |
| MDL_Ca | -0.006427457 | Yes |
| MDL_Li | -0.020601779 | Yes |
| MDL_Al | -0.028804713 | Yes |
| Determinism_Ca | -0.031452338 | Yes |
| MDL_Co | -0.033385007 | Yes |
| Entropy_Li | -0.044124857 | Yes |
| Determinism_Ni | -0.048105079 | Yes |
| Entropy_Ca | -0.068325786 | Yes |
| MDL_Cr | -0.078030836 | Yes |
| Entropy_Al | -0.09122596 | Yes |
| Entropy_Co | -0.105659709 | Yes |
| MDL_Fe | -0.128893502 | Yes |
| Entropy_Cr | -0.167258915 | Yes |
| MDL_As | -0.171607981 | Yes |
| MDL_Cd | -0.199946789 | Yes |
| Entropy_As | -0.379551271 | Yes |
| Entropy_Fe | -0.404300467 | Yes |
| Entropy_Cd | -0.613573066 | Yes |
| Determinism_Cu | -0.700031087 | Yes |
| Determinism_Co | -0.7229317 | Yes |
| Determinism_Li | -0.927509995 | Yes |
| Determinism_Fe | -1.693252621 | Yes |
| Determinism_Zn | -1.826370946 | No |
| Determinism_Cd | -2.069910206 | Yes |
| Determinism_Cr | -2.585054024 | Yes |
| Intercept | -7.703959822 | No |

### Example 8 - Pediatric cancer

Subjects were evaluated for pediatric cancer using a similar method as described above with respect to Examples 2 and 3. A total of 28 children were recruited from a hospital cancer center. Twenty-two were pediatric cancer cases and 6 were controls. Diagnoses were made using standard clinical protocols-blood testing and histopathology and confirmed by an oncologist. Table 10 illustrates the features used and their corresponding *β* values. The *β* values are obtained by estimating each respective feature in the respective cohort that describes a change in log odds of pediatric cancer status associated with a 1-unit change for the respective feature.

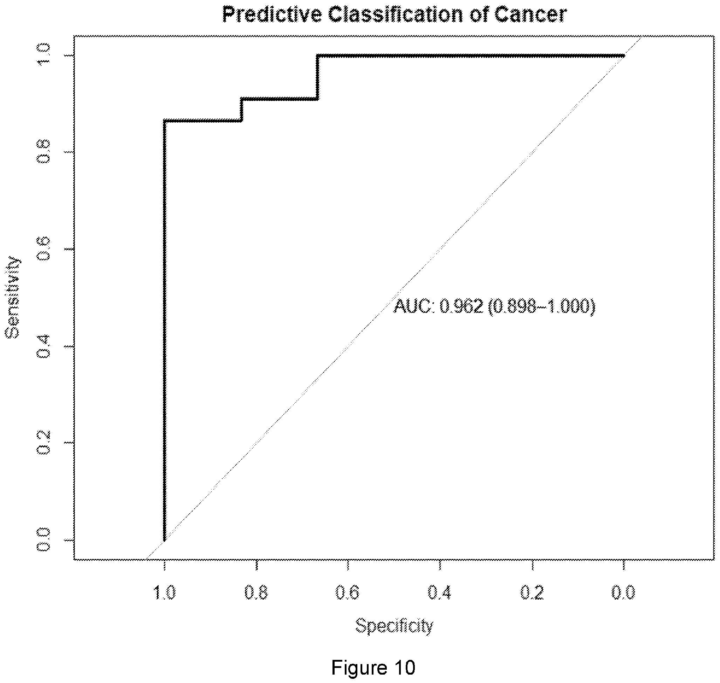

Figure 10 illustrates the ROC curve for evaluating accuracy of the disclosed method of evaluating a subject for pediatric cancer. As shown in Figure 10, the ROC curve derived from experimental data to evaluate the performance of the disclosed classification method has an AUC corresponding to 0.962, indicating that the disclosed method has above 95% accuracy across the cohort of 28 children for pediatric cancer based on tooth sampling.

**Table 10. Features with empirical x values for pediatric cancer determination.**

| **Feature** | ***β* value** | **Feature in Table 2 or 3?** |
|---|---|---|
| Determinism_CrAs | 0.83118208 | No |
| Determinism_CrPb | 0.8064229 | No |
| Determinism_AsMo | 0.75871856 | No |
| Determinism_AsSn | 0.67313952 | No |
| Determinism_Mg25Cu | 0.66743556 | No |
| Determinism_AlSr | 0.65730066 | No |
| Determinism_CrNi | 0.61364904 | No |
| Determinism _MnNi | 0.59432577 | No |
| Determinism_CrSn | 0.58804872 | No |
| Determinism_Al | 0.58617909 | Yes |
| Determinism_CrMo | 0.58520657 | No |
| Determinism_AsPb | 0.58326161 | No |
| Determinism_CrZn | 0.56050935 | No |
| Determinism_CrSr | 0.54090339 | No |
| Determinism_AlCr | 0.53743572 | No |
| Determinism_Mg25Al | 0.48446496 | No |
| Determinism_Cr | 0.4817402 | Yes |
| Determinism_AlPb | 0.45549817 | No |
| Determinism_CrMn | 0.45130031 | No |
| Determinism_AsSr | 0.44475806 | No |
| Determinism_AlBa | 0.42164038 | No |
| Determinism_AlZn | 0.37990421 | No |
| Determinism_AlMn | 0.37500187 | No |
| Determinism_Mg25 | 0.3683976 | No |
| Determinism_AlSn | 0.35158099 | No |
| Determinism_AlCa43 | 0.34405451 | No |
| Determinism_CrCu | 0.3360983 | No |
| Determinism_MnBa | 0.33540969 | No |
| Determinism_MoSn | 0.33192421 | No |
| Determinism_Mn | 0.31460247 | Yes |
| Determinism_MnSr | 0.31368106 | No |
| Determinism_AsBa | 0.30838099 | No |
| Determinism_Ca43Sn | 0.29984189 | No |
| Determinism_AlMo | 0.28857433 | No |
| Determinism_AlNi | 0.2837695 | No |
| Determinism_Mg25Cr | 0.28189452 | No |
| Determinism_As | 0.28036618 | Yes |
| Determinism_MnMo | 0.26611626 | No |
| Determinism_MnZn | 0.25921097 | No |
| Determinism_Mg25Ni | 0.2517347 | No |
| Determinism_Mg25Ca43 | 0.24790031 | No |
| Determinism_Ca43Cu | 0.23667494 | No |
| Determinism_Mg25As | 0.2302827 | No |
| Determinism_AlAs | 0.22992372 | No |
| Determinism_Mg25Sr | 0.21738528 | No |
| Determinism_Mo | 0.21074891 | No |
| Determinism_Ca43Pb | 0.19990923 | No |
| Determinism_MnCu | 0.19801204 | No |
| Determinism_AlCu | 0.19386486 | No |
| Entropy_AsMo | 0.19141784 | No |
| Determinism_Mg25Sn | 0.18876961 | No |
| Determinism_Ca43As | 0.18366859 | No |
| Determinism_Mg25Zn | 0.18155086 | No |
| Determinism_MnAs | 0.16882166 | No |
| Determinism_Mg25Pb | 0.16563132 | No |
| Determinism_Mg25Mo | 0.16249869 | No |
| Entropy_Mg25Al | 0.15440345 | No |
| Entropy_CrMo | 0.15286797 | No |
| Entropy_AlSr | 0.15164735 | No |
| Determinism_Ca43Zn | 0.14229664 | No |
| Entropy_AlCu | 0.14029367 | No |
| Entropy_Mg25Ca43 | 0.13724276 | No |
| Entropy_Cr | 0.13724044 | Yes |
| Determinism_LiCu | 0.13655695 | No |
| Entropy_AlMo | 0.13573654 | No |
| Entropy_CrZn | 0.13488609 | No |
| Determinism_Ca43 | 0.1342346 | No |
| Entropy_AlZn | 0.13367488 | No |
| Entropy_AsSn | 0.13157993 | No |
| Entropy_CrSr | 0.13155615 | No |
| Entropy_As | 0.12753161 | No |
| Determinism_CuAs | 0.12740537 | No |
| Entropy_CrSn | 0.12636765 | No |
| Entropy_CrNi | 0.12602336 | No |
| Determinism_MnSn | 0.12491913 | No |
| Entropy_CrCu | 0.11837983 | No |
| Entropy_AlBa | 0.11789151 | No |
| Entropy_Al | 0.11758586 | Yes |
| Entropy_CrAs | 0.11754868 | No |
| Entropy_CrBa | 0.1097003 | No |
| Entropy_Ca43Sn | 0.10626234 | No |
| Entropy_AlCa43 | 0.10583429 | No |
| Entropy_CuMo | 0.10061917 | No |
| Entropy_SnBa | 0.09721063 | No |
| Determinism_LiSn | 0.09101129 | No |
| Entropy_MnNi | 0.09012707 | No |
| Entropy_Mg25Cu | 0.08972304 | No |
| Entropy_CrMn | 0.08918573 | No |
| Entropy_CuSn | 0.08872076 | No |
| Entropy_Ca43 | 0.08599791 | No |
| Entropy_AlSn | 0.08431399 | No |
| MDL_AsMo | 0.08345802 | No |
| Entropy_CrPb | 0.08287164 | No |
| Entropy_Ca43Cu | 0.08059603 | No |
| MDL_CrPb | 0.07907872 | No |
| Determinism_Mg25Mn | 0.07527645 | No |
| MDL_CrMo | 0.07412785 | No |
| Determinism_Ca43Mn | 0.071783 | No |
| Entropy_MnSr | 0.07124762 | No |
| Entropy_AlMn | 0.07101429 | No |
| Determinism_MnPb | 0.07067955 | No |
| MDL_AlMo | 0.0695314 | No |
| Entropy_Mg25Mo | 0.06869675 | No |
| MDL_CrSn | 0.06663217 | No |
| MDL_AsSn | 0.0656736 | No |
| Entropy_Ca43Sr | 0.06557269 | No |
| Determinism_LiCa43 | 0.06345066 | No |
| MDL_Cr | 0.06302333 | Yes |
| Entropy_Mg25Sn | 0.06282819 | No |
| Entropy_MnAs | 0.06200469 | No |
| Determinism_CuSr | 0.06199107 | No |
| Entropy_MoBa | 0.06028466 | No |
| Entropy_Ca43Ni | 0.06008138 | No |
| Entropy_Mg25As | 0.0581392 | No |
| MDL_CrAs | 0.0579383 | No |
| Determinism_Ca43Mo | 0.05760132 | No |
| MDL_Mg25Al | 0.05743773 | No |
| Determinism_Cu | 0.05482349 | No |
| MDL_CrNi | 0.05476749 | No |
| Entropy_AsPb | 0.05328563 | No |
| Determinism_CrBa | 0.05265275 | No |
| Entropy_Sn | 0.05221241 | Yes |
| Entropy_AsSr | 0.05198491 | No |
| Entropy_AsBa | 0.05172377 | No |
| Determinism_Ca43Sr | 0.0514286 | No |
| Entropy_MnCu | 0.05120313 | No |
| Entropy_Mo | 0.0507179 | No |
| Entropy_MnSn | 0.05033457 | No |
| Entropy_MnZn | 0.05032908 | No |
| MDL_AlSn | 0.0492198 | No |
| Entropy_Ca43Mo | 0.04889074 | No |
| MDL_As | 0.04884107 | Yes |
| Entropy_MnMo | 0.04873734 | No |
| MDL_CrZn | 0.04864297 | No |
| MDL_AlZn | 0.04828068 | No |
| Entropy_SrSn | 0.04789085 | No |
| Entropy_AlAs | 0.04671146 | No |
| Entropy_Mg25Pb | 0.04592187 | No |
| Determinism_Ca43Ba | 0.04530086 | No |
| Entropy_CuSr | 0.04508388 | No |
| Entropy_AlCr | 0.04458986 | No |
| MDL_AlCa43 | 0.04451255 | No |
| MDL_AlBa | 0.04436354 | No |
| Entropy_Ca43Ba | 0.04403844 | No |
| MDL_Ca43Sn | 0.04378155 | No |
| MDL_AlSr | 0.04372138 | No |
| Entropy_Mg25Ni | 0.04350715 | No |
| MDL_CuSn | 0.04335379 | No |
| MDL_Mg25Ca43 | 0.04214704 | No |
| Entropy_Mg25Sr | 0.04175057 | No |
| Entropy_Mg25 | 0.04174833 | No |
| Entropy_MnBa | 0.04106751 | No |
| Entropy_AlNi | 0.04059491 | No |
| MDL_Al | 0.0404738 | Yes |
| Entropy_CuAs | 0.039935 | No |
| Entropy_MoSn | 0.03948098 | No |
| MDL_MnNi | 0.03774724 | No |
| Entropy_Mg25Cr | 0.03757149 | No |
| MDL_AlAs | 0.03692396 | No |
| MDL_AlCu | 0.03685177 | No |
| MDL_CrCu | 0.03670764 | No |
| MDL_CrSr | 0.03568829 | No |
| MDL_CuMo | 0.03207434 | No |
| MDL_Ca43Cu | 0.03182457 | No |
| MDL_Mg25Cu | 0.0317094 | No |
| MDL_SnPb | 0.0313948 | No |
| Determinism_CuMo | 0.03131308 | No |
| Determinism_LiPb | 0.03108278 | No |
| MDL_MnAs | 0.0307727 | No |
| MDL_AsPb | 0.03076553 | No |
| Entropy_AlPb | 0.03053911 | No |
| Entropy_Mn | 0.02955969 | Yes |
| Entropy_LiAl | 0.02955272 | No |
| Entropy_Ca43Pb | 0.02952587 | No |
| Entropy_Ca43As | 0.02947391 | No |
| Entropy_Mg25Zn | 0.02932978 | No |
| Entropy_Cu | 0.02928631 | Yes |
| MDL_Mg25Mo | 0.02897986 | No |
| MDL_AlNi | 0.02876477 | No |
| MDL_AlCr | 0.0284849 | No |
| MDL_MnMo | 0.02803741 | No |
| MDL_Ca43 | 0.02724049 | No |
| MDL_Ca43Ni | 0.02631872 | No |
| MDL_Ca43Mo | 0.02598735 | No |
| MDL_AlMn | 0.02590792 | No |
| MDL_CrBa | 0.025747 | No |
| MDL_CrMn | 0.02478309 | No |
| Entropy_Ca43Zn | 0.02464592 | No |
| Entropy_MnPb | 0.02456997 | No |
| MDL_AlPb | 0.02445773 | No |
| MDL_AsSr | 0.02432544 | No |
| MDL_Sn | 0.02333864 | Yes |
| MDL_LiAl | 0.02157883 | No |
| MDL_Mo | 0.02078952 | No |
| MDL_MnSr | 0.02077614 | No |
| MDL_Mg25Sn | 0.02036126 | No |
| MDL_Cu | 0.02027604 | Yes |
| MDL_SnBa | 0.01998098 | No |
| Determinism_CuZn | 0.01880794 | No |
| MDL_Ca43Sr | 0.01840131 | No |
| Entropy_LiMo | 0.018315 | No |
| MDL_MoSn | 0.01762092 | No |
| MDL_Mg25As | 0.01753597 | No |
| Entropy_LiNi | 0.01735916 | No |
| MDL_MnCu | 0.01697323 | No |
| MDL_MoBa | 0.01628387 | No |
| Entropy_SnPb | 0.01604937 | No |
| Entropy_Ca43Cr | 0.01592327 | No |
| MDL_MnSn | 0.0158718 | No |
| MDL_CuAs | 0.01565716 | No |
| MDL**_**Ca43As | 0.01552682 | No |
| MDL_MnZn | 0.01520005 | No |
| MDL_LiMo | 0.01378473 | No |
| MDL_Mg25Cr | 0.01369645 | No |
| MDL_Mg25Ni | 0.01356079 | No |
| MDL_MnBa | 0.01260993 | No |
| Entropy_CuZn | 0.01254063 | No |
| Entropy_Ca43Mn | 0.01229527 | No |
| Entropy_Sr | 0.01194537 | Yes |
| MDL_Ca43Ba | 0.01133665 | No |
| MDL_Ca43Pb | 0.01119663 | No |
| MDL_Ca43Zn | 0.01097782 | No |
| MDL_LiNi | 0.01062496 | No |
| MDL_AsBa | 0.01055529 | No |
| Entropy_LiCr | 0.0102781 | No |
| MDL_CuSr | 0.00967959 | No |
| MDL_Mg25Pb | 0.0079405 | No |
| MDL_Ca43Cr | 0.00697082 | No |
| MDL_MnPb | 0.00669827 | No |
| MDL_Mg25 | 0.00653391 | No |
| MDL_LiCr | 0.00552944 | No |
| MDL_Mg25Zn | 0.00522171 | No |
| MDL_Mn | 0.00505689 | Yes |
| MDL_LiPb | 0.00501943 | No |
| MDL_Sr | 0.00334692 | Yes |
| MDL_Ca43Mn | 0.00260287 | No |
| MDL_MoPb | 0.0021107 | No |
| Entropy_Mg25Mn | 0.00210428 | No |
| MDL_CuZn | 0.00172756 | No |
| MDL_SrSn | 0.00152638 | No |
| MDL_Zn | 0.00140798 | Yes |
| Entropy_LiPb | 0.00134958 | No |
| MDL_Mg25Sr | 0.00075164 | No |
| Determinism_LiZn | 8.93E-05 | No |
| MDL_LiCa43 | -0.0022607 | No |
| MDL_SrBa | -0.0031041 | No |
| MDL_ZnMo | -0.0044459 | No |
| MDL_ZnSr | -0.0044924 | Yes |
| MDL_LiMn | -0.0045107 | No |
| Determinism_Ca43Cr | -0.0045376 | No |
| MDL_LiSn | -0.0069244 | No |
| MDL_Mg25Mn | -0.0074759 | No |
| Entropy_MoPb | -0.0075005 | No |
| Entropy_Zn | -0.0079352 | Yes |
| Entropy_SrBa | -0.0082067 | No |
| MDL_SrPb | -0.0082519 | No |
| MDL_LiSr | -0.0094466 | No |
| Entropy_LiMn | -0.0120857 | No |
| Entropy_ZnSr | -0.0123194 | Yes |
| MDL_ZnSn | -0.0157546 | Yes |
| MDL_LiZn | -0.0167321 | No |
| MDL_Pb | -0.0168931 | Yes |
| Entropy_LiCa43 | -0.0183219 | No |
| Determinism_LiAs | -0.0195469 | No |
| MDL_CuBa | -0.0217746 | No |
| MDL_Ba | -0.0222979 | Yes |
| MDL_NiZn | -0.0225005 | No |
| MDL_ZnBa | -0.0225495 | Yes |
| Entropy_Mg25Ba | -0.0226595 | No |
| MDL_ZnAs | -0.0227291 | Yes |
| MDL_CuPb | -0.0229478 | No |
| Entropy_BaPb | -0.0241505 | No |
| Determinism_LiNi | -0.0242261 | No |
| MDL_BaPb | -0.0243734 | No |
| Entropy_ZnMo | -0.0251275 | No |
| Entropy_LiSr | -0.0253481 | No |
| Entropy_CuBa | -0.0261639 | No |
| Entropy_CuPb | -0.0269427 | No |
| Entropy_Pb | -0.0280515 | Yes |
| MDL_LiBa | -0.0281562 | No |
| MDL_LiAs | -0.0294673 | No |
| Entropy_LiBa | -0.0304657 | No |
| MDL_NiPb | -0.0305375 | No |
| MDL_Mg25Ba | -0.0309118 | No |
| Determinism_CuPb | -0.031625 | No |
| MDL_NiSr | -0.031904 | No |
| Entropy_SrPb | -0.0334324 | No |
| MDL_ZnPb | -0.0335973 | Yes |
| MDL_LiMg25 | -0.0337371 | No |
| MDL_Ni | -0.0343914 | Yes |
| MDL_LiCu | -0.0344311 | No |
| Entropy_LiZn | -0.0356069 | No |
| MDL_Li | -0.0364019 | Yes |
| MDL_NiBa | -0.0369198 | No |
| Determinism_LiAl | -0.0396184 | No |
| MDL_SrMo | -0.0412142 | No |
| MDL_NiSn | -0.0433706 | No |
| Entropy_ZnBa | -0.0437905 | Yes |
| Entropy_NiZn | -0.0441552 | No |
| Entropy_NiPb | -0.0441841 | No |
| Entropy_ZnSn | -0.0479249 | Yes |
| Entropy_LiCu | -0.0506353 | No |
| Entropy_LiSn | -0.0520773 | No |
| MDL_NiAs | -0.0529876 | No |
| Entropy_LiAs | -0.056885 | No |
| Entropy_NiBa | -0.0578575 | No |
| Entropy_Li | -0.0584355 | Yes |
| Determinism_LiMg25 | -0.0615968 | No |
| MDL_NiMo | -0.0637464 | No |
| MDL_NiCu | -0.0657485 | No |
| Entropy_ZnPb | -0.066002 | Yes |
| Entropy_ZnAs | -0.0703648 | Yes |
| Entropy_Ni | -0.0708359 | Yes |
| Entropy_LiMg25 | -0.071669 | No |
| Determinism_Ca43Ni | -0.0761811 | No |
| Entropy_NiCu | -0.0779726 | No |
| Entropy_Ba | -0.0820585 | Yes |
| Determinism_ZnSr | -0.0900876 | Yes |
| Determinism_SrPb | -0.0916928 | No |
| Entropy_NiAs | -0.0950552 | No |
| Determinism_ZnBa | -0.0959118 | Yes |
| Determinism_LiMn | -0.0978331 | No |
| Entropy_NiSn | -0.1060182 | No |
| Determinism_SrSn | -0.1094853 | No |
| Determinism_CuBa | -0.1107066 | No |
| Entropy_SrMo | -0.1111222 | No |
| Determinism_SrBa | -0.1138937 | No |
| Entropy_NiSr | -0.1199173 | No |
| Determinism_LiMo | -0.1247136 | No |
| Determinism_Pb | -0.1284508 | Yes |
| Entropy_NiMo | -0.129908 | No |
| Determinism_Sr | -0.1328462 | Yes |
| Determinism_LiBa | -0.1403608 | No |
| Determinism_LiCr | -0.1514256 | No |
| Determinism_MoPb | -0.1527844 | No |
| Determinism_Mg25Ba | -0.1566375 | No |
| Determinism_SnPb | -0.1656892 | No |
| Determinism_LiSr | -0.1711748 | No |
| Determinism_CuSn | -0.1742272 | No |
| Determinism_Sn | -0.1751359 | Yes |
| Determinism_MoBa | -0.183775 | No |
| Determinism_ZnAs | -0.184485 | Yes |
| Determinism_ZnSn | -0.1924023 | Yes |
| Determinism_ZnMo | -0.2001491 | No |
| Determinism_SnBa | -0.207863 | No |
| Determinism_NiPb | -0.2160932 | No |
| Determinism_Li | -0.2579859 | Yes |
| Determinism_ZnPb | -0.2617748 | Yes |
| Determinism_NiMo | -0.3166897 | No |
| Determinism_Ba | -0.3180302 | Yes |
| Determinism_Zn | -0.3387266 | No |
| Determinism_BaPb | -0.3854676 | No |
| Determinism_NiCu | -0.3949591 | No |
| Determinism_SrMo | -0.4133329 | No |
| Determinism_NiSr | -0.4561735 | No |
| Determinism_NiZn | -0.5695511 | No |
| Determinism_NiAs | -0.5844446 | No |
| Determinism_NiBa | -0.5948487 | No |
| Determinism_Ni | -0.5994648 | Yes |
| Determinism_NiSn | -0.7561256 | No |
| Intercept | -21.741388 | No |

## Claims

1. A method for evaluating a subject for a first biological condition associated with metal metabolism, the method comprising:
sampling, with a laser beam, each respective position in a plurality of positions along a reference line on a biological sample associated with metal metabolism of the subject, wherein the biological sample associated with metal metabolism of the subject is an *ex vivo* hair shaft sample, thereby obtaining a plurality of ion samples, each ion sample in the plurality of ion samples corresponding to a different position in the plurality of positions, and each position in the plurality of positions representing a different period of growth of the biological sample associated with metal metabolism;
analyzing each ion sample in the plurality of ion samples with an inductively coupled plasma mass spectrometer thereby obtaining a first dataset comprising a plurality of traces, each trace in the plurality of traces being a concentration of a corresponding elemental isotope, in a plurality of elemental isotopes, over time collectively determined from the plurality of ion samples;
deriving a second dataset comprising a number and a duration of recurrence from the plurality of traces comprising a set of features, each respective feature in the set of features being determined by a variation of a single isotope or a combination of isotopes in the plurality of traces; and
inputting the set of features into a trained classifier thereby determining by the trained classifier that the subject has the first biological condition associated with metal metabolism with an accuracy of at least about 80%, wherein the first biological condition associated with metal metabolism is autism spectrum disorder (ASD).

2. The method of claim 1, wherein the plurality of elemental isotopes is selected from the elemental isotopes listed in Table 1.

3. The method of claim 1, wherein each feature in the set of features is associated with a single respective trace of the plurality of traces or with two respective traces of the plurality of traces.

4. The method of claim 3, wherein the set of features is selected from the features listed in Table 2, 3, 4, 5, 6, 7, 8, 9, or 10, optionally wherein the set of features further includes one or more features listed in Table 3.

5. The method of claim 1, wherein evaluating the subject for the first biological condition associated with metal metabolism further includes discriminating between the first biological condition associated with metal metabolism and a second biological condition associated with metal metabolism distinct from the first biological condition associated with metal metabolism; wherein the second biological condition is attention-deficit/hyperactivity disorder (ADHD).

6. The method of claim 1, wherein the reference line corresponds to a longitudinal direction of the *ex vivo* hair shaft sample.

7. The method of claim 1, further comprising pretreating the biological sample associated with metal metabolism of the subject with a solvent or a surfactant, or irradiating the biological sample associated with metal metabolism of the subject with a low powered laser to remove any debris from the biological sample associated with metal metabolism of the subject, prior to the sampling.

8. The method of claim 1, wherein the sampling comprises irradiating, with a laser, the biological sample associated with metal metabolism of the subject, thereby extracting a plurality of particles from the biological sample associated with metal metabolism of the subject; and ionizing the plurality of particles with an inductively coupled plasma mass spectrometer, thereby obtaining the plurality of ion samples.

9. The method of claim 1, wherein the plurality of positions is sequenced such that a first position in the plurality of positions along the biological sample associated with metal metabolism of the subject corresponds to a position closest to a tip of the biological sample associated with metal metabolism of the subject.

10. The method of claim 1, wherein each trace in the plurality of traces comprises a plurality of data points, each data point being an instance of the respective position in the plurality of positions; wherein the deriving the second dataset includes removing, from the plurality of data points, such data points that do not meet a first criteria; and wherein the first criteria includes a mean absolute difference between adjacent data points in the plurality of data points being three times a standard deviation of the mean absolute difference between adjacent points.

11. The method of claim 1, wherein the concentration of the corresponding elemental isotope corresponds to a relative abundance of the corresponding elemental isotope to a control elemental isotope, the control elemental isotope included in the plurality of ion samples; optionally wherein the control elemental isotope is sulfur.

12. The method of claim 1, wherein the set of features is selected from the group consisting of a mean diagonal length, a determinism, a recurrence time, an entropy, a trapping time, and a laminarity.

13. The method of claim 1, wherein the trained classifier computes: $p \left(subject\right) = \frac{1}{1 + {e}^{- \left(α+{β}_{1}{x}_{1}+⋯+{β}_{k}{x}_{k}\right)}}$ wherein,
*p(subject)* is a probability that the subject has the first biological condition associated with metal metabolism,
*e* is Euler's number,
*α* is a calculated parameter associated with the probability that the subject has the first biological condition associated with metal metabolism when 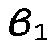 *x*₁ + ... + 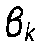 *xₖ* equals to zero,
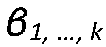 corresponds to a weight parameter associated with each feature in the set of features including features from 1 through *k*, and
*x*_{*1,* ... , *k*} corresponds to a value derived for each feature in the set of features, the set of features including features from 1 through *k*.

14. The method of claim 13, further comprising, responsive to determining that *p(subject)* is above a predetermined threshold, determining that the subject has the first biological condition associated with metal metabolism.

15. The method of claim 1, wherein the first biological condition associated with metal metabolism is related to a periodic dysregulation of metabolism of a plurality of metals, the plurality of metals corresponding to the plurality of elemental isotopes.

16. A device for evaluating a subject for a biological condition associated with metal metabolism, the device comprising one or more processors, and memory storing one or more programs for execution by the one or more processors, the one or more programs comprising instructions for:
directing a sampling, with a beam, of each respective position in a plurality of positions along a reference line on a biological sample associated with metal metabolism of the subject, wherein the biological sample associated with metal metabolism of the subject is an *ex vivo* hair shaft sample, thereby obtaining a plurality of ion samples, each ion sample in the plurality of ion samples corresponding to a different position in the plurality of positions, and each position in the plurality of positions representing a different period of growth of the biological sample associated with metal metabolism;
receiving a first dataset comprising a plurality of traces of each ion sample in the plurality of ion samples obtained with a laser ablation inductively coupled plasma mass spectrometer, each trace in the plurality of traces being a concentration of a corresponding elemental isotope, in a plurality of elemental isotopes, over time collectively determined from the plurality of ion samples;
deriving a second dataset comprising a number and a duration of recurrence from the plurality of traces comprising a set of features, each respective feature in the set of features being determined by a variation of a single isotope or a combination of isotopes in the plurality of traces; and
inputting the set of features into a trained classifier thereby determining by the trained classifier that the subject has the biological condition associated with metal metabolism with an accuracy of at least about 80%, wherein the biological condition associated with metal metabolism is autism spectrum disorder (ASD).

17. A non-transitory computer readable storage medium and one or more computer programs embedded therein for classification, the one or more computer programs comprising instructions which, when executed by a computer system, cause the computer system to perform a method evaluating a subject for a biological condition associated with metal metabolism, the method comprising:
directing a sampling, with a beam, of each respective position in a plurality of positions along a reference line on a biological sample associated with metal metabolism of the subject, wherein the biological sample associated with metal metabolism of the subject is an *ex vivo* hair shaft sample, thereby obtaining a plurality of ion samples, each ion sample in the plurality of ion samples corresponding to a different position in the plurality of positions, and each position in the plurality of positions representing a different period of growth of the biological sample associated with metal metabolism;
receiving a first dataset comprising a plurality of traces of each ion sample in the plurality of ion samples obtained with a laser ablation inductively coupled plasma mass spectrometer, each trace in the plurality of traces being a concentration of a corresponding elemental isotope, in a plurality of elemental isotopes, over time collectively determined from the plurality of ion samples;
deriving a second dataset comprising a number and a duration of recurrences from the plurality of traces comprising a set of features, each respective feature in the set of features being determined by a variation of a single isotope or a combination of isotopes in the plurality of traces; and
inputting the set of features into a trained classifier thereby determining by the trained classifier that the subject has the biological condition associated with metal metabolism with an accuracy of at least about 80%, wherein the biological condition associated with metal metabolism is autism spectrum disorder (ASD).

## Patentansprüche

1. Verfahren zur Untersuchung eines Subjekts auf einen ersten biologischen Zustand im Zusammenhang mit dem Metallstoffwechsel, das Verfahren umfassend:
Probenahme jeder einzelnen Position entlang einer Referenzlinie auf einer biologischen Probe des Subjekts, die mit dem Metallstoffwechsel im Zusammenhang steht, mittels eines Laserstrahls, wobei die biologische Probe des Subjekts, die mit dem Metallstoffwechsel im Zusammenhang steht, eine ex vivo-Haarschaftprobe ist, wodurch eine Vielzahl von Ionenproben gewonnen wird, wobei jede Ionenprobe aus der Vielzahl von Ionenproben einer anderen Position aus der Vielzahl von Positionen entspricht und jede Position aus der Vielzahl von Positionen einen anderen Wachstumszeitraum der mit dem Metallstoffwechsel im Zusammenhang stehenden biologischen Probe darstellt;
Analysieren jeder Ionenprobe aus der Vielzahl von Ionenproben mit einem induktiv gekoppelten Plasma-Massenspektrometer, wodurch ein erster Datensatz erhalten wird, der eine Vielzahl von Kurven umfasst, wobei jede Kurve aus der Vielzahl von Kurven eine Konzentration eines entsprechenden Elementisotops aus einer Vielzahl von Elementisotopen darstellt, die gemeinsam über Zeit aus der Vielzahl von Ionenproben bestimmt wurde;
Ableiten eines zweiten Datensatzes, umfassend eine Anzahl und eine Dauer der Wiederholung aus der Vielzahl von Kurven, umfassend eine Menge von Merkmalen, wobei jedes jeweilige Merkmal in der Menge von Merkmalen durch eine Variation eines einzelnen Isotops oder einer Kombination von Isotopen in der Vielzahl von Kurven bestimmt wird; und
Eingeben der Menge von Merkmalen in einen trainierten Klassifikator, wodurch durch den trainierten Klassifikator mit einer Genauigkeit von mindestens etwa 80 % festgestellt wird, dass das Subjekt den ersten biologischen Zustand hat, der mit dem Metallstoffwechsel im Zusammenhang steht, wobei der erste biologische Zustand, der mit dem Metallstoffwechsel im Zusammenhang steht, eine Autismus-Spektrum-Störung (ASD) ist.

2. Verfahren nach Anspruch 1, wobei die Vielzahl von Elementisotopen aus den in Tabelle 1 aufgeführten Elementisotopen ausgewählt wird.

3. Verfahren nach Anspruch 1, wobei jedes Merkmal in der Menge von Merkmalen mit einer jeweiligen einzelnen Kurve aus der Vielzahl von Kurven oder mit zwei jeweiligen Kurven aus der Vielzahl von Kurven assoziiert ist.

4. Verfahren nach Anspruch 3, wobei die Menge an Merkmalen aus den in den Tabellen 2, 3, 4, 5, 6, 7, 8, 9 oder 10 aufgeführten Merkmalen ausgewählt wird, optional wobei die Menge an Merkmalen ferner ein oder mehrere der in Tabelle 3 aufgeführten Merkmale umfasst.

5. Verfahren nach Anspruch 1, wobei die Beurteilung des Subjekts hinsichtlich des ersten biologischen Zustands, der mit dem Metallstoffwechsel im Zusammenhang steht, ferner die Unterscheidung zwischen dem ersten biologischen Zustand, der mit dem Metallstoffwechsel im Zusammenhang steht und einem zweiten biologischen Zustand, der mit dem Metallstoffwechsel im Zusammenhang steht, umfasst, der sich von dem ersten biologischen Zustand, der mit dem Metallstoffwechsel im Zusammenhang steht, unterscheidet; wobei der zweite biologische Zustand Aufmerksamkeitsdefizit- /Hyperaktivitätsstörung (ADHS) ist.

6. Verfahren nach Anspruch 1, wobei die Referenzlinie einer Längsrichtung der ex vivo-Haarschaftprobe entspricht.

7. Verfahren nach Anspruch 1, das ferner das Vorbehandeln der biologischen Probe des Subjekts, die mit dem Metallstoffwechsel im Zusammenhang steht, mit einem Lösungsmittel oder einem Tensid oder das Bestrahlen der biologischen Probe des Subjekts, die mit dem Metallstoffwechsel im Zusammenhang steht, mit einem Laser geringer Leistung umfasst, um vor der Probenahme etwaige Rückstände aus der biologischen Probe des Subjekts, die mit dem Metallstoffwechsel im Zusammenhang steht, zu entfernen.

8. Verfahren nach Anspruch 1, wobei die Probenahme das Bestrahlen der biologischen Probe des Subjekts, die mit dem Metallstoffwechsel im Zusammenhang steht, mit einem Laser umfasst, wodurch eine Vielzahl von Partikeln aus der biologischen Probe des Subjekts, die mit dem Metallstoffwechsel im Zusammenhang steht, extrahiert wird; und das Ionisieren der Vielzahl von Partikeln mit einem induktiv gekoppelten Plasma-Massenspektrometer, wodurch die Vielzahl von Ionenproben gewonnen wird.

9. Verfahren nach Anspruch 1, wobei die Vielzahl von Positionen so angeordnet ist, dass eine erste Position aus der Vielzahl von Positionen entlang der biologischen Probe, die mit dem Metallstoffwechsel des Subjekts im Zusammenhang steht, der Position entspricht, die einer Spitze der biologischen Probe, die mit dem Metallstoffwechsel des Subjekts im Zusammenhang steht, am nächsten liegt.

10. Verfahren nach Anspruch 1, wobei jede Kurve in der Vielzahl von Kurven eine Vielzahl von Datenpunkten umfasst, wobei jeder Datenpunkt eine Instanz der jeweiligen Position in der Vielzahl von Positionen darstellt; wobei das Ableiten des zweiten Datensatzes das Entfernen solcher Datenpunkte aus der Vielzahl von Datenpunkten umfasst, die ein erstes Kriterium nicht erfüllen; und wobei das erste Kriterium vorsieht, dass die mittlere absolute Differenz zwischen benachbarten Datenpunkten in der Vielzahl von Datenpunkten das Dreifache einer Standardabweichung der mittleren absoluten Differenz zwischen benachbarten Punkten beträgt.

11. Verfahren nach Anspruch 1, wobei die Konzentration des entsprechenden Elementisotops einer relativen Häufigkeit des entsprechenden Elementisotops im Verhältnis zu einem Referenz-Elementisotop entspricht, optional wobei das Referenz-Elementisotop in der Vielzahl von Ionenproben enthalten ist; wobei das Referenz-Elementisotop Schwefel ist.

12. Verfahren nach Anspruch 1, wobei die Menge an Merkmalen aus der Gruppe ausgewählt wird bestehend aus einer mittleren Diagonallänge, einem Determinismus, einer Wiederholungszeit, einer Entropie, einer Einfangzeit und einer Laminarität.

13. Verfahren nach Anspruch 1, wobei der trainierte Klassifikator Folgendes berechnet: $p \left(Subjekt\right) = \frac{1}{1 + {e}^{- \left(α+{β}_{1}{x}_{1}+⋯+{β}_{k}{x}_{k}\right)}}$ wobei,
*p(Subjekt)* die Wahrscheinlichkeit ist, dass das Subjekt den ersten biologischen Zustand, der mit dem Metallstoffwechsel im Zusammenhang steht, aufweist,
*e* die Eulersche Zahl ist,
*α* ein berechneter Parameter ist, der mit der Wahrscheinlichkeit zusammenhängt, dass das Subjekt den ersten biologischen Zustand, der mit dem Metallstoffwechsel im Zusammenhang steht, aufweist, wenn β₁x₁ + ... + βₖxₖ gleich Null ist,
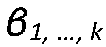 einem Gewichtsparameter entspricht, der jedem Merkmal in der Menge der Merkmale zugeordnet ist, einschließlich der Merkmale von 1 bis k, und
*x_{1, ..., k}* einem Wert entspricht, der für jedes Merkmal in der Menge der Merkmale abgeleitet wird, wobei die Menge der Merkmale die Merkmale von 1 bis k umfasst.

14. Verfahren nach Anspruch 13, das ferner umfasst, dass als Reaktion auf die Feststellung, dass *p(Subjekt)* über einem vorgegebenen Schwellenwert liegt, festgestellt wird, dass das Subjekt den ersten biologischen Zustand, der mit dem Metallstoffwechsel im Zusammenhang steht, aufweist.

15. Verfahren nach Anspruch 1, wobei der erste biologische Zustand, der mit dem Metallstoffwechsel im Zusammenhang steht, mit einer periodischen Dysregulation des Stoffwechsels einer Vielzahl von Metallen zusammenhängt, wobei die Vielzahl von Metallen der Vielzahl von Elementisotopen entspricht.

16. Vorrichtung zur Beurteilung eines Subjekts hinsichtlich eines biologischen Zustands, der mit dem Metallstoffwechsel im Zusammenhang steht, die Vorrichtung umfassend einen oder mehrere Prozessoren sowie einen Speicher, der ein oder mehrere Programme zur Ausführung durch den einen oder die mehreren Prozessoren speichert, das eine oder die mehreren Programme umfassend Anweisungen für:
Anweisen einer Probenahme mit einem Strahl an jeder jeweiligen Position aus einer Vielzahl von Positionen entlang einer Referenzlinie auf einer biologischen Probe, die mit dem Metallstoffwechsel des Subjekts im Zusammenhang steht, wobei die biologische Probe, die mit dem Metallstoffwechsel des Subjekts im Zusammenhang steht, eine ex vivo-Haarschaftprobe ist, wodurch eine Vielzahl von Ionenproben gewonnen wird, wobei jede Ionenprobe aus der Vielzahl von Ionenproben einer anderen Position aus der Vielzahl von Positionen entspricht und jede Position aus der Vielzahl von Positionen einen anderen Wachstumszeitraum der mit dem Metallstoffwechsel im Zusammenhang stehenden biologischen Probe darstellt;
Erhalten eines ersten Datensatzes, der eine Vielzahl von Kurven jeder Ionenprobe aus der Vielzahl von Ionenproben umfasst, die mit einem Laserablations-induktiv gekoppelten Plasma-Massenspektrometer gewonnen wurden, wobei jede Kurve aus der Vielzahl der Kurven eine Konzentration eines entsprechenden Elementisotops aus einer Vielzahl von Elementisotopen darstellt, die gemeinsam über Zeit aus der Vielzahl der Ionenproben bestimmt wurde;
Ableiten eines zweiten Datensatzes, der eine Anzahl und eine Dauer der Wiederholung aus der Vielzahl von Kurven umfasst, wobei die Vielzahl von Kurven eine Menge von Merkmalen umfasst und jedes jeweilige Merkmal in der Menge von Merkmalen durch eine Variation eines einzelnen Isotops oder einer Kombination von Isotopen in der Vielzahl von Kurven bestimmt wird; und
Eingeben der Menge von Merkmalen in einen trainierten Klassifikator, wodurch durch den trainierten Klassifikator mit einer Genauigkeit von mindestens etwa 80 % festgestellt wird, dass das Subjekt den biologischen Zustand, der mit dem Metallstoffwechsel im Zusammenhang steht, aufweist, wobei der biologische Zustand, der mit dem Metallstoffwechsel im Zusammenhang steht, eine Autismus-Spektrum-Störung (ASD) ist.

17. Nichtflüchtiges, computerlesbares Speichermedium und ein oder mehrere darin eingebettete Computerprogramme zur Klassifizierung, das eine oder die mehreren Computerprogramme umfassend Befehle, die wenn sie von einem Computersystem ausgeführt werden, das Computersystem veranlassen, ein Verfahren zur Beurteilung eines Subjekts hinsichtlich eines biologischen Zustands, der mit dem Metallstoffwechsel im Zusammenhang steht, durchzuführen, das Verfahren umfassend:
Anweisen einer Probenahme mit einem Strahl an jeder jeweiligen Position aus einer Vielzahl von Positionen entlang einer Referenzlinie auf einer biologischen Probe, die mit dem Metallstoffwechsel des Subjekts im Zusammenhang steht, wobei die biologische Probe, die mit dem Metallstoffwechsel des Subjekts im Zusammenhang steht, eine ex vivo-Haarschaftprobe ist, wodurch eine Vielzahl von Ionenproben gewonnen wird, wobei jede Ionenprobe aus der Vielzahl von Ionenproben einer anderen Position aus der Vielzahl von Positionen entspricht und jede Position aus der Vielzahl von Positionen einen anderen Wachstumszeitraum der mit dem Metallstoffwechsel im Zusammenhang stehenden biologischen Probe darstellt;
Erhalten eines ersten Datensatzes, der eine Vielzahl von Kurven jeder Ionenprobe aus der Vielzahl von Ionenproben umfasst, die mit einem Laserablations-induktiv gekoppelten Plasma-Massenspektrometer gewonnen wurden, wobei jede Kurve aus der Vielzahl von Kurven eine Konzentration eines entsprechenden Elementisotops aus einer Vielzahl von Elementisotopen darstellt, die gemeinsam über Zeit aus der Vielzahl der Ionenproben bestimmt wurde;
Ableiten eines zweiten Datensatzes, der eine Anzahl und eine Dauer der Wiederholung aus der Vielzahl von Kurven umfasst, wobei die Vielzahl von Kurven eine Menge von Merkmalen umfasst und jedes jeweilige Merkmal in der Menge von Merkmalen durch eine Variation eines einzelnen Isotops oder einer Kombination von Isotopen in der Vielzahl von Kurven bestimmt wird; und
Eingeben der Menge von Merkmalen in einen trainierten Klassifikator, wodurch durch den trainierten Klassifikator mit einer Genauigkeit von mindestens etwa 80 % festgestellt wird, dass das Subjekt den biologischen Zustand, der mit dem Metallstoffwechsel im Zusammenhang steht, aufweist, wobei der biologische Zustand, der mit dem Metallstoffwechsel im Zusammenhang steht, eine Autismus-Spektrum-Störung (ASD) ist.

## Revendications

1. Méthode d'évaluation d'un sujet relative à un premier état biologique associé à un métabolisme des métaux, la méthode comprenant :
l'échantillonnage, avec un faisceau laser, de chaque position respective d'une pluralité de positions le long d'une ligne de référence sur un échantillon biologique associé à un métabolisme des métaux du sujet, dans laquelle l'échantillon biologique associé à un métabolisme des métaux du sujet est un échantillon de tige de cheveu *ex vivo*, et ainsi l'obtention d'une pluralité d'échantillons d'ions, chaque échantillon d'ions de la pluralité d'échantillons d'ions correspondant à une position différente de la pluralité de positions, et chaque position de la pluralité de positions représentant une période de croissance différente de l'échantillon biologique associé au métabolisme des métaux ;
l'analyse de chaque échantillon d'ions de la pluralité d'échantillons d'ions avec un spectromètre de masse à plasma couplé par induction, et ainsi l'obtention d'un premier ensemble de données comprenant une pluralité de traces, chaque trace de la pluralité de traces étant une concentration d'un isotope élémentaire correspondant, d'une pluralité d'isotopes élémentaires, déterminée collectivement au fil du temps à partir de la pluralité d'échantillons d'ions ;
la dérivation d'un second ensemble de données comprenant un nombre et une durée de récurrences à partir de la pluralité de traces comprenant un ensemble de caractéristiques, chaque caractéristique respective de l'ensemble de caractéristiques étant déterminée par une variation d'un seul isotope ou d'une combinaison d'isotopes de la pluralité de traces ; et
l'entrée de l'ensemble de caractéristiques dans un classificateur entraîné, et ainsi la détermination, par le classificateur entraîné, du fait que le sujet présente le premier état biologique associé au métabolisme des métaux avec une précision d'au moins environ 80 %, dans laquelle le premier état biologique associé au métabolisme des métaux est un trouble du spectre autistique (TSA).

2. Méthode selon la revendication 1, dans laquelle la pluralité d'isotopes élémentaires est choisie parmi les isotopes élémentaires énumérés dans le tableau 1.

3. Méthode selon la revendication 1, dans laquelle chaque caractéristique de l'ensemble de caractéristiques est associée à une seule trace respective de la pluralité de traces ou à deux traces respectives de la pluralité de traces.

4. Méthode selon la revendication 3, dans laquelle l'ensemble de caractéristiques est sélectionné parmi les caractéristiques énumérées dans le tableau 2, 3, 4, 5, 6, 7, 8, 9, ou 10, facultativement, dans laquelle l'ensemble de caractéristiques comporte en outre une ou plusieurs caractéristiques énumérées dans le tableau 3.

5. Méthode selon la revendication 1, dans laquelle l'évaluation du sujet relative au premier état biologique associé à un métabolisme des métaux comporte en outre l'établissement d'une distinction entre le premier état biologique associé au métabolisme des métaux et un second état biologique associé à un métabolisme des métaux distinct du premier état biologique associé au métabolisme des métaux ; dans laquelle le second état biologique est un trouble du déficit de l'attention avec ou sans hyperactivité (TDAH).

6. Méthode selon la revendication 1, dans laquelle la ligne de référence correspond à une direction longitudinale de l'échantillon de tige de cheveux *ex vivo.*

7. Méthode selon la revendication 1, comprenant en outre le prétraitement de l'échantillon biologique associé au métabolisme des métaux du sujet avec un solvant ou un tensioactif, ou l'irradiation de l'échantillon biologique associé au métabolisme des métaux du sujet avec un laser à faible puissance pour éliminer tout débris de l'échantillon biologique associé au métabolisme des métaux du sujet, avant l'échantillonnage.

8. Méthode selon la revendication 1, dans laquelle l'échantillonnage comprend l'irradiation, avec un laser, de l'échantillon biologique associé au métabolisme des métaux du sujet, et ainsi l'extraction d'une pluralité de particules à partir de l'échantillon biologique associé au métabolisme des métaux du sujet ; et l'ionisation de la pluralité de particules avec un spectromètre de masse à plasma couplé par induction, et ainsi l'obtention de la pluralité d'échantillons d'ions.

9. Méthode selon la revendication 1, dans laquelle la pluralité de positions est séquencée de sorte qu'une première position de la pluralité de positions le long de l'échantillon biologique associé au métabolisme des métaux du sujet correspond à une position la plus proche d'une pointe de l'échantillon biologique associé au métabolisme des métaux du sujet.

10. Méthode selon la revendication 1, dans laquelle chaque trace de la pluralité de traces comprend une pluralité de points de données, chaque point de données étant une instance de la position respective de la pluralité de positions ; dans laquelle la dérivation du second ensemble de données comporte la suppression, à partir de la pluralité de points de données, de tels points de données qui ne répondent pas à un premier critère ; et dans laquelle le premier critère comporte une différence absolue moyenne entre des points de données adjacents de la pluralité de points de données étant à trois fois un écart-type de la différence absolue moyenne entre des points adjacents.

11. Méthode selon la revendication 1, dans laquelle la concentration de l'isotope élémentaire correspondant correspond à une abondance relative de l'isotope élémentaire par rapport à un isotope élémentaire témoin, l'isotope élémentaire témoin inclus dans la pluralité d'échantillons d'ions ; facultativement, dans laquelle l'isotope élémentaire témoin est le soufre.

12. Méthode selon la revendication 1, dans laquelle l'ensemble de caractéristiques est sélectionné dans le groupe consistant en une longueur diagonale moyenne, un déterminisme, un temps de récurrence, une entropie, un temps de piégeage et une laminarité.

13. Méthode selon la revendication 1, dans laquelle le classificateur entraîné calcule : $p \left(subject\right) = \frac{1}{1 + {e}^{- \left(α+{β}_{1}{x}_{1}+⋯+{β}_{k}{x}_{k}\right)}}$ dans laquelle,
*p(subject)* est une probabilité que le sujet présente le premier état biologique associé au métabolisme des métaux,
*e* est un nombre d'Euler,
*α* est un paramètre calculé associé à la probabilité que le sujet présente le premier état biologique associé au métabolisme des métaux lorsque *β*₁*x*₁ + ... *+ βₖx* ₖ est égal à zéro,
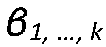 correspond à un paramètre de poids associé à chaque caractéristique de l'ensemble de caractéristiques incluant des caractéristiques de 1 à *k*, et
*x_{1, ... , k}* correspond à une valeur dérivée pour chaque caractéristique de l'ensemble de caractéristiques, l'ensemble de caractéristiques incluant des caractéristiques de 1 à *k*.

14. Méthode selon la revendication 13, comprenant en outre, en réponse à la détermination du fait que *p(subject)* est supérieur à un seuil prédéterminé, la détermination du fait que le sujet présente le premier état biologique associé au métabolisme des métaux.

15. Méthode selon la revendication 1, dans lequel le premier état biologique associé au métabolisme des métaux est lié à une dérégulation périodique du métabolisme d'une pluralité de métaux, la pluralité de métaux correspondant à la pluralité d'isotopes élémentaires.

16. Dispositif d'évaluation d'un sujet relative à un état biologique associé à un métabolisme des métaux, le dispositif comprenant un ou plusieurs processeurs, et une mémoire stockant un ou plusieurs programmes pour exécution par les un ou plusieurs processeurs, les un ou plusieurs programmes comprenant des instructions pour :
la direction d'un échantillonnage, avec un faisceau, de chaque position respective d'une pluralité de positions le long d'une ligne de référence sur un échantillon biologique associé à un métabolisme des métaux du sujet, dans lequel l'échantillon biologique associé à un métabolisme des métaux du sujet est un échantillon de tige de cheveu *ex vivo*, et ainsi l'obtention d'une pluralité d'échantillons d'ions, chaque échantillon d'ions de la pluralité d'échantillons d'ions correspondant à une position différente de la pluralité de positions, et chaque position de la pluralité de positions représentant une période de croissance différente de l'échantillon biologique associé au métabolisme des métaux ;
la réception d'un premier ensemble de données comprenant une pluralité de traces de chaque échantillon d'ions de la pluralité d'échantillons d'ions obtenus avec un spectromètre de masse à plasma à ablation laser couplé par induction, chaque trace de la pluralité de traces étant une concentration d'un isotope élémentaire correspondant, d'une pluralité d'isotopes élémentaires, déterminée collectivement au fil du temps à partir de la pluralité d'échantillons d'ions ;
la dérivation d'un second ensemble de données comprenant un nombre et une durée de récurrences à partir de la pluralité de traces comprenant un ensemble de caractéristiques, chaque caractéristique respective de l'ensemble de caractéristiques étant déterminée par une variation d'un seul isotope ou d'une combinaison d'isotopes de la pluralité de traces ; et
l'entrée de l'ensemble de caractéristiques dans un classificateur entraîné, et ainsi la détermination, par le classificateur entraîné, du fait que le sujet présente l'état biologique associé au métabolisme des métaux avec une précision d'au moins environ 80 %, dans lequel l'état biologique associé au métabolisme des métaux est un trouble du spectre autistique (TSA).

17. Support de stockage non transitoire lisible par ordinateur et un ou plusieurs programmes informatiques incorporés dans celui-ci à des fins de classification, les un ou plusieurs programmes informatiques comprenant des instructions qui, lorsqu'elles sont exécutées par un système informatique, amènent le système informatique à exécuter une méthode d'évaluation d'un sujet relative à un état biologique associé à un métabolisme des métaux, la méthode comprenant :
la direction d'un échantillonnage, avec un faisceau, de chaque position respective d'une pluralité de positions le long d'une ligne de référence sur un échantillon biologique associé à un métabolisme des métaux du sujet, dans lesquels l'échantillon biologique associé à un métabolisme des métaux du sujet est un échantillon de tige de cheveu *ex vivo*, et ainsi l'obtention d'une pluralité d'échantillons d'ions, chaque échantillon d'ions de la pluralité d'échantillons d'ions correspondant à une position différente de la pluralité de positions, et chaque position de la pluralité de positions représentant une période de croissance différente de l'échantillon biologique associé au métabolisme des métaux ;
la réception d'un premier ensemble de données comprenant une pluralité de traces de chaque échantillon d'ions de la pluralité d'échantillons d'ions obtenus avec un spectromètre de masse à plasma à ablation laser couplé par induction, chaque trace de la pluralité de traces étant une concentration d'un isotope élémentaire correspondant, d'une pluralité d'isotopes élémentaires, déterminée collectivement au fil du temps à partir de la pluralité d'échantillons d'ions ;
la dérivation d'un second ensemble de données comprenant un nombre et une durée de récurrences à partir de la pluralité de traces comprenant un ensemble de caractéristiques, chaque caractéristique respective de l'ensemble de caractéristiques étant déterminée par une variation d'un seul isotope ou d'une combinaison d'isotopes de la pluralité de traces ; et
l'entrée de l'ensemble de caractéristiques dans un classificateur entraîné, et ainsi la détermination, par le classificateur entraîné, du fait que le sujet présente l'état biologique associé au métabolisme des métaux avec une précision d'au moins environ 80 %, dans lequel l'état biologique associé au métabolisme des métaux est un trouble du spectre autistique (TSA).
